(19) Europäisches Patentamt
European Patent Office
Office européen des brevets

(11) EP 1 763 578 B1

# (12) EUROPÄISCHE PATENTSCHRIFT

(45) Veröffentlichungstag und Bekanntmachung des Hinweises auf die Patenterteilung:
**10.09.2014 Patentblatt 2014/37**

(21) Anmeldenummer: **05756616.8**

(22) Anmeldetag: **01.07.2005**

(51) Int Cl.:
*C12N 9/18* (2006.01) *C12N 9/20* (2006.01)

(86) Internationale Anmeldenummer:
**PCT/EP2005/007133**

(87) Internationale Veröffentlichungsnummer:
**WO 2006/002955 (12.01.2006 Gazette 2006/02)**

## (54) TANNASE

TANNASE

TANNASE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **02.07.2004 DE 102004032216**

(43) Veröffentlichungstag der Anmeldung:
**21.03.2007 Patentblatt 2007/12**

(73) Patentinhaber: **Leibniz-Institut für Pflanzengenetik und Kulturpflanzenforschung
06466 Gatersleben (DE)**

(72) Erfinder:
- **BOER, Erik
06484 Quedlinburg (DE)**
- **KUNZE, Gotthard
06466 Gatersleben (DE)**

(74) Vertreter: **Grünecker, Kinkeldey, Stockmair & Schwanhäusser
Leopoldstrasse 4
80802 München (DE)**

(56) Entgegenhaltungen:
- **HATAMOTO O ET AL: "Cloning and sequencing of the gene encoding tannase and a structural study of the tannase subunit from Aspergillus oryzae" GENE: AN INTERNATIONAL JOURNAL ON GENES AND GENOMES, ELSEVIER, AMSTERDAM, NL, Bd. 175, Nr. 1, 10. Oktober 1996 (1996-10-10), Seiten 215-221, XP004043318 ISSN: 0378-1119 & DATABASE EMBL-SVA 21. Januar 1999 (1999-01-21), gefunden im EBI Database accession no. D63338.1 & DATABASE UniProt 28. Februar 2003 (2003-02-28), gefunden im EBI Database accession no. P78581**
- **BHAT TEJ K ET AL: "Microbial degradation of tannins - A current perspective" BIODEGRADATION, Bd. 9, Nr. 5, 1998, Seiten 343-357, XP002352944 ISSN: 0923-9820**
- **BUI D M ET AL: "Cloning and expression of an Arxula adeninivorans glucoamylase gene in Saccharomyces cerevisiae" APPLIED MICROBIOLOGY AND BIOTECHNOLOGY, Bd. 44, Nr. 5, 1996, Seiten 610-619, XP009056633 ISSN: 0175-7598**
- **TAG K ET AL: "SHORT COMMUNICATION. FAST DETECTION OF HIGH MOLECULAR WEIGHT SUBSTANCES IN WASTEWATER BASED ON AN ENZYMATIC HYDROLYSIS COMBINED WITH THE ARXULA BOD SENSOR SYSTEM" JOURNAL OF CHEMICAL TECHNOLOGY AND BIOTECHNOLOGY, WILEY & SONS, CHICHESTER, GB, Bd. 75, Nr. 11, November 2000 (2000-11), Seiten 1080-1082, XP001060195 ISSN: 0268-2575**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 763 578 B1

Printed by Jouve, 75001 PARIS (FR)

SEQUENCE LISTING

<110> Institut fuer Pflanzengenetik und Kulturpflanzenforschung
<120> Polypeptide mit Tannase- und/oder Lipase-Aktivität
<130> P36471HV091
<160> 4
<170> PatentIn version 3.1
<210> 1
<211> 1465
<212> DNA
<213> Arxula adeninivorans
<400> 1

```
gactcaattg caggcacggt gccgattacg taccccccca tgtgcttagt gcctttcatg     60
ctacggatgt tggacactat aaaagatacg taaaatggca agcataccat tctttgttga    120
gatgaagcat tttctcggac aatctttatt gacaagtctg cttgcggcag gagcctttgg    180
atcctcgctt gccgaagtct gtacttcctc ccgcatccgg accgccttac caaaggatgg    240
agccatcgca gggatctcta tggacccaga cagtatcact gccaatccag tgtataatgc    300
atctgctggc tatagcgtgt tttaccccga gggaaacttt gattactgca atgtgactgt    360
ttcctactgt catattggca agggtgacaa agtcaatctg cagtattggc ttcctagtcc    420
agacaagttc caaaaccgtt acctggctac aggcggcggg ggatatgcca tcaactctgg    480
aactcagtca ctgcctggag gggtcatgta tggagcagtt gctggtagaa ccgatggagg    540
atttggaggg tttgatgtcc aagtttctga agccatcttg tacgccaatg gatctctcaa    600
ttacgatagt ctatacatgt ttggatatcg agcaattggt gagcagacca tgattggcca    660
ggagttagcg cgaggattct gtgaattggg ggacgagaag aagatttaca catactacca    720
ggggtgttcg gaaggagtac gtgaaggctg gagtcaaatc ctaaaatttc cagatctcta    780
cgatggagta atccctgctg cccctgcctt cagatatggg catcagcaag tgaaccacct    840
gtttccaggg gtcatagaac aaggcatgaa ctattaccct ccaccttgtg aaatggctcg    900
tatcgtcaat gccacaattg aggcttgcga caagctggat ggcaagatag acggagtagt    960
gtccaggaca gatctgtgtc tgttgaactt tgactttaat tctacaattg ggctccatta   1020
cacttgcgaa gcaggctcca accctatgac gggagactcc accccagcac aaaacggtac   1080
tgtttccacc aaggctgctg agcttgctcg ggtgttgaca gaagggctcc atgattcaca   1140
aggcaacaag gcatacgtct tttatcagat taccgccggg tatgacgatg cagacaccaa   1200
gtacaacccct gccaccgggc agtttgaatt gtcagtgagc agtcttggtg gtgagtgggt   1260

tacaaagctc ttgcagcttg tcgaccttga caatctacca aaccttgaca atgttactgt   1320
ggacacgctg gttgattgga tgcaatgcgg ttggcaaact tacgaagatg tgttacagac   1380
aaccaggcct gatctttctc tgtatgaaag agccggagga aagatcttga cattccacgg   1440
ggagtctgac aacagcatcc cctaa                                         1465
```

<210> 2
<211> 3001
<212> DNA
<213> Arxula adeninivorans
<400> 2

```
tcgcaattca tctcccaagc ccttttgctt atggcaagcg aattaacagg tgggtgtaca      60
aattgtgacc cgcccaccac aggcccaagt attgtgggct attggctgtc aatcaaccgt     120
tatcgcctcg gaagttcccc tcacgggccc gtaccgtttg cgctcaccag cgtgcccggc     180
ctgcagggtt ctatcggcat taccccgcaa aacggcgcgt gcatcatgta aacagagagt     240
gcaggggggt gagaaaaaag agaaaaccct tgaatgactg tttggtcggc aaaacgactc     300
aatcaacgtc gaactaattc caaacggtcg tgggatcgta tctctgcgat ctcattgcag     360
ttcatgtccg gatccacttg aacaactccg gtctggaatg aaaaatttaa ggggtagctc     420
tgcatttctg gcgtttgggc cagcagacat gcagatgtgc agaatcggtc ccgttccggg     480
gtaagattag ctatggcttt tggagagatc agcatagttt acacgttcat gtgcgcagat     540
aggcgtgaac aagaggtgta tataatgggg tcccattcgc atgcaattta agaagtgcgc     600
gatcttattg ctggtcggtt gaacatgctg aagctcctgc tacttgtggc ccaactggtg     660
gcttttgtat ttggtgcagc gctgcctgaa gagcgtgtat atgctcgcga tgctgtgacc     720
ccaccctctg aagatccgtt ctacacgcca gatgatggtt atgagaagga agagcctggt     780
acaattctgc gatggagaca cgctcctcag atgcctgctt ttactgtttt taagcagaat     840
cttaatgcgt ccattcagat cctgtaccgt actactgata ctcagggaca gcccacggcc     900
actgtggtga ctgtcatgat ccctcacaac catgccgaag gaaagctgct gtcgtaccag     960
tcctgggaag actctgcttg gatcaactgt gctccgtcgt atgcaattca gtttggtgct    1020
aaccctcaag ggatcatttc tcaggtggat atgcttactc ttcaatccgc tctcaatgag    1080
ggttggattg tttcaactcc cgattatgag ggtccaaagt cttcgttcac gtcagccatc    1140
atctccggac aggccactct ggactcgatt agagcagtgc tcaagtcgga gtcgtttacc    1200
ggggtcaagc ctgattcaaa ggtagccatg tggggatact ctgggggatc aatcgcatca    1260
ggttgggcag cagctctcca acccacgtat gcgccagagc tcaaaattgc aggagcagct    1320
```

ctcggaggag taatccagaa cattactagt gtggccgttc aggtgaataa gggcccgttt 1380

gttggtctgg taccggctgg aatcaaggga ttatcatccc aatatccaga gctagaagat 1440

tacattaacg atcaacttct tcccgacaag agagatgact ttgaaaaggc cggaaagcag 1500

tgtctttcag tagacgttct gacttatgcg ttccaggatt ggttttcata cacaaaggct 1560

ggggaccgag ttttgtacaa tgagaccatt caaaaagtcc tggatgagaa tgccatggga 1620

aagcagaaac ctcagattcc attgctgttt taccacggtg tccatgatga ggttatgcca 1680

attgctgatg tggacaagct ttattacgag tactgttcca atggcgttac cgtggaatac 1740

tacagagagg agggatcaga gcatgttctt gaaatgatca ctggattccc taaagcttac 1800

aattatgtca agaacctgct cgatggtggc tccgtgagct cgggatgcca gagacacgat 1860

gtgttttcta acgccttcga tgaagatgca ttgcctacct actctgccga aatttgggga 1920

attctcaagg gtttgctcgg agcccctgtt ggaccggctg ccatcagtta atctaaacta 1980

atgttggtcc tctgttggtg ttattaacgc agtggttttg actttgactt ctgatcattt 2040

tatcaaacag tcgttctgtg aagtcaacag ggcatattct cacttaatga aggagctgcg 2100

cggtttcggc tcgaataatt ttgtttttgg ttttgttttt gtttttgtt tttatttaaa 2160

ttcattatta aacagtctct tcgaaagtta acatgggcta tagacactaa gcttcaattg 2220

ttcgaaggta aaaaattgga cttaaaagta cattagatag tcttacttag acttaaaatc 2280

tgtttattag acagtccttt tgataagtca cgtggaccat gctagactgt gttcgtatat 2340

acttgtagtt actattcaat tgtttgaagg ccgacctctc aattcagtga gccctttgct 2400

ttagcaactt caaacataaa aaaaaaaaaa aagtgcggct gaaagcgatt tgtgaagtac 2460

ctctagtagg tcttaagatt ctcattttca ggatctggtg ttgtggcgca atggtgagcg 2520

catcggattt cggttgtgaa tcttccgaag gttgcaggtt cgagtcctgt caacatcgga 2580

tctttttttg caatccgttt tgtctgagtt aaggagattg acaatagctg ggctctgtct 2640

tgtcttcgta attgcaacag aaaataaagt tgtcattatt gaagcctctt tttcgcccta 2700

cggtctgttt aaagtatcaa ggttaaagtt ttacagagat catcgcttta gcatatcaac 2760

aactctcctt ctttcgacat cagtcctgaa ttgctaacct tacttttgct caattaattt 2820

ctagagcgac gcattcatct aataatctgt cttttcaaac tttaagcctg acatgcaccg 2880

gataagattt aaaactatcc atcctaaatg caatcataga tgaagtcctt atatacgcag 2940

tatataatct atatttttcg atcgcactga ctagaagaaa ttggaatcac atatcgccca 3000

t 3001

<210> 3

<211> 587
<212> PRT
<213> Arxula adeninivorans
<400> 3

```
Met Ala Ser Ile Pro Phe Phe Val Glu Met Lys His Phe Leu Gly Gln
1               5                   10                  15

Ser Leu Leu Thr Ser Leu Leu Ala Ala Gly Ala Phe Gly Ser Ser Leu
            20                  25                  30

Ala Glu Val Cys Thr Ser Ser Arg Ile Arg Thr Ala Leu Pro Lys Asp
        35                  40                  45

Gly Ala Ile Ala Gly Ile Ser Met Asp Pro Asp Ser Ile Thr Ala Asn
    50                  55                  60

Pro Val Tyr Asn Ala Ser Ala Gly Tyr Ser Val Phe Tyr Pro Glu Gly
65                  70                  75                  80

Asn Phe Asp Tyr Cys Asn Val Thr Val Ser Tyr Cys His Ile Gly Lys
                85                  90                  95

Gly Asp Lys Val Asn Leu Gln Tyr Trp Leu Pro Ser Pro Asp Lys Phe
            100                 105                 110

Gln Asn Arg Tyr Leu Ala Thr Gly Gly Gly Gly Tyr Ala Ile Asn Ser
        115                 120                 125

Gly Thr Gln Ser Leu Pro Gly Gly Val Met Tyr Gly Ala Val Ala Gly
    130                 135                 140

Arg Thr Asp Gly Gly Phe Gly Gly Phe Asp Val Gln Val Ser Glu Ala
145                 150                 155                 160

Ile Leu Tyr Ala Asn Gly Ser Leu Asn Tyr Asp Ser Leu Tyr Met Phe
                165                 170                 175

Gly Tyr Arg Ala Ile Gly Glu Gln Thr Met Ile Gly Gln Glu Leu Ala
            180                 185                 190

Arg Gly Phe Cys Glu Leu Gly Asp Glu Lys Lys Ile Tyr Thr Tyr Tyr
        195                 200                 205

Gln Gly Cys Ser Glu Gly Val Arg Glu Gly Trp Ser Gln Ile Leu Lys
    210                 215                 220
```

Phe Pro Asp Leu Tyr Asp Gly Val Ile Pro Ala Ala Pro Ala Phe Arg
225 230 235 240

Tyr Gly His Gln Gln Val Asn His Leu Phe Pro Gly Val Ile Glu Gln
245 250 255

Gly Met Asn Tyr Tyr Pro Pro Pro Cys Glu Met Ala Arg Ile Val Asn
260 265 270

Ala Thr Ile Glu Ala Cys Asp Lys Leu Asp Gly Lys Ile Asp Gly Val
275 280 285

Val Ser Arg Thr Asp Leu Cys Leu Leu Asn Phe Asp Phe Asn Ser Thr
290 295 300

Ile Gly Leu His Tyr Thr Cys Glu Ala Gly Ser Asn Pro Met Thr Gly
305 310 315 320

Asp Ser Thr Pro Ala Gln Asn Gly Thr Val Ser Thr Lys Ala Ala Glu
325 330 335

Leu Ala Arg Val Leu Thr Glu Gly Leu His Asp Ser Gln Gly Asn Lys
340 345 350

Ala Tyr Val Phe Tyr Gln Ile Thr Ala Gly Tyr Asp Asp Ala Asp Thr
355 360 365

Lys Tyr Asn Pro Ala Thr Gly Gln Phe Glu Leu Ser Val Ser Ser Leu
370 375 380

Gly Gly Glu Trp Val Thr Lys Leu Leu Gln Leu Val Asp Leu Asp Asn
385 390 395 400

Leu Pro Asn Leu Asp Asn Val Thr Val Asp Thr Leu Val Asp Trp Met
405 410 415

Gln Cys Gly Trp Gln Thr Tyr Glu Asp Val Leu Gln Thr Thr Arg Pro
420 425 430

Asp Leu Ser Leu Tyr Glu Arg Ala Gly Gly Lys Ile Leu Thr Phe His
435 440 445

Gly Glu Ser Asp Asn Ser Ile Pro Ala Gly Ser Ser Val His Phe Tyr
450 455 460

Glu Ser Val Arg Asn Val Met Tyr Pro Gly Ile Ser Phe Asn Gln Ser
465 470 475 480

Thr Asp Ala Met Gly Glu Trp Tyr Arg Leu Tyr Leu Val Pro Gly Ala
485 490 495

Ala His Cys Ser Ile Asn Ala Leu Gln Pro Asn Gly Pro Phe Pro Gln
500 505 510

Thr Thr Leu Glu Val Met Ile Asp Trp Val Glu Asn Gly Asn Thr Pro
515 520 525

Thr Thr Leu Gln Ala Thr Tyr Leu Val Gly Asp Asn Lys Gly Lys Pro
530 535 540

Ala Glu Ile Cys Pro Trp Pro Leu Arg Pro Thr Trp Thr Asp Glu Gly
545 550 555 560

Ser Lys Leu Gln Cys Val Tyr Asp His Thr Ser Ile Asn Thr Trp Met
565 570 575

Tyr Asp Phe Asn Ala Phe Ser Leu Pro Val Tyr
580 585

<210> 4
<211> 448
<212> PRT
<213> Arxula adeninivorans
<400> 4

Met Leu Lys Leu Leu Leu Leu Val Ala Gln Leu Val Ala Phe Val Phe
1 5 10 15

Gly Ala Ala Leu Pro Glu Glu Arg Val Tyr Ala Arg Asp Ala Val Thr
20 25 30

Pro Pro Ser Glu Asp Pro Phe Tyr Thr Pro Asp Asp Gly Tyr Glu Lys
35 40 45

Glu Glu Pro Gly Thr Ile Leu Arg Trp Arg His Ala Pro Gln Met Pro
50 55 60

Ala Phe Thr Val Phe Lys Gln Asn Leu Asn Ala Ser Ile Gln Ile Leu
65 70 75 80

Tyr Arg Thr Thr Asp Thr Gln Gly Gln Pro Thr Ala Thr Val Val Thr
85 90 95

```
Val Met Ile Pro His Asn His Ala Glu Gly Lys Leu Leu Ser Tyr Gln
            100                 105                 110

Ser Trp Glu Asp Ser Ala Trp Ile Asn Cys Ala Pro Ser Tyr Ala Ile
        115                 120                 125

Gln Phe Gly Ala Asn Pro Gln Gly Ile Ile Ser Gln Val Asp Met Leu
    130                 135                 140

Thr Leu Gln Ser Ala Leu Asn Glu Gly Trp Ile Val Ser Thr Pro Asp
145                 150                 155                 160

Tyr Glu Gly Pro Lys Ser Ser Phe Thr Ser Ala Ile Ile Ser Gly Gln
                165                 170                 175

Ala Thr Leu Asp Ser Ile Arg Ala Val Leu Lys Ser Glu Ser Phe Thr
            180                 185                 190

Gly Val Lys Pro Asp Ser Lys Val Ala Met Trp Gly Tyr Ser Gly Gly
        195                 200                 205

Ser Ile Ala Ser Gly Trp Ala Ala Ala Leu Gln Pro Thr Tyr Ala Pro
    210                 215                 220

Glu Leu Lys Ile Ala Gly Ala Ala Leu Gly Gly Val Ile Gln Asn Ile
225                 230                 235                 240

Thr Ser Val Ala Val Gln Val Asn Lys Gly Pro Phe Val Gly Leu Val
                245                 250                 255

Pro Ala Gly Ile Lys Gly Leu Ser Ser Gln Tyr Pro Glu Leu Glu Asp
            260                 265                 270

Tyr Ile Asn Asp Gln Leu Leu Pro Asp Lys Arg Asp Asp Phe Glu Lys
        275                 280                 285

Ala Gly Lys Gln Cys Leu Ser Val Asp Val Leu Thr Tyr Ala Phe Gln
    290                 295                 300

Asp Trp Phe Ser Tyr Thr Lys Ala Gly Asp Arg Val Leu Tyr Asn Glu
305                 310                 315                 320

Thr Ile Gln Lys Val Leu Asp Glu Asn Ala Met Gly Lys Gln Lys Pro
                325                 330                 335

Gln Ile Pro Leu Leu Phe Tyr His Gly Val His Asp Glu Val Met Pro
            340                 345                 350
```

```
Ile Ala Asp Val Asp Lys Leu Tyr Tyr Glu Tyr Cys Ser Asn Gly Val
        355                 360                 365

Thr Val Glu Tyr Tyr Arg Glu Glu Gly Ser Glu His Val Leu Glu Met
    370                 375                 380

Ile Thr Gly Phe Pro Lys Ala Tyr Asn Tyr Val Lys Asn Leu Leu Asp
385                 390                 395                 400

Gly Gly Ser Val Ser Ser Gly Cys Gln Arg His Asp Val Phe Ser Asn
                405                 410                 415

Ala Phe Asp Glu Asp Ala Leu Pro Thr Tyr Ser Ala Glu Ile Trp Gly
            420                 425                 430

Ile Leu Lys Gly Leu Leu Gly Ala Pro Val Gly Pro Ala Ala Ile Ser
        435                 440                 445
```

## Beschreibung

Die vorliegende Erfindung betrifft Nukleinsäuren und von diesen kodierte Polypeptide mit Tannase- Aktivität sowie Verfahren zu deren Herstellung. Ferner betrifft die vorliegende Erfindung Expressionskonstrukte, Wirtszellen, Antikörper und Biosensoren sowie deren Verwendung bei der Reduktion von Tanninen in Zusammensetzungen, die diese enthalten.

Die Größe des Weltmarktes für industriell hergestellte Enzyme hat sich in den letzten 25 Jahren mehr als verzehnfacht und beträgt heute deutlich über 500 Mio €. Enzyme werden in folgenden Bereichen angewendet Chemie- und Pharmaindustrie, Kosmetikaherstellung, Lebensmittel-, Leder-, Papier-, Stärke-, Waschmittel- und Textilindustrie sowie in der Tierernährung. Der größte Anteil an Nutzem von Enzymen findet sich in der Waschmittel- und Stärkeindustrie. Der Markt für Futterenzyme weist noch einen relativ kleinen Anteil auf, gehört jedoch zu den am schnellsten wachsenden Segmenten.

Die Synthese von Polymeren, Pharmazeutika und Agrochemikalien wird häufig durch teure Prozesse behindert, die schädliche Nebenprodukte liefern, und die oft nur wenig selektiv in Hinblick auf optische Isomere sind. Enzyme haben eine Großzahl an bemerkenswerten Eigenschaften, die viele der gegenwärtigen Probleme bei der Katalyse lösen: sie wirken auf einzelne funktionale Gruppen, unterscheiden zwischen ähnlichen funktionellen Gruppen innerhalb eines Moleküls, sind spezifisch für Enantiomere und wirken in Reaktionsmischungen in äußerst geringen molaren Anteilen katalytisch. Aufgrund der Spezifität enzymatischer Reaktionen stellen Enzyme eine einzigartige Lösung dar, eine effektive Umwandlung zu erreichen, die chemisch oft nur sehr schwer nachzuahmen ist. Enzyme leisten innerhalb der verschiedenen Industriezweige vielfältige Beiträge zur Wirtschaftlichkeit von Produktionsprozessen. So können Enzyme Ausbeute und Rohstoffnutzung verbessern, Herstellungskosten reduzieren, die Haltbarkeit von Produkten unterstützen, sowie technologische Eigenschaften von Prozesshilfsstoffen oder deren Endproduktion positiv verandem. Ein weiterer Vorteil bei der Verwendung in enzymatisch katalysierten Reaktionen in der Chemieindustrie ist die Schonung der Umwelt, da die Enzyme biologisch abbaubar sind, die Prozesse weniger Energie benötigen und weniger Chemikalien eingesetzt werden können.

Da der Bedarf an besseren Katalysatoren und umweltschonenderen Technologien in der Chemie-, Pharma- und Futtermittelindustrie zunimmt, besteht auch ein gesteigerter Bedarf an effektiveren und robusteren Enzymen für verschiedenste Industriezweige. Einer weiteren Verbreitung der industriellen Nutzung von Enzymen steht lediglich die geringe Anzahl hoch aktiver und gereinigter, kommerziell erhältlicher Enzyme entgegen.

Enzyme von kommerziellem Interesse wurden bislang hauptsächlich aus Mikroorganismen, insbesondere aus Pilzen und Bakterien, vor allem aus den *Aspergillus-Arten* gewonnen, wie *A. niger, A. oryzae, A. awamori, A. alliaceus, A. usamii, A. ficum, A. saitoi* und *A. melleus.* Die Gewinnung aus Mikroorganismen ist in der Regel ökonomischer als aus Pflanzen oder Tieren, darüber hinaus sind Mikroorganismen zur Synthese eines sehr breiten Spektrums hydrolytischer Enzyme fähig, zu deren Bildung der tierische oder pflanzliche Organismus nicht in der Lage ist. Da die Mikroorganismen oft an extreme Lebensbedingungen angepasst sind (Temperatur, pH, Osmolarität), sind mikrobielle Enzyme in vielen Fällen in dieser Hinsicht auch stabiler als Enzyme von Pflanzen und Tieren. Einen weiteren Vorteil stellt die bessere Standardisierbarkeit von mikrobiellen Enzympräparationen dar. Es werden nämlich vorzugsweise solche Pilz- und Bakterienstämme eingesetzt, die unter industriellen Produktionsbedingungen zu schneller Vermehrung und hoher Biosyntheseleistung fähig sind. Ziel ist dabei nicht nur eine hohe Enzymkonzentration, sondern auch eine hohe Ausbeute in geringer Zeit und bei kleinen Volumina. Entsprechend geeignete Stämme werden entweder aus der Wildpopulation selektiert und weiterentwickelt, oder aber man überträgt mittels gentechnischer Methoden gezielt Erbinformationen für bestimmte Enzyme auf geeignete Produktionsstämme. Dabei sucht man insbesondere nach solchen, die das oder die gewünschten Enzyme in möglichst großen Mengen und unter Verwendung spezifischer Nährmedien synthetisieren. Die Gentechnik ermöglicht eine ressourcenschonendere Produktion.

In der Nahrungsmittel- und Futtermittelindustrie spielen Enzyme eine große Rolle. Die Wirkung von Enzymen wird bereits seit Jahrtausenden in zahlreichen Prozessen der Lebensmittelgewinnung und Konservierung genutzt, ohne dass man Kenntnis über die Enzyme besaß (z. B. Gärung von Wein, Fermentation von Milch, Teigherstellung). Heute werden in der Nahrungsmittelindustrie Enzyme in den verschiedensten Bereichen eingesetzt Futterenzyme, als Zusatzstoffe in der Tierernährung europaweit erfasst, stammen von Mikroorganismen, die in der Natur weit verbreitet vorkommen. *Aspergillus niger* und *Penicillium* spec. z. B. produzieren Enzyme zum Abbau unterschiedlichster Substrate, allen gemeinsam ist jedoch die Produktion von Enzymen zur Spaltung von pflanzlichen Zellwandbestandteilen aus hochpolymeren Kohlenhydraten. Die beiden bedeutendsten Produktkategorien innerhalb der derzeit für die Tierernährung eingesetzten Enzyme stellen die Phytase sowie die Enzyme zum Abbau von Nicht-Stärke-Polysacchariden dar. Sie werden eingesetzt, um normalerweise schwer- oder nicht verdauliche Substanzen im Tierfutter für das Tier verdaubar zu machen, so dass dem Tier mehr Nährstoffe zur Verfügung stehen. Ergebnisse aus der Landwirtschaft belegen, dass Tiere, die mit enzymhaltigen Futtermitteln gefüttert werden, eine höhere Mastleistung zeigen.

Seit langem ist bekannt, dass in Pflanzen enthaltene Tannine Interaktionen mit den für die Ernährung essentiellen Makromolekülen einer Pflanze eingehen und diese dadurch anti-nutritive Eigenschaften aufweisen. Dies kann

bei Nutztieren zu Entwicklungsstörungen führen, da sie tanninhaltige pflanzliche Nahrung nicht vollständig verwerten können. Zusätzlich können Tannine krankhafte Leberschäden bei Nutztieren verursachen, in höheren Dosen sogar toxisch sein.

Tannine (frz.: *tannin* = Gerbstoff) sind weitverbreitete, leicht wasserlösliche, polyphenolische Verbindungen pflanzlichen Ursprungs mit einem Molekulargewicht zwischen 500 und 3000 D, wobei die auftretenden Verbindungen sehr vielfältig und oft sehr charakteristisch für ihre pflanzliche Herkunft sind. Sie können mit verschiedenen Proteinen (z. B. Enzymen) oder anderen Makromolekülen unlösliche Komplexe eingehen (s. Übersichtsartikel von Chung K., Wong, T., Wei, C und Huang Y, Critical Reviews in Food Science and Nutrition, 38(6), 421-464, 1998). Tannine sind im Holz, in der Rinde, in den Früchten, Blättern, Wurzeln vieler Pflanzen anzutreffen und stellen oft einen Abwehrmechanismus der Pflanzen gegen andere Organismen, wie Pilze und Bakterien, dar. Aufgrund der enormen Vielfalt werden Tannine in zwei Hauptgruppen unterteilt: Hydrolysierbare Tannine (Gallotannine, Ellagitannine) und kondensierte, komplexe Tannine. Die hydrolysierbaren Gallotannine und Ellagitannine repräsentieren dabei den weitaus größten Teil der bisher charakterisierten Tannine. Beide Gruppen wirken sehr toxisch auf die meisten Mikroorganismen.

Besonders für Herbivoren stellt die Komplexbildung von Tanninen mit pflanzlichen Proteinen ein Problem bei der Futterverwertung dar, da sie nicht in der Lage sind, diese zu spalten. So beeinträchtigen Tannine die Aufnahme von Nährstoffen und die Proteinspaltung durch die Bildung von körpereigenen Verdauungsenzymen oder durch direkte Organschädigung. Wird Tannin-haltiges Pflanzenmaterial in hohen Konzentrationen konsumiert, können hydrolysierbare Tannine Vergiftungen verursachen und so toxisch wirken. Bei monogastrischen Tieren können hydrolysierbare Tannine auch durch einen gesunden oder kranken Gastrointestinaltrakt aufgenommen werden und ernsthafte Leber- und Nierenschäden verursachen.

Doch nicht nur für Nutztiere bestehen negative Effekte durch Tannin. Durch das Ausscheiden dieser für die Tiere unverwertbaren Verbindungen treten auch für die Umwelt hohe Belastungen auf. So sind Tannine einerseits relativ beständig gegenüber biologischem Abbau, andererseits jedoch aufgrund ihrer polaren Eigenschaften sehr leicht wasserlöslich. Dadurch können sie über landwirtschaftliche Abwässer und über die Ausbringung tierischer Abfallprodukte (Gülle) bei der Düngung von Feldern leicht in den biologischen Kreislauf geraten. Durch Regen- und Sickerwasser gelangen sie ins Grundwasser und weiter in Bäche, Flüsse und Seen. Hier können sie bereits in geringen Konzentrationen (6.5 mg/l) zu Fischsterben führen. In höheren Konzentrationen wirken Tannine toxisch auf verschiedene Mikroorganismen, die im biologischen Ablauf der Abwasserreinigung involviert sind. So können z. B. methanogene und nitrifizierende Bakterien, die im biologischen Reinigungssystem eine Schlüsselrolle haben, inhibiert werden. Parallel werden auch die zur Kompostierung organischer Materialien benötigten Mikroorganismen durch Konzentrationen an Tannin (1-2%) beeinflusst.

Die Tannase, auch Tannin-Acyl-Hydrolase genannt (EC 3.1.1.20), 1786 von Scheele entdeckt, katalysiert die Hydrolyse von Ester- und Depsid-Bindungen in hydrolysierbaren Gallatestern, wie Gallotanninen, wobei Glukose und Gallussäure entsteht, die nicht mehr an Proteine bindet. Für eine Übersicht über Tannasen, deren Eigenschaften und Verwendungen, siehe Lehka, P. K und Lonsane B. K., Advances in Applied Microbiology, Vol. 44, 1997 und Bhat, T. K., Singh, B. und Sharma O. P., Biodegradation 9, 343-357, 1998.

Tannine bestehen in der Regel aus einer Glycosyleinheit (1), deren Hydroxyfunktionen teilweise oder vollständig mit Gallussäure (2) verestert ist.

**(1)** 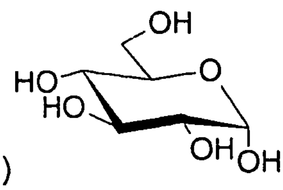

**(2)** 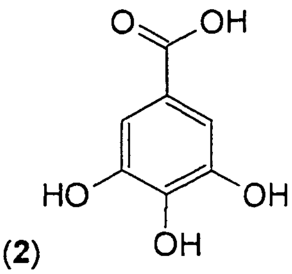

Um Gallotannin (3) vollständig in Glukose und mono-Gallusäure abzubauen, sind zwei Enzymaktivitäten notwendig; eine Esterase-Aktivität zur Abspaltung der Gallussäure-Reste vom Glukose-Molekül und eine Depsidase-Aktivität zum Zerlegen der Gallussäure-Reste in mono-Gallussäure-Moleküle.

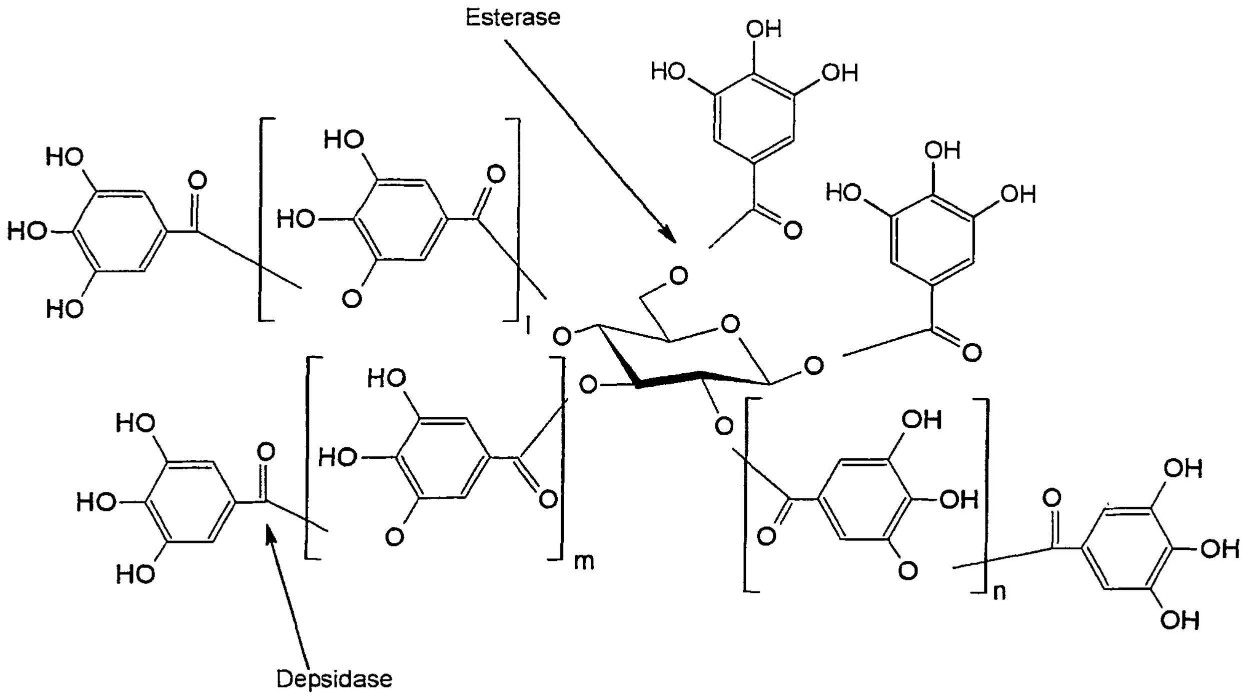


**(3)**

Bakterien und Pilze sind bekannte Tannase-Produzenten. Z. B. wurden *Aspergillus-Arten* als die Tannase-Produzenten bei Flüssig- und Fest-Fermentationen beschrieben (Deschamps A. M., Outuk, G. und Lebcault, J. M., J. of Fermentation Technology, 61, 55-59, 1983).

Tannasen aus *Aspergillus niger* (Gewinnung z: B. bei Freudenberg et al., Z. Physiol. Chem. 164, 262, 1927, beschrieben) und *Aspergillus oryzae* (siehe Yamada, K. et al., J. Ferment. Tech. 45, 233, 1967) werden bei der Herstellung von Instant-Tees eingesetzt (siehe z. B. US Patente No. 5,820,901 und US 6,482,450, sowie GB-1-1,249-932), da bei der Extraktion von schwarzem Tee mit heißen Wasser phenolhaltige Substanzen präzipitieren, die bei einem Aufguss von Tee-Extrakt mit kaltem Wasser stören würden. Die Tannase hydrolysiert diese sogenannte Tee-Creme, ein Komplex aus Polyphenolgallat und Koffein. Weitere Nutzen von Tannase, die bekannt sind, betreffen die Verwendung als Stabilisatoren von Malz-Polyphenolen, bei der Klärung von Bier und Fruchtsäften und in der Verhinderung der Phenol-Induzierten Oxidation von Äthylalkohol und Essigsäure zu Aldehyden in Wein- und Fruchtsäften. Tannasen werden auch zur Herstellung von Gallussäure eingesetzt, die ein Substrat für die chemische Synthese von Propylgallat und Trimethoprim (Anti-AIDS-Medikament) ist.

In der Lebensmittelherstellung findet Tannase wegen der hohen Kosten für die Herstellung dieses Enzyms wenig Anwendung. Diverse Publikationen im Stand der Technik befassen sich daher mit der Ausbeutesteigerung der Tannasegewinnung aus *Aspergillus niger.* Pinto et al. (Brazilian J. Microbiol., 2001, 32:24-26) haben einen anderen Weg gewählt und verschiedene *Aspergillus niger* Stämme auf ihre Tannase-Synthese untersucht. Die höchste gemessene Aktivität betrug 1,9 mU/g in der Stunde. Ramirez-Coronel (Ramirez-Coronel MA, Viniegra-Gonzalez G, Darvill A, Augur C (2003) A novel tannase from Aspergillus niger with ß-glucosidase activity. Microbiology 149: 2941-2946) reinigten eine Tannase aus *Aspergillus niger* Festphasen-Kulturen und fanden zwei Isoformen mit 180 und 90 kDa. Die Tannase wies einen isoelektrischen Punkt bei 3,8, ein Temperaturoptimum bei 60-70°C sowie ein pH-Optimum von 6,0 auf und kann Gallussäure sowohl von kondensierten als auch hydrolysierbaren Tanninen entfernen. Die spezifische Aktivität des gereinigten Enzyms betrug jedoch bei 50 mM Tanninsäure als Substrat nur 0,84 Einheiten/mg.

Es besteht daher ein großer Bedarf an einer Tannase, die ein vorteilhaftes Kosten-Leistungsverhältnis aufweist, d. h. geringere Kosten bei höherer Aktivität und größerer Stabilität, im Vergleich zu Tannasen aus dem Stand der Technik.

Diese Aufgabe wird durch die Bereitsttellung der folgenden Nukleinsäuren gelöst:

1. Nukleinsäure, umfassend eine aus der folgenden Gruppe ausgewählte Nukleinsäuresequenz:

   a) Nukleinsäuresequenz nach SEQ ID NO: 1;
   b) Nukleinsäuresequenz, die für das Polypeptid nach SEQ ID NO: 3 kodiert.

Darüber hinaus stellt die vorliegende Erfindung folgende Gegenstände bereit:

2. Expressionskonstrukt, umfassend eine Nukleinsäure gemäß Punkt 1, wobei die Nukleinsäuresequenz operativ mit einer regulatorischen Nukleinsäuresequenz verbunden ist, die Signale für die Transkriptions- und/oder Translationssteuerung enthält und die Expression der Nukleotidsequenz in der Wirtszelle kontrolliert.

3. Wirtszelle, die das Expressionskonstrukt gemäß Punkt 2 umfasst.

4. Wirtszelle nach Punkt 3, die eine prokaryotische Zelle ist.

5. Wirtszelle nach Punkt 3, die eine eukaryotische Zelle ist.

6. Wirtszelle nach Punkt 3 oder 5, die eine nicht-menschliche Zelle ist.

7. Wirtszelle nach Punkt 5 oder 6, die eine Hefe ist.

8. Wirtszelle nach Punkt 7, die Arxula adeninivorans ist.

9. Verfahren zum Herstellen eines Polypeptids mit Tannase-Aktivität, umfassend die folgenden Schritte: a) Kultivieren einer Wirtszelle gemäß einem der Punkte 3-8 unter für eine Expression des Polypeptids geeigneten Bedingungen, b) Anreichern des Polypeptids.

10. Verfahren gemäß Punkt 9, wobei die Wirtszelle Arxula adeninivorans ist und das Polypeptid isoliert und aufgereinigt wird.

11. Verfahren gemäß einem der Punkte 9 oder 10, wobei das Polypeptid in hohen Mengen in das Kulturmedium sezemiert wird.

12. Polypeptid mit Tannase-Aktivität, herstellbar mittels eines Verfahrens gemäß einem der Punkte 9, 10 oder 11.

13. Polypeptid mit Tannase-Aktivität, umfassend die in SEQ ID NO: 3 angegebene Sequenz, umfassend optional weitere aminoterminale Methionine.

14. Polypeptid, das durch die Nukleinsäuren gemäß Punkt 1 kodiert wird.

15. Antikörper oder Fragment davon, der spezifisch an ein Polypeptid gemäß einem der Punkte 12-14 bindet.

16. Antikörper gemäß Punkt 15, der ein monoklonaler Antikörper ist.

17. Hybridoma-Zelllinie, die einen monoklonalen Antikörper gemäß Punkt 16 bildet.

18. Biosensor, der ein Polypeptid gemäß einen der Punkte 12-14 oder ein Polypeptid, gewonnen mittels des Verfahrens gemäß Punkt 9-11, umfasst.

19. Unlösliches Trägermaterial, an das ein Polypeptid gemäß einem der Punkte 12-14 oder ein Polypeptid, gewonnen mittels des Verfahrens gemäß Punkt 9-11, gebunden ist.

20. Enzymatische Zusammensetzung, die ein Polypeptid gemäß einem der Punkte 12-14 oder ein Polypeptid, gewonnen mittels des Verfahrens gemäß Punkt 9-11, umfasst.

21. Transgene Pflanze, enthaltend eine Nukleinsäure gemäß Punkt 1.

22. Transgene Pflanze, die das Polypeptid gemäß einem der Punkte 12-14 über-exprimiert.

23. Verwendung einer transgenen Pflanze gemäß Punkt 21 oder 22 bei der Herstellung von Tannin-reduzierten Nahrungsmitteln oder Futtermitteln.

24. Verfahren zur Detektion oder Quantifizierung von Tannase in einer Probe, das das Kontaktieren einer Probe,

von der angenommen wird, dass sie Tannase enthält, mit einem spezifischen Tannase-Antikörper oder Fragment desselben, gemäß einem der Punkte 15-16 umfasst, sowie die Detektion der Bindung des Antikörpers oder Fragments.

25. Verfahren zur Detektion oder Quantifizierung von Tanninen in einer Probe, umfassend: (i) das Kontaktieren einer Probe, von der angenommen wird, dass sie Tannine enthält, mit dem Biosensor gemäß Punkt 18, sowie (ii) die Detektion von Tanninen.

26. Verfahren zur Reduktion der Menge an Tanninen in einer Zusammensetzung, umfassend das Kontaktieren der Zusammensetzung mit einem Polypeptid gemäß einem der Punkte 12-14 oder einem Polypeptid, gewonnen mittels des Verfahrens gemäß Punkt 9-11, mit der Wirtszelle gemäß einem der Punkte 3-8, mit dem unlöslichen Trägermaterial gemäß Punkt 19 oder mit der enzymatischen Zusammensetzung gemäß Punkt 20.

27. Verfahren gemäß Punkt 26 zur Reduktion der Menge an Tanninen in Nahrungsmitteln, Futtermitteln und/oder in einer mit diesen Stoffen kontaminierten Zusammensetzung.

28. Verfahren gemäß Punkt 27 zur Reduktion der Menge an Tanninen in landwirtschaftlichen und industriellen Abwässern.

29. Verfahren gemäß Punkt 27 zur Verwendung innerhalb der Chemie-, Pharma-, Nahrungsmittel-, Waschmittel-, Leder-, Papier- und/oder Kosmetikindustrie.

30. Verwendung des Polypeptids gemäß einem der Punkte 12-14, der Wirtszelle gemäß einem der Punkte 3-9, des unlöslichen Trägermaterials gemäß Punkt 19 oder der enzymatischen Zusammensetzung gemäß Punkt 20 zur Reduktion der Menge an Tanninen in einer Zusammensetzung.

31. Verwendung gemäß Punkt 30, wobei die Tannin-haltige Zusammensetzung ein Futtermittel, Nahrungsmittel oder eine mit Tannin kontaminierte Zusammensetzung ist.

32. Verwendung gemäß Punkt 31 zur besseren Futterverwertung durch Nutztiere.

33. Verwendung gemäß Punkt 30, wobei die Tannin-haltige Zusammensetzung landwirtschaftliches oder industrielles Abwasser ist.

34. Verwendung des Polypeptids gemäß einem der Punkte 12-14 oder des Polypeptids, gewonnen mittels des Verfahrens gemäß Punkt 9-11 in der Chemie-, Pharma-, Nahrungsmittel-, Waschmittel-, Papier- und/oder Kosmetikindustrie.

35. Verwendung des Polypeptids gemäß einem der Punkte 12-14 oder des Polypeptids, gewonnen mittels des Verfahrens gemäß Punkt 9-11, zur Herstellung von Gallussäure.

36. Verwendung des Biosensors gemäß Punkt 18 zur Detektion von Tanninen in Abwässern, Nahrungsmitteln oder Futtermitteln.

Nachfolgend werden einige der verwendeten Ausdrücke erklärt und definiert.

<u>Nukleinsäure</u>

Der Ausdruck "Nukleinsäure" oder "Nukleinsäuremolekül" bezieht sich auf eine DNA, RNA, oder PNA, die entweder durch Isolierung aus genomischer DNA oder aus cDNA nach bekannten Standardverfahren (Sambrook et *al*., 1989) erhalten und aufgereinigt oder künstlich durch bekannte Verfahren (Sambrook et *al*., 1989), wie z. B. der Oligonukleotid-Synthese, generiert wird oder als ribosomale RNA oder mRNA aus dem Organismus Isoliert oder als PNA synthetisiert wird. Eine "PNA" ist dabei eine Peptidnukleinsäure, bei der anstelle, des Phosphorsäurerückgrates der DNA 2-Aminoethylglycin-Bindungen auftreten. Beschrieben werden Nukleinsäuren, in denen bis zu 20% der Nukleotide in 10 aufeinanderfolgenden Nukleotiden, bevorzugt jedoch 1 Nukleotid aus einem Block von 10 aufeinanderfolgenden Nukleotiden durch Nukleotide (z. B. Inosin, etc.) ersetzt sind die In Hefen nicht natürlich vorkommen. Die DNA oder RNA und Polymere davon können entweder als Einzel- oder Doppelstrang, offen oder geschlossen, zirkulär bzw. linear vorkommen.

Die beschriebenen Nukleinsäuren können weiterhin Modifikationen enthalten, die die Erzeugung eines direkt oder indirekt nachweisbaren Signals erlauben. Dem Fachmann sind dabei folgende Modifikationen bekannt

(i) radioaktive Modifikationen, d. h. radioaktive Phosphorylierung oder radioaktive Markierung mit Schwefel, Wasserstoff, Kohlenstoff, Stickstoff,
(ii) farbige Gruppen (z. B. Digoxygenin, etc.),
(iii) fluoreszierende Gruppen (z. B. Fluorescein, etc.),
(iv) chemolumineszierende Gruppen,
(v) Gruppen zur Immobilisierung an einerfesten Phase (z. B. Biotin, Streptag, Antikörper, Antigene, etc.) und/oder
(vi) Gruppen, die eine Indirekte oder direkte Reaktion, mit Hilfe von Antikörpern, Antigenen, Enzymen und/oder Substanzen mit Affinität zu Enzymen oder Enzymkomplexen erlauben,

oder eine Kombination von Modifikationen gemäß zwei oder mehr der unter (i) bis (vi) genannten Modifikationen. Als "Modifikation" werden im Sinne dieser Erfindung direkt oder Indirekt nachweisbare Gruppen oder Gruppen zur Immobilisierung an eine feste Phase bezeichnet, die an die Nukleinsäure angehängt sind. Direkt nachweisbar sind Metallatome, radioaktive, farbige oder fluoreszierende Gruppen. Indirekt nachweisbar sind immunologisch oder enzymatisch nachweisbare Gruppen, wie z. B. Antigene und Antikörper, Haptene oder Enzyme bzw. enzymatisch wirkende Teile von Enzymen. Diese indirekten Gruppen werden in nachfolgenden Reaktionen nachgewiesen. Bevorzugt sind Haptene, die an ein Oligonukleotid gekoppelt sind, und die man in einer anschließenden Antikörperreaktion nachweist

Ebenfalls beschriebenen wird eine besondere Nukleinsäuresequenz die implizit auch konservativ veränderte Varianten von ihr umfasst (z.B. die nach dem degenerierten genetischen Code möglichen Codon-Substitutionen). Degenerierte Codon-Substitutionen können z. B. erhalten werden, in dem Sequenzen hergestellt werden, in denen die dritte Position von einer oder mehreren (oder allen) ausgewählten Codons durch "mixed-base" und/oder Desoxyinosinresten ausgetauscht wird (Batzer et al., Nucleic Acid Res. 19, 5081, 1991). Der Ausdruck "Nukleinsäure" wird austauschbar mit Gen, cDNA und durch ein Gen kodierte mRNA verwendet.

Nulkeinsäuresequenz

Eine hierin beschriebene Nukleinsäuresequenz oder ein hierin beschriebenes Nukleinsäuremolekül bezieht sich auf DNA-, cDNA-oder RNA-Sequenzen bzw. -Moleküle. Der Ausdruck umfasst Sequenzen und Moleküle, die irgendeines der bekannten Basen-Analoga von DNA und RNA, enthalten.

So wie, aber nicht limitiert auf: 4-Acetylcytosine, 8-Hydroxy-N6-Methyladenosin, Aziridinylcytosin, Pseudoisocytosin, 5-(Carboxyhydroxymethyl)uracil, 5-Fluorouracil, 5-Bromouracil, 5-Carboxymethylaminomähtyl-2-Thiouracil, 5-Carboxymethylaminomähtyluracil, Dihydrouracil, Inosin, $N_6$-Isopentenyladenin, 1-Methyladenin, 2,2-Dimethyl-Guanin, 2-Methyladenin, 2-Methylguanin, 3-Methylcytosin, 5-Methylcytosin, $N_8$-Methyladenin, 7-Methylguanin, 5-Methylaminomethyluracil, 5-Methoxyamino-Methyl-2-Thiouracil, beta-D-Mannosylqueosin, 5'-Methoxycarbonyl-Methyluracil, 5-Methoxyuracil, 2-Methylthio-$N_6$-Isopentenyladenin, Uracil-5-Oxyessigsauremethylester, Uracil-5-Oxoessigsäure, Oxybutoxosin, Pseudouracil, Queosin, 2-Thiocytosin, 5-Methyl-2-Thiouracil, 2-Thiouracil, 4-Thiouracil, 5-Methyluracil, N-Uracil-5-Oxyessigsäuremethylester, Uracil-5-Oxyessigsäure und 2,6-Diaminopurin.

Polypeptid, Peptid, Protein

Die Ausdrücke "Polypeptid", "peptid" und "Protein" werden hier untereinander austauschbar verwendet und beziehen sich auf ein Polymer von Aminosäureresten. Aminosäurepolymere, in denen eine oder mehrere Aminosäuren durch ein künstliches chemisches Analogon der korrespondierenden natürlich vorkommenden Aminosäure ersetzt sind, werden ebenso beschrieben. Vorzugsweise ist das Polypeptid im wesentlichen frei von irgendwelchen anderen verunreinigenden Polypeptiden oder anderen Verunreinigungen, die in der natürlichen Umgebung vorkommen und die bei der Anwendung im Lebensmittelbereich, der Abwasserreinigung, oder einer therapeutischen, diagnostischen oder prophylaktischen Anwendung stören würden.

Tannase- und/oder Lipase-Polypeptid

Der Ausdruck "Tannase- und/oder Lipase-Polypeptid bzw. "Protein mit Tannase- und/oder Lipaseaktivität" bezieht sich auf ein Polypeptid, das die Aminosäuresequenz von SEQ ID NO: 3 oder 4 umfasst und Tannase- und/oder Lipase-Aktivität aufweist, sowie damit verwandte Polypeptide. Verwandte Polypeptide schließen Derivate und Varianten, also beispielsweise Polypeptide mit mindestens 70%-iger Identität, Orthologe, Splice- und Prozessierungsvarianten und Fragmente der Polypeptide gemäß SEQ ID NO:3 oder 4 ein. Tannasen und/oder Lipasen können reife Polypeptide sein, wie hier definiert, oder aber Vorläuferpolypeptide und können oder können nicht einen

aminoterminalen Methioninrest aufweisen, je nach der Herstellungsmethode.

Reifes Tannase- und/oder Lipase-Polypeptid

Als "reife Tannase bzw. Lipase" wird ein Tannase- und/oder Lipase-Polypeptid bezeichnet, dem die Leadersequenz fehlt. Ein exemplarisches reifes Tannase-Polypeptid umfasst die Aminosäuren 29 bis 448 nach SEQ ID NO: 3, die Leadersequenz entspricht demnach den Aminosäuren 1-28.

Tannase-bzw. Lipase-Aktivität

Unter Tannase- und/oder Lipase-Aktivität wird eine Tannin- bzw. Lipid-spaltende Aktivität verstanden, die auf Ester gerichtet ist Diese Aktivitäten können sich auch erheblich (z. B. wegen veränderter $k_M$-Werte oder in der $v_{max}$) von den maximal erreichbaren Aktivitäten der Tannase und/oder Lipase nach SEQ ID NO: 3 oder SEQ ID NO:4 unterschelden. Die Tannase-Aktivität kann dabei nach Sharma et al. (Sharma S, Bhat TK, Dawra RK (2000) A spectrophotometric method for assay of tannase using Rhodanine. Anal. Biochem. 279: 85-89) bestimmt werden, der Lipase-Test ist weiter unten beschrieben.

Allel

Der Ausdruck "Allel" bezieht sich auf eine von zahlreichen möglichen, natürlich vorkommenden alternativen Formen eines Genes, die an einem bestimmten Ort auf dem Chromosom eines Organismus oder einer Population von Organismen lokalisiert ist.

Derivate

Der Ausdruck "Derivate" bezieht sich auf Polypeptide, die durch chemische Synthese oder Expression in geeigneten Wirbelorganismen ein verändertes Glycosylierungsmuster aufweisen. Die beschriebenen Tannase- und/oder Lipase-Polypeptide schließen Glycosylierungsderivate ein, wobei die Zahl und/oder die Art der Glycosylierungsstellen im Vergleich zu den Sequenzen nach SEQ ID NO: 3 oder 4 verändert wurde. Z.B. umfassen die beschriebenen Tannase- und/oder Lipase-Derivate im Vergleich zu den Sequenzen nach SEQ ID NO: 3 oder 4 eine größere oder kleinere Zahl an N-verknüpften Glycosylierungsstellen. Eine N-verknüpfte Glycosyllerungsstelle ist durch die Sequenz Asn-X-Ser oder Asn-X-Thr charakterisiert, wobei X ein beliebiger Aminosäurerest, mit Ausnahme von Prolin, seinkann. Die Substitution(en) von Aminosäureresten zur Herstellung dieser Sequenz liefert eine potentiell neue Stelle zur Anknüpfung einer Kohlenhydratkette. Alternativ führen Substitutionen, die diese Sequenz eliminieren, zu einer Deletion von existierenden N-verknüpften Kohlenhydratketten. Ferner wird eine Neuanordnung der N-verknüpften Kohlenhydratketten beschrieben, wobei ein oder mehrere N-verknüpfte Glycosyllerungsstellen (üblicherweise solche, die natürlich vorkommen) entfernt werden und ein oder mehrere neue N-verknüpfte Orte generiert werden.

Der Begriff "Derivate" beschreibt weiterhin chemisch modifizierte Polypeptide, z. B. PEGylierte oder HESylierte Derivate.

Variante

Der Ausdruck "Varianten" bezieht sich auf ein Polypeptid, das eine Aminosäuresequenz umfasst, die eine oder mehrere Aminosäuresubstitutionen, Deletionen (interne Deletionen und/oder terminale Deletionen), und/oder insertionen (wie interne Insertionen und/oder terminale insentionen. wie z. B. in einem Fusionspolypeptide) im Vergleich zu der Aminosäuresequenz nach SEQ ID NO: 3 und 4 aufweist (mit oder ohne Leadersequenz). Varianten können natürlich vorkommen oder rekombinant oder durch chemische Synthese hergestellt werden. Beispielsweise weisen die Varianten Substitutionen von 1 bis 3, 1 bis 5, 1 bis 10, 1 bis 15, 1 bis 20, 1 bis 25, 1 bis 50, 1 bis 75, 1 bis 100 oder mehr als 100 Aminosäuren auf, und/oder insertionen und/oder Deletionen im gleichen Umfang, wobei die Substitutionen konservativ oder nicht-konservativ oder eine Kombination davon sein können. Varianten sind z. B. Cystein-Varianten, wobei ein oder mehrere Cystein-Reste durch eine andere Aminosäure (z. B. Serin) ersetzt werden oder die Cystein-Reste entfernt werden. Cystein-Varianten sind nützlich, wenn Tannase- und/oder Lipase-Polypeptide in eine biologisch aktive Form zurückgefaltet werden müssen, wie nach eine isolierung aus unlöslichen Einschlusskörpern. Cystein-Varianten weisen im Allgemeinen weniger Cysteinreste als die native Form auf und haben üblicherweise eine gerade Zahl an Cysteinen, um interaktionen, die aus ungepaarten Cysteinen resultieren, zu minimieren.

Splice-Variante

Der Ausdruck "Splice-Variante" bezieht sich auf ein Nukleinsäuremolekül, für gewöhnlich RNA, die durch eine alternative Prozessierung der intronsequenzen in einem RNA-Transkript von Aminosäuresequenzen nach SEQ ID NO: 3 oder 4 generiert wird.

Fragment

Der Ausdruck "Fragment" bezieht sich auf ein Polypeptid, mit einer Verkürzung am Aminoterminus (mit oder ohne Leadersequenz) und/oder einer Verkürzung am Carboxyterminus.

Fusionspolypeptid

Der Ausdruck "Fusionspolypeptid" bezieht sich auf ein durch Fusion einer oder mehrerer Aminosäuren (z. B. eines heterologen Peptides oder Polypeptides) mit den Amino- oder Carboxyterminus einer Tannase und/oder Lipase gebildetes Polypeptid.

Ortholog

Der Ausdruck "Ortholog" bezieht sich auf ein Polypeptid einer anderen Art, die zur entsprechenden Nuklein- oder Aminosäure korrespondiert Z. B. werden Tannase-Polypeptide aus Maus und Mensch als Orthologe voneinander betrachtet

Identität

In der nachfolgenden Beschreibung soll unter "Identität" der Grad der Verwandtschaft zwischen zwei oder mehr Nukleinsäuren, Peptiden oder Polypeptiden verstanden werden, der durch die Übereinstimmung zwischen den Sequenzen mittels bekannter Verfahren, z. B. der computergestützten Sequenzvergleiche (Basic local alignment search tool., S. F. Altschul et al., J. Mol. Biol. 215, 403-410, 1990) bestimmt werden kann. Der Prozentsatz der "Identität" ergibt sich aus dem Prozentsatz identischer Bereiche in zwei oder mehr Sequenzen unter Berücksichtigung von Lücken oder anderen Sequenzbesonder heiten.

Bevorzugte Verfahren zur Bestimmung der Identität erzeugen zunächst die größte Übereinstimmung zwischen den untersuchten Sequenzen. Computerprogramme zur Bestimmung der Identität zwischen zwei Sequenzen, sind jedoch nicht beschränkt auf, das GAG Programmpaket, einschließlich GAP (Devereux, J., et al., Nucleic Acid Research 12(12), 287, 1984); Genetics Computer Group Unlversity of Wisconsin, Madison (WI)); BLASTP, BLASTN, und FASTA (Altschul, S. et al., J. Mol. Biol. 215, 403-410, 1999). Das BLASTX Programm kann vom National Centre of Biotechnology Information (NCBI) und aus weiteren Quellen bezogen werden (BLAST Handbuch, Altschul S., et al., NCB NLM NIH Bethesda MD 20894; Altschul, S., et al., Mol. Biol. 215, 403-410, 1990). Auch der bekannte Smith Waterman-Algorithmus kann zur Bestimmung der Identität herangezogen werden.

Bevorzugte Parameter für den Aminosäuresequenz-Vergleich umfassen die nachstehenden:

| | |
|---|---|
| Algorithmus: | Needleman und Wunsch, J. Mol. Biol. 48, 443-453, 1970; |
| Vergleichsmatrix: | BLOSUM 62 aus Henikoff und Henikoff, PNAS USA 89, 19, 10915-10919 |
| Lückenwert (GAP Penalty): | 12 |
| Lückenlängen-Wert (GAP Length Penalty): | 4 |
| Ähnlichkeits-Schwellenwert (Treshold of Similarity): | 0 |

Das GAP-Programm ist zur Verwendung mit vorstehenden Parametern geeignet. Die vorstehenden Parameter sind die Standardparameter (default parameters) für Aminosäure-Sequenzvergleiche, wobei Lücken an den Enden den Identitätswert nicht verringern. Bei sehr kurzen Sequenzen im Vergleich zur Referenzsequenz kann es weiterhin notwendig sein, den Erwartungswert (expectation value) auf bis zu 100 000 zu erhöhen und gegebenenfalls die Wortlänge (word size) auf bis zu 2 zu verkleinern.

Bevorzugte Parameter für den Nukleinsäuresequenz-Vergleich umfassen die nachstehenden:

| | |
|---|---|
| Algorithmus: | Needleman und Wunsch, J. Mol. Biol. 48, 443-453 (1970); |

(fortgesetzt)

| | |
|---|---|
| Vergleichsmatrix: | Übereinstimmung (matches) = +10, Nicht-Übereinstimmung = 0 |
| Lückenwert (GAP Penalty): | 50 |
| Lückenlängen-Wert (GAP Length Penalty): | 3 |

Das GAP- Programm ist zur Verwendung mit vorstehenden Parametem geeignet. Die vorstehenden Parameter sind die Standardparameter (default parameters) für Nukleinsäure-Sequenzvergleiche.

Weitere beispielhafte Algorithmen, Lücken-Öffnungs-Werte (Gap opening penalties), Lückenausdehnungs-Werte (gap extension penalties), Vergleichsmatrizen einschließlich der im Programm-Handbuch, Wisconsin-Paket, Version 9, September 1997, genannten, können verwendet werden. Die Auswahl wird von dem durchzuführenden Vergleich abhängen (z. B. DNA/DNA, Protein/Protein, Protein/DNA) und weiterhin davon, ob der Vergleich zwischen Sequenzpaaren, wobei GAP oder Best Fit bevorzugt sind, oder zwischen einer Sequenz und einer umfangreichen Sequenz-Datenbank, wobei FASTA oder BLAST bevorzugt sind, durchgeführt wird. Eine mit dem oben genannten Algorithmus ermittelte Übereinstimmung von 70% wird hierin als 70% identität bezeichnet Entsprechendes gilt für höhere Identitätsgrade.

Aminosäuresubstitution (konservativ und nicht-konservativ)

Der Ausdruck "konservative Aminosäuresubstitution" bezieht sich auf einen Ersatz einer nativen Aminosäure durch eine andere, so dass nur wenig oder keine Änderung der Polarität oder Ladung an der Position der Aminosäure eintritt. Z. B. resultiert eine konservative Substitution aus dem Ersatz eines nicht-polaren Restes in einem Polypeptid durch irgendeinen anderen nicht-polaren Rest. Weiterhin kann irgendein nativer Rest auch durch Alanin ersetzt werden, wie für die "alanine scanning mutagenesis" beschrieben (Cunnigham et al., Science 244, 1081-1085, 1998). Allgemeine Regeln für konservative Aminosäuresubstitutionen sind in der Tabelle I aufgeführt.

**Tabelle I**
Konservative Aminosäuresubstitutionen

| Original-Reste | Beispielhafte Substitutionen | Bevorzugte Substitutionen |
|---|---|---|
| Ala | Val, Leu Ile | Val |
| Arg | Lys, Gin, Asn | Lys |
| Asn | Gln, His, Lys, Arg | Gln |
| Asp | Glu | Glu |
| Cys | Ser | Ser |
| Gln | Asn | Asn |
| Glu | Asp | Asp |
| Gly | Pro, Ala | Ala |
| His | Asn, Gin, Lys, Arg | Arg |
| Ile | Leu, Val, Met, Ala Phe, Norleucine | Leu |
| Leu | Norleucine, Ile Val, Met, Ala, Phe | Ile |
| Lys | Arg, Gln, Asn | Arg |
| Met | Leu, Phe, Ile | Leu |
| Phe | Leu, Val, Ile, Ala Tyr | Leu |
| Pro | Leu, Val, Ile, Ala Tyr | Leu |
| Pro | Ala | Ala |
| Ser | Thr | Thr |
| Thr | Ser | Ser |
| Trp | Tyr, Phe | Tyr |
| Tyr | Trp, Phe, Thr, Ser | Phe |
| Val | Ile, Met, Leu, Phe | Leu |

(fortgesetzt)

Konservative Aminosäuresubstitutionen

| Original-Reste | Beispielhafte Substitutionen | Bevorzugte Substitutionen |
|---|---|---|
| | Ala, Norleucine | |

Konservative Aminosäuresubstitutionen können auch nicht natürlich vorkommende Aminosäurereste umfassen, die typischerweise durch chemische Peptidsynthese und nicht durch Synthese in biologischen Systemen eingefügt werden. Diese schließen Peptidomimetika und andere revertierten und reversen Formen von Aminosäure-Einheiten ein. Von den konservativen Modifikationen in der Aminosäuresequenz (und den korrespondierenden Modifikationen in der kodierenden Nukleinsäuresequenz) wird angenommen, dass sie Tannase- und/oder Lipase-Polypeptide produzieren, die ähnliche funktionelle und chemische Charakteristika, wie die natürlich vorkommenden Tannase- und/oder Lipase-Polypeptide aufweisen.

Der Ausdruck "nicht-konservative Substitution" bezieht sich auf einen Ersatz eines Mitglieds aus einer Aminosäureklasse (hydrophobe, neutrale hydrophile, saure, basische, aromatische Aminosäure und solche, die die Kettenorientierung beeinflussen) durch ein Mitglied aus einer anderen Aminosäureklasse. Solche ausgetauschten Reste können in Regionen der Tannase- und/oder Lipase-Polypeptide eingefügt werden, die homolog mit Tannase- und/oder Lipase-Polypeptid Orthologen sind, die nicht aus Hefe stammen, oder in nicht-homologen Regionen des Moleküls.

Bei der Einführung von Aminosäuresubstitutionen muss der Hydropathie-Index berücksichtigt werden. Jeder Aminosäure ist ein Hydropathieindex auf der Basis ihrer Hydrophobizität und Ladungscharakteristika zugeordnet. Es sind dies: Isoleucin (+ 4.5), Valin (+ 4,2), Leucin (+3,8), Phenlyalanin (2,8), Cystein/Cystin (+2.5), Methionin (+1.9), Alanin (1,8), Glycin (-0,4), Threonin (-0,7), Serin (-0,8), Tryptophan (-0,9), Tyrosin (-1,3), Prolin (-1,6), Histidin (-3,2), Glutamat (-3,5), Glutamin (-3,5), Aspartat (-3,5), Asparagin (-3,5), Lysin (-3,9) und Arginin (-4.5).

Die Bedeutung des hydropathischen Aminosäureindex für die Übertragung der interaktiven biologischen Funktion auf ein Protein ist dem Fachmann auf seinem Gebiet bekannt (Kyte et al., J. Mol. Biol., 157, 105-131, 1982). Es ist bekannt, dass bestimmte Aminosäuren durch andere mit einem ähnlichen Hydropathie-Index oder "score" ausgetauscht werden können und die biologische Aktivität dadurch wenig beeinflusst wird. Bei der Herstellung von solchen auf dem Hydropathie-Index beruhenden Veränderungen, sind Substitutionen von Aminosäuren, deren Hydropathie-Indizes sich um +/-2 unterscheiden, bevorzugt, solche zwischen +/- 1 sind besonders bevorzugt und solche zwischen +/- 0.5 sind noch mehr bevorzugt.

Die Substitution ähnlicher Aminosäuren kann alternativ auf der Basis ihrer Hydrophilität vorgenommen werden, insbesondere, wenn dadurch ein biologisch äquivalentes Protein oder Peptid hergestellt wird, das für immunologische Zwecke verwendet werden soll.

Die folgenden Hydrophilie-Werte wurden den einzelnen Aminosäuren zugeordnet: Arginin (+3,0), Lysin (+3,0), Aspartat (+3,0 +/-1), Glutamat (+ 3,0 +/- 1), Serin (+ 0,3), Asparagin (+ 0,2), Glutamin (+ 0,2), Glycin (0), Threonin (-0,4), Prolin (- 0,5 +/- 1), Alanin (-0,5), Histidin (-0,5), Cystein (-1,0), Methionin (-1,3), Valin (-1,5), Leucin (1,8), Isoleucin (-1,8), Tyrosin (-2,3), Phenylalanin (-2,5), Tryptophan (-3,4). Bei der Herstellung von solchen auf ähnlichen Hydrophilie-Werten beruhenden Veränderungen, sind Substitutionen von Aminosäuren, deren Hydrophilie-Indices sich um +/-2 unterscheiden, bevorzugt, solche zwischen +/- 1 sind besonders bevorzugt und solche zwischen +/- 0.5 sind noch mehr bevorzugt. Man kann auch Epitope von primären Aminosäuresequenzen auf der Basis der Hydrophilie ermitteln. Diese Regionen werden auch als "Epitop-Kernregionen" bezeichnet.

Aminosäuresubstitutionen können z. B. verwendet werden, um wichtige Reste innerhalb der Tannase und/oder Lipase zu ermitteln oder um die Affinität der Tannase- und/oder Lipase-Polypeptide, die hier beschrieben sind, zu erhöhen und zu erniedrigen.

<u>Promotor</u>

Unter einem "Promotor" wird eine Anordnung von Nukleinsäure-Kontrollsequenzen verstanden, die die Transkription einer Nukleinsäure steuern. Der Ausdruck "Promotor" umfasst die notwendigen Nukleinsäuresequenzen in der Nähe des Transkriptionsstarts ein, so wie im Falle eines Polymerase-Typ-II Promotors, ein TATA Box-Element. Ein Promotor schließt optional auch distale "Enhancer" oder "Repressor"-Elemente ein, die auch mehrere Tausend Basenpaare vom Transkriptionsstart entfernt sein können. Ein "konstitutiver" Promotor ist ein Promotor, der unter den meisten experimentellen oder Entwicklungsbedingungen aktiv ist. Ein "induzierbarer" Promotor ist ein Promotor, der einer Kontrolle durch z. B. experimentelle Bedingungen oder die Entwicklung des Organismus unterliegt.

Operativ verknüpft

Der Ausdruck "operativ verknüpft" bezieht sich hier auf eine Anordnung flankierender Sequenzen, wobei die flankierenden Sequenzen so konfiguriert oder angeordnet sind, dass sie ihre übliche Funktion ausüben. Somit ist eine flankierende Sequenz, die operativ an eine kodierende Sequenz verknüpft ist, fähig, die Replikation, Transkription, und/oder Translation der kodierenden Sequenz zu beeinflussen. Eine kodierende Sequenz wird z. B. operativ an einen Promotor geknüpft, wenn der Promotor geeignet ist, die Transkription der kodierenden Sequenz zu kontrollieren. Eine flankierende Sequenz braucht nicht fortlaufend zu der kodierenden Sequenz sein, so lange sie ihre korrekte Funktion ausübt. So können z. B. unübersetzte, nicht-transkribierte Sequenzen zwischen einer Promotorsequenz und der kodierenden Sequenz vorliegen, die Promotorsequenz kann aber immer noch als "operativ verknüpft" mit der kodierenden Sequenz angesehen werden.

Vektor

Der Ausdruck "Vektor" bezieht sich auf irgendein Molekül (z. B. Nukleinsäure, Plasmid oder Virus), das zum Transfer von kodierender Information in eine Wirtszelle geeignet ist.

Expressionskonstrukt

Der Ausdruck "Expressionskonstrukt" bezieht sich auf einen Vektor, der zur Verwendung in einer Wirtszelle geeignet ist und Nukleinsäuresequenzen aufweist, die die Expression heterologer Nukleinsäuresequenzen steuern und/oder kontrollieren. Typischerweise enthält das "Expressionskonstrukt" einen Promotor.

Wirtszelle

Der Ausdruck "Wirtszelle" bezieht sich auf eine Zelle, die transformiert wurde oder die geeignet ist, mit eine Nukleinsäuresequenz transformiert zu werden und dann das ausgewählte Gen von Interesse exprimiert. Der Ausdruck schließt die Nachkommenschaft der Elternzelle ein, unabhängig davon, ob die Nachkommenschaft der Elternzelle morphologisch oder genetisch identisch zur Originalzelle ist, solange das ausgewählte Gen vorhanden ist.

Transfektion

Der Ausdruck "Transfektion" bezieht sich auf die Aufnahme fremder oder exogener DNA durch eine Zelle. Eine große Zahl an Transfektionstechniken ist dem Fachmann auf seinem Gebiet bekannt (siehe Sambrook, Fritsch & Maniatis, Molecular Cloning: A Laboratory Manual, 2nd Ed., Cold Spring Harbor Laboratory, Cold Spring Harbor, N. Y., 1989). Solche Techniken können verwendet werden, um eine oder mehrere exogene DNA-Einheiten in geeignete Wirtszellen einzuführen.

Transformation

Der Ausdruck "Transformation", so wie er hier verwendet wird, bezieht sich auf eine Veränderung in den genetischen Charakteristika einer Zelle. Eine Zelle gilt dann als transformiert, wenn sie im Vergleich zu ihrem nativen Status genetisch modifiziert ist. Auf eine Transfektion oder Transduktion folgend, kann die transformierte DNA mit der der Zelle durch physikalische Integration in ein Chromosom der Zelle rekombinieren. Die DNA kann transient als ein episomales Element, ohne repliziert zu werden, erhalten bleiben oder kann unabhängig als ein Plasmid replizieren. Von einer Zelle wird angenommen, dass sie stabil mit einer DNA transformiert wurde, wenn die DNA mit der Zellteilung repliziert wird.

Hybridisierung

Unter einer Hybridisierung wird die Doppelstrangbildung zweier identischer oder ähnlicher Nukleinsäurefragmente (DNA, RNA, PNA) verstanden. Von spezifischer Hybridisierung spricht man, wenn die Hybridisierung unter stringenten Bedingungen durchgeführt wird und zu einem stabilen Nukleinsäurehybrid führt. Im Sinne dieser Beschreibung weist das Merkmal "Sequenz, die mit einer Sequenz nach SEQ ID NO: 1 oder 2 spezifisch hybridisiert" auf eine Sequenz hin, die unter moderat-stringenten oder hoch-stringenten Bedingungen mit der Sequenz nach SEQ ID NO: 1 oder 2 hybridisiert.

Hoch-stringente Bedingungen

Der Ausdruck "hoch-stringente Bedingungen" bezieht sich auf Bedingungen, die eine Hybridisierung von DNA-Strängen erlauben, die zueinander hoch komplementär sind. Die Hybridisierungsstringenz wird im Wesentlichen durch die Temperatur, die lonenstärke und die Konzentration an denaturierenden Detergenzien, wie Formamid, bestimmt. Beispiele "hoch-stringenter Bedingungen" zur Hybridisierung und zum Waschen sind 0,0015 M Natriumcitrat bei 65-68°C oder 0,015 M Natriumchlorid, 0,0015 M Natriumcitrat und 50% Formamid bei 42°C (siehe Sambrook, Fritsch & Maniatis, Molecular Cloning: A Laboratory Manual, 2nd Ed., Cold Spring harbor Laboratory, Cold Spring Harbor, N. Y., 1989).

Hoch-stringente Hybridisierungsbedingungen können auch durch höhere Temperaturen, niedrigere lonenstärke, höhere Formamidkonzentrationen oder denaturlerende Reagenzien erzielt werden. Andere Reagenzien können bei der Hybridisierung und den Waschpuffem eingeschlossen werden, damit eine nicht-spezifische und/oder Hintergrund-Hybridisierung vermindert werden. Beispiele sind 0,1% Rinderserumalbumin, 0,1% Polyvinylpyrrolidon, 0,1% Natrium-Pyrophosphat, 0,1% Natriumdodecylsulfat ($NaDodSO_4$ oder SDS), Ficoll, Denhard's Lösung, Ultraschall-behandelte Lachsspermien-DNA (oder andere nicht-komplementäre DNA) und Dextransulfat. Die Konzentration und Art dieser Additive kann gewechselt werden, ohne die Stringenz der Hybridisierungsbedingungen wesentlich zu verändern. Hybridislerungsexperimente werden üblicherweise bei pH 6,8-7,4 durchgeführt, bei üblichen lonenstärken ist die Hybridisierungsrate nahezu unabhängig vom pH-Wert.

Moderat-stringente Bedingungen

Der Ausdruck "moderat-stringente Bedingungen" bezieht sich auf Bedingungen, unter denen ein DNA-Duplex mit einem größeren Grad an Basenpaar-Fehlpaarungen gebildet werden kann, im Vergleich zu "hoch-stringenten Bedingungen". Der Grad an Homologie, das zur Ausbildung eines DNA-Duplex notwendig ist, beträgt unter moderat stringenten Bedingungen vorzugsweise mindestens 70%, besonders bevorzugt mindestens 75%, beispielsweise mindestens 77, 78, 79, oder 80%. Beispiele für übliche "moderat-stringente Bedingungen" sind Lösungen mit 0,015 M Natriumchlorid, 0,0015 M Natriumcitrat bei 50-65°C oder 0,015 M Natriumchlorid, 0,0015 M Natriumcitrat und 20% Formamid bei 37-50°C. Beispielhaft erlaubt eine "moderat-stringente Bedingung" bei 50°C in 0,015 M Natriumionen ungefähr 21 % Fehlpaarungen.

Biosensoren

Der Ausdruck "Biosensor" betriff hier einen Aufbau, in dem eine biologische Komponente (z. B. ein Enzym, ein Antikörper oder ein Mikroorganismus) unmittelbar mit einem Signalwandler (z. B. einer Elektrode, einer optischen Faser, einem Piezokristall oder einem Transistor) verbunden ist, um das Vorliegen eines Stoffes detektieren zu können.

Hefe

Der Ausdruck "Hefe", so wie er hier gebraucht wird, schließt Hefen (Endomycetales), basidiosporogene Hefen und Hefen, die zu den "imperfekten Pilzen" (Blastomyzeten) gehören, ein. Zur Biochemie und Genetik von Hefen sei verwiesen auf: Bacil, M., Horecker, B. J. und Stopani, A. O. M. Editors, 2nd Edition, 1987. Die Hefegattung *Arxula* mit ihrer bekanntesten Art, *A. adeninivorans* wurde von Middelhoven et *al.* 1984 zum ersten Mal beschrieben, gehört zu den Ascomyceten und ist eine nicht-konventionell verwendete, nicht-pathogene Hefe, die als einer der wenigen Organismen Tannin als C-Quelle nutzen kann (s. zu weiteren Charakteristika dieser Hefe auch die deutsche Patentanmeldung DE100 22 334-A1).

Die hierin beschriebenen Proteine mit Tannase- und/oder Lipase-Aktivität weisen, im Vergleich zu den Tannasen aus dem Stand der Technik, wesentlich höhere, und damit vorteilhaftere spezifische Aktivitäten auf. Z. B. weist die Tannase I der vorliegenden Erfindung (SEQ ID NO: 3) im Vergleich zur käuflich von der Firma Kikkoman erhältlichen Tannase aus *Aspergillus oryza,* die zur Behandlung von Tee-Extrakt im industriellen Maßstab vertrieben wird, eine 4000-fach höhere spezifische Aktivität und eine 3.6-fach höhere Affinität gegenüber Tannin auf. Auch im Vergleich zur *Aspergillus niger* Tannase weist die Tannase I der vorliegenden Erfindung eine 3000-5000-fach höhere spezifische Aktivität auf.

Weiterhin spalten die hierin beschriebenen Tannasen nicht nur hydrolysierbare, sondern auch kondensierte Tannine mit hoher Aktivität und weisen somit ein weites Substratspektrum auf. Zusätzlich Ist dabei die Stabilität der Enzyme im Vergleich zu bekannten Tannasen wesentlich höher. Eine weitere Eigenschaft der hierin beschriebenen Tannasen ist, dass sie auch zur Lipidspaltung fähig sind, also Lipase-Aktivität aufweisen. Beschriebenen werden daher kostengünstige effiziente Enzyme (hohe Aktivität, leichte Herstellung, hohe Stabilität), die in verschiedensten industriellen Zweigen eingesetzt werden können, besonders aber für einen Einsatz im Umweltschutz und in der Tierfütterung geeignet sind.

Die erfindungsgemäß bereitgestellte Tannase I (SEQ ID NO: 3) hat ein Temperaturoptimum bei 40°C und ein pH-Optimum zwischen pH 5,0 und 6,5. 95% der Enzymaktivität bleiben auch nach mehrstündiger Inkubation bei 35 - 45°C erhalten. Der $K_M$-Wert beträgt 0.15 mM Tannin und das Molekulargewicht ist 300 000 Da für das native und 76 000 Da für das denaturierte Enzym. Die Tannase I zeigt eine sehr hohe spezifische Aktivität gegenüber einem sehr breiten Substratspektrum; so werden neben hydrolysierten Tannlnen auch kondensierte Tannine gespalten.

Die weiterhin beschriebene Tannase II (SEQ ID NO: 4) hat ein Temperaturoptimum bei 30°C und ein pH-Optimum zwischen pH 5,0 und 6,5. 95% der Enzymaktivität bleiben auch nach mehrstündiger Inkubation bei 25 - 35°C erhalten. (Der $K_M$-Wert beträgt 1.1 mM Tannin und das Molekulargewicht beträgt 100 000 Da für das native Enzym. Die denaturierte Form weist ein Molekulargewicht von 54 000 Da auf.

Vorliegend beschrieben werden Nukleinsäuresequenzen nach SEQ ID NO: 1 oder SEQ ID NO: 2, solche, die für ein Polypeptid nach SEQ ID NO: 3 oder SEQ ID NO: 4 kodieren, sowie solche, die für ein Polypeptid kodieren, dass mindestens 70%, vorzugsweise 80%, noch bevorzugter 90%, oder 95 % Identität mit dem Polypeptid nach SEQ ID NO: 3 oder SEQ ID NO: 4 ist, wobei das Polypeptid eine Tannase- und/oder Lipase-Aktivität des Polypeptids nach SEQ ID NO: 3 oder SEQ ID NO: 4 aufweist.

Ferner werden Nukleinsäuresequenzen beschrieben die für allellsche oder Splicevarianten der Nukleinsäuresequenz nach SEQ ID NO: 1 oder SEQ ID NO: 2 kodieren, sowie Fragmente der oben genannten Nukleinsäuresequenzen, die für Aminosäuren mit mindestens 25, bevorzugt 25 bis 100 Aminosäuren, noch bevorzugter 25 bis 500 und am bevorzugtesten 25 bis 1000 Aminosäuren kodieren. In jedem Fall weisen die von diesen kodierten Polypeptide eine Tannase- und/oder Lipase-Aktivität des Polypeptids nach SEC ID NO: 3 oder SEQ ID NO: 4 auf.

Beschrieben werden auch Nukleinsäuresequenzen, die die Nukleinsäuren 460-680 der SEQ ID NO: 1 oder SEQ ID NO: 2 umfassen. Diese Nukleinsäuren schließen die Lipid-Bindungsstellen und das "nukleophile Serin" im aktiven Zentrum ein. Lipasen sind Serinesterasen mit nukleophilem Serin im aktiven Zentrum; es erfolgt ein nukleophiler Angriff des Serins aus dem aktiven Zentrum an das Substrat (z. B. Triglycerid) und durch die Bildung eines tetraedrischen Intermediates. Ferner werden Nukleinsäuresequenzen beschrieben, die unter moderat-stringenten oder hoch-stringenten Bedingungen mit einem komplementären Strang zu den oben genannten Nukleinsäuren hybridisieren, und Nukleinsäuresequenzen, die durch Degeneration des genetischen Codes, durch Deletionen, Insertionen, terminale Additionen und/oder Nukleotidsubstitutionen mindestens eines Nukleotides von den oben genannten Nukleinsäuren abgeleitet sind. Bei Deletionen und Insertionen von Nukleotiden werden bevorzugt Vielfache von 3 Nukleotiden deletiert oder insertiert, um Rasterverschiebungen zu vermeiden. Das gleiche gilt für terminale Additionen. Der Umfang von Deletionen, Insertionen und terminalen Additionen kann variieren zwischen 3 und 300 Nukleotide, bevorzugt 3 - 90 Nukleotide, besonders bevorzugt 3 - 30. Die Deletionen, Insertionen und terminalen Additionen können dabei jeweils einzeln oder Insgesamt den bevorzugten Umfang haben, wobei auch mehrere dieser Deletionen, Insertionen und terminalen Additionen vorgesehen sein können. Gegebenenfalls können auch Deletionen und Insertionen gleichzeitig erfolgen, desgleichen Insertionen und Additionen oder Deletionen und Additionen. Im Fall der Nukleotidsubstitutionen kann bereits durch den Austausch eines einzelnen Nukleotids eine veränderte Aminosäuresequenz hervorgerufen werden. In bevorzugten Ausführungsformen sind zwischen 1 und 50, besonders bevorzugt zwischen 1 und 10, Nukleotide substituiert Nukleotidsubstitutionen können selbstverständlich sowohl mit Deletion, Insertion als auch mit Addition kombiniert auftreten. In jedem Fall weisen die von diesen kodierten Polypeptide Tannase- und/oder Lipase-Aktivität des Polypeptids nach SEQ ID NO: 3 oder SEQ ID NO: 4 auf.

Ferner werden Nukleinsäuresequenzen beschrieben, die komplementär zu den oben genannten sind. Des weiteren werden Nukleinsäuresequenzen beschrieben, die ein Polypeptid kodieren, das von einem Polypeptid nach SEQ ID NO: 3 oder SEQ ID NO: 4 abgeleitet ist, und sich von dem Polypeptid nach SEQ ID NO: 3 oder SEQ ID NO: 4 durch mindestens eine Substitution, Insertion, Deletion, C- und/oder N-terminale Verkürzung oder Addition unterscheidet, wobei die von den Nukleinsäuren kodierten Polypeptide Tannase- und/oder Lipase-Aktivität des Polypeptids nach SEQ ID NO: 3 oder SEQ ID NO: 4 aufweisen. Der Umfang der Substitutionen, Insertionen und Deletionen kann variieren zwischen 1 und 10 Resten, bevorzugt 1 - 30 Resten, besonders bevorzugt 1 - 10. Bis zu 15 Reste können vom N- und/oder C-terminalen Ende des Polypeptids entfernt werden, zum Beispiel bis zu 6 oder bis zu 11 Reste.

In einer bevorzugten Ausführungsform bezieht sich die Erfindung auf ein Expressionskonstrukt, das eine erfindungsgemäße Nukleinsäure umfasst, wobei die Nukleinsäuresequenz operativ mit einer regulatorischen Nukleinsäuresequenz verbunden ist, die Signale für die Transkriptions- und/oder Translationssteuerung enthält und die Expression der Nukleotidsequenz in der Wirtszelle kontrolliert, sowie eine Wirtszelle, die das Expressionskonstrukt enthält. Ein Ziel ist, die Tannase sehr stark zu exprimieren, damit sie möglichst unabhängig von der Zusammensetzung des Kultivierungsmediums in möglichst hohen Konzentrationen ins Medium sezerniert werden kann. Die Wahl des Expressionsvektors, auf dessen Grundlage das Expressionskonstrukt gebildet werden soll, kommt daher besondere Bedeutung zu. Es versteht sich von selbst, dass sowohl hinsichtlich der regulativen Elemente, wie Promotoren, Enhancer, etc., sowie der für eine autonome Replikation verantwortlichen Sequenzen bei der Auswahl Rücksicht auf die vorgesehene Wirtszelle genommen werden muss. Wirtszellen können dabei prokaryotische oder eukaryotische Zellen sein. Hefen sind als Wirtszellen bevorzugt. In einer besonders bevorzugten Ausführungsform ist die Wirtszelle *Arxula adeninivorans.*

Wie weiter unten ausgeführt, kann das Tannase-Gen z. B. in eine Expressionskassette mit *TEF1*-Promotor (*Arxula*)-*PHO*5-Terminator (*S. cerevisiae)* eingebaut werden (Rösel H, Kunze G, Curr. Genet. 33, 157-163, 1998; Wartmann T, Böer E, Pico AH, Sieber H, Bartelsen O, Gellissen G, Kunze G, FEMS Yeast Res. 2, 363-369, 2002). Bei dem *TEF1*-Promotor handelt es sich um einen sehr starken konstitutiven Promotor, der alle Voraussetzungen für eine sehr gute Tannase-Genexpression erfüllt. Die erhaltene Kassette mit *TEF1*-Promotor - *Tannase*-Gen - *PHO5*-Terminator wird anschließend in das *Arxula*-Plasmid pAL-ALEU2m eingebaut, welches nach Linearisierung mit *Esp*3I direkt in *A. adeninivorans* G1211 (*aleu*2) transformiert werden kann. Die erhaltenen Transformanten haben dieses Plasmid stabil in der 25S-rDNA-Region integriert (Rösel und Kunze 1998, Wartmann T, Stoltenburg R, Böer R, Sieber H, Bartelsen O, Gellissen G, Kunze G, FEMS Yeast Res. 3, 223-232, 2003).

Parallel können die *Arxula*-Plasmide mit Selektionsmarkern wie *AILV1-Gen* (Wartmann T et al. Yeast 14, 1017-1025, 1998), *ATRP1-Gen* (EP 01938205.0) oder den aus *E. coli* stammenden *hph*-Gen (kodiert Resistenz gegenüber Hygromycin B - Rösel und Kunze Curr. Genet. 33, 157-163, 1998) ausgestattet sein. Auch der Einsatz von autonom replizierenden Sequenzen (ARS), die in *A. adeninivorans* funktionstüchtig sind, ist möglich. Die mit solch einem Plasmid transformierten *A. adeninivorans* Zellen enthalten dieses Plasmid mit der entsprechenden Tannase-Expressionskassette als zirkuläres Plasmid in hoher Kopienzahl.

Ferner bezieht sich die vorliegende Erfindung auf ein Verfahren zum Herstellen eines Polypeptids mit Tannase-Aktivität, umfassend die folgenden Schritte: a) Kultivieren einer erfindungsgemäßen Wirtszelle unter für eine Expression des Polypeptids geeigneten Bedingungen, b) Anreichern des Polypeptids.

Geeignete Bedingungen schließen die Anzucht des Organismus unter Verwendung einer C-Quelle bzw. Anzucht in Tannin-haltigen Medien ein, z. B. Medien mit 0,5% Glukose, 0,5% Tannin, 0.5% Gallat oder 0,5 % Protocatechusäure. Die Menge an sezemierter Tannase und/oder Lipase kann Ober Antikörperreaktionen bzw. über Messungen der Tannase- und/oder Lipase-Aktivität (z. B. spektrophotometrisch oder über HPLC) bestimmt werden. Die Anreicherung erfolgt mit herkömmlichen Methoden, z. B. Säulenchromatographie (z. B. Affinitäts-, Gelfiltrations-, Anionenaustausch-Chromatographie), spezifische Faltungen, Elektrophoresen etc.

Das Anreichem erfolgt z. B. über Ultrazentrifugation oder PEG 6000 Fällung mit anschließender DEAE-Zellulose Chromatographie (Anionenaustauscher). Die Aktivitäten lassen sich biochemisch bzw. mit HPLC (Nachweis der Spaltprodukte).

In einer besonderen Ausführungsform ist die Wirtszelle *Arxula adeninivorans* und das Polypeptid wird isoliert und aufgereinigt Die Patentanmeldung DE 100 22 334-A1 beschreibt mögliche Techniken zur Anzucht und Proteinexpression In *Arxula.* Eine Isolierung und Anreicherung kann z. B. mittels Ultrazentrifugation bzw. PEG 600 Fällung (Anreicherung der sezernierten Proteine), Chromatographie mit DEAE-Zellulose Säule (Reinigung) oder Chromatographie mit HiLoad™ 16/60 Superdex™ 200 pg Säule (Amersham) erfolgen, wenn ein höherer Reinheitsgrad erreicht werden soll.

Besonders bevorzugt ist es, dass das Polypeptid durch Auswahl geeigneter Vektoren In hohen Mengen In das Kulturmedium sezerniert wird. Durch Anzucht in Tannin-haltigem Medium, wie oben beschrieben, lassen sich die Ausbeuten erhöhen. Ferner kann z. B. die C-Quelle im Medium verändert werden (z. B. Gallussäure), die Kultivierung kann bis in die stationäre Wachstumsphase erfolgen oder es können starke konstitutive bzw. regulierbare Promotoren bei der Herstellung von rekombinanten Proteinen verwendet wer den.

Die Erfindung richtet sich ferner auf ein Polypeptid mit Tannase-Aktivität, das mit einem der oben genannten Verfahren herstellbar ist.

Ferner bezieht sich die Erfindung auf ein Polypeptid mit Tannase-Aktivität, umfassend eine Aminosäuresequenz mit der in SEQ ID NO: 3 angegebenen Sequenz, die optional weitere aminoterminale Methionine umfasst.

Beschrieben wird ferner ein Fragment der Aminosäuresequenz nach SEQ ID NO: 3 oder SEQ ID NO: 4 mit mindestens 25 Aminosäuren, bevorzugter 50-100 Aminosäuren, noch bevorzugter 200-500 Aminosäuren, am bevorzugsten 500-2000 Aminosäuren, eine Aminosäuresequenz für ein Allel, eine Splice- bzw. eine Prozessierungsvariante, eine Aminosäuresequenz mit mindestens einer konservativen Substitution, und/oder Insertion, und/oder Deletion und/oder mindestens einer C- oder N-terminalen Verkürzung, wobei das Polypeptid eine Tannase- und/oder Lipase-Aktivität des Polypeptids nach SEQ ID NO: 3 oder SEQ ID NO: 4 aufweist

Weiterhin beschrieben wird eine Aminosäuresequenz, die die Aminosäuren 139-227 (schließt Lipid-Bindungsregion sowie das "nukleophile Serin" ein) umfasst sowie eine Aminosäuresequenz mit mindestens einer der oben genannten Modifikationen bzw. der oben genannten Sequenzen, wobei das kodierte Polypeptid eine Aktivität des Polypeptids nach SEQ ID NO: 3 oder SEQ ID NO: 4 aufweist.

Ferner werden Derivate und Fusionspolypeptide der oben genannten Aminosäuresequenzen beschrieben, wobei das Derivat oder Fusionspolypeptid eine Tannase- und/oder Lipase-Aktivität des Polypeptids nach SEQ ID NO: 3 oder SEQ ID NO: 4 aufweist

Ganz allgemein sind solche Veränderungen In den beschriebenen Polypeptiden im Vergleich zu den Polypeptiden nach Sequenz ID NO: 3 oder SEQ ID NO: 4 bevorzugt, die die Eigenschaften der Tannase- und/oder Lipase in der gewünschten Richtung (z. B. Aktivitätssteigerung, bestimmtes pH- oder Temperaturoptimum, verändertes Substratspektrum) verändem.

In einer bevorzugten Ausführungsform wird das Polypeptid durch eine der vorher genannten Nukleinsäuren kodiert.

Die Erfindung umfasst weiter einen Antikörper oder Fragmente (Fab, $F(ab)_2$, Fv) davon, die spezifisch an das in Anspruch 1 genannte Polypeptid binden. Eine besondere Ausführungsform betrifft polyklonale Antikörper, wie z. B. den and-Atan1p Antikörper, der weiter unten beschrieben ist und gegen Tannase I (SEQ ID NO: 3) gerichtet ist. In einer weiteren bevorzugten Ausführungsform ist der Antikörper ein monoklonaler Antikörper, der bevorzugt von einer Hybridoma-Zelllinie gebildet wird. Techniken zur Herstellung dieser Antikörper und bestimmter Antikörper-Subtypen, weiter oben genannt, sind im Stand der Technik gut bekannt

Weitere Aspekte der Erfindung richten sich auf einen Biosensor, der ein erfindungsgemäßes Polypeptid umfasst, ein unlösliches Trägermaterial und eine enzymatische Zusammensetzung, die jeweils eines der oben genannten Polypeptide, die eine Tannase-Aktivität aufweisen, umfassen. Hier wird auf die deutsche Patentanmeldung DE100 34 578-A1 verwiesen, in der Biosensoren auf Hefe-Myzelbasis beschrieben sind.

Als Biosensoren kommen z. B. mikrobielle Biosensoren infrage, die Tannasegene als Reportergene verwenden (s. z. B. Östrogensensor - Patentanmeldung 103 45 388.5), Enzymsensoren zum Nachweis von Tanninen mittels Tannase (z. B. in Tierfutter, Abwasser von Konservenfabriken, Tabak-, Kaffee-, Tee-Herstellern, Weinherstellung) und Antikörpersensoren zum Nachweis von Tanninen.

Ferner richtet sich die Erfindung auf eine transgene Pflanze, die eine der oben genannten Nukleinsäuren umfasst bzw. eines der oben genannten Polypeptide überexprimiert. Dafür kommen z. B. Pflanzen mit hohem Tanningehaft (z. B. Schmettelingsblütier, wie Erbse oder Bohne) infrage. Die transgenen Pflanzen werden mit im Stand der Technik gängigen Verfahren hergestellt

Ein weiterer Aspekt dieser Erfindung richtet sich auf eine der oben genannten transgenen Pflanzen zur Verwendung bei der Herstellung von Tannin-reduzierten Nahrungsmitteln oder Futtermitteln. Auf diese Weise können z. B. auch Pflanzen mit hohem Tanningehalt (z. B. Schmetterlingsblütler) verfüttert werden.

Ferner richtet sich die Erfindung auf ein Verfahren zur Detektion oder Quantifizierung der erfindungsgemäßen Tannase in einer Probe; hierzu wird die Tannase enthaltende Probe mit einem für die erfindungsgemäße Tannase-spezifischen Antikörper oder einem Fragment desselben in Kontakt gebracht und die Bindung des spezifischen Antikörpers bzw. Antikörperfragmentes an das Protein mit den üblichen Methoden nachgewiesen und quantifiziert. Detektion und Quantifizierung kann auch durch die Verwendung eines entsprechend gebildeten Biosensors erfolgen.

Weiter bezieht sich die Erfindung auf ein Verfahren zur Reduktion der Menge an Tanninen in einer Zusammensetzung durch Kontaktieren der Zusammensetzung mit dem in Anspruch 1 genannten Polypeptid, bzw. in den Ansprüchen genannten Wirtszellen, unlöslichen Trägermaterialien, enzymatischen Zusammensetzungen oder Tannase-haltigen Kulturmedien.

So könnten beispielsweise transgene Arxula-Zellen mit einem tanninhaltigen Tierfutter gemischt werden. *Arxula* wurde bereits in den 80iger Jahren in der "Single Cell"-Produktion erfolgreich eingesetzt und als Futter für Schweine genutzt (Malchin/Mecklenburg-Vorpommem). Die gleichzeitige Verfüfterung von tanninhaltigem Futter mit Tannase-produzierenden Mikroorganismen könnte zu einer erheblich verbesserten Verwertung des tanninhaltigen Futters führen. Gleichzeitig führt der Abbau der Tannine im Tierfutter vor bzw. auch nach Aufnahme durch das Tier zu einer Verringerung der Abwasserbelastung mit Tanninen. Alternativ kann tanninhaltiges Abwasser durch Zugabe von Tannase direkt bearbeitet werden.

Besonders bevorzugt ist es, das Verfahren zur Reduktion der Tannine in Futter- und Nahrungsmitteln, kontaminierten Zusammensetzungen bzw. landwirtschaftlichen und Industriellen Abwässern einzusetzen. Insbesondere werden Futter- und Nah-rungsmittel mit hohem Tanningehalt (z. B. basierend auf Schmetterlingsblütlem), oder Abwässer von Mastbetrieben und von Tee-, Kaffee-, Tabak- Herstellern mit Tannasen behandelt. Ferner richtet sich die Erfindung auf die Verringerung des Gehaltes an Tanninen in Produkten der Chemie-, Pharma-, Nahrungsmittel-, Waschmittel- , Leder-, Papier-, und/oder Kosmetikindustrie.

Ferner bezieht sich die vorliegende Erfindung auf eine Verwendung der in den Ansprüchen genannten Polypeptide, der Wirtszellen, unlöslichen Trägermaterialien, enzymatischen Zusammensetzungen oder Tannase-haltigen Kulturmedien zur Reduktion der Menge an Tanninen in einer Zusammensetzung.

Besonders bevorzugt ist dabei, dass die Tannin- haltige Zusammensetzung ein Nahrungs- oder Futtermittel, eine mit diesen Stoffen kontaminierte Zusammensetzung bzw. landwirtschaftliches oder industrielles Abwasser ist.

In einer besonders bevorzugten Ausführungsform wird das in Anspruch 1 genannte Polypeptid zur Verbesserung der Futterverwertung durch Nutztiere eingesetzt. Bevorzugt wird die Tannase vor dem Verfüttern Tannin-haltiger Futtermittel zugeben, es ist aber auch eine Verabreichung des Futters zusammen mit dem Enzym denkbar. Die Applikation sollte dann z. B. in Kombination mit Phytase erfolgen.

In einer weiteren Ausführungform richtet sich die Erfindung auf die Verwendung des in Anspruch 1 genannten Polypeptids zur Herstellung von Gallussäure aus Tanninen. Dazu werden Tannin- haltige Materialien mit den erfindungsgemäßen Polypeptiden in gereinigter Form oder als Kulturüberstand oder mit erfindungsgemäßen Polypeptiden sezemierenden Wirtszellen inkubiert. Für den Abbau der Tannine geeignete Bedindungen können vom Fachmann durch

Reaktionsversuche bestimmt werden.

Weiter richtet sich die Erfindung auf die Verwendung des Blosensors zur Detektion von Tanninen in Abwässern, Nahrungs- und Futtermitteln. Es können aber auch Biosensoren zum Nachweis von Tannase eingesetzt werden.

Ein weiterer Aspekt der Erfindung bezieht sich auf die Verwendung der in Anspruch 1 genannten Nukleinsäure bzw. des in Anspruch 1 genannten Polypeptids zur Herstellung eines Medikaments zur Behandlung von Verdauungsstörungen, Übergewicht und Nahrungsmittelunverträglichkeiten.

Die Figuren und Beispiele erläutern die Erfindung, sowie die beschriebenen Gegenstände.

**Flgurenbeschreibung:**

**Figur 1:** Akkumulation von Tannase im Kultivierungsmedium von *A. adeninivorans* LS3. Dazu wurde *A. adeninivorans* LS3 in HMM + 2 % (w/v) Glukose bei 30°C kultiviert, geemtet und in einem Schüttelkolben mit gleichem Volumen an HMM mit 0,5 % (w/v) Tannin umgesetzt. Die Hefezellen wurden darin bis zu 96 h bei 30°C kultiviert, abzentrifugiert und die im Medium vorhandene Tannaseaktivität gemessen. 1 E Tannase entspricht den Abbau von 1 μmol Tannin pro Minute.

**Figur 2:** Gen- und Restriktionskarte des *ATAN1*-Gens.

**Figur 3:** DNA-Sequenz des *ATAN1*-Gens von *A. adeninivorans* LS3 und die davon abgeleitete Aminosäuresequenz der Tannase I (Atan1p). Die potentielle CAAT-Box in der Promotor-Region wurde unterstrichen. Zusätzlich wurden vom Atan1p die Signalsequenz (Aminosäure-Pos. 1-28) und die potentiellen N-Glycosyllerungsstellen hervorgehoben.

**Figur 4:** Phylogenetischer Stammbaum für *Arxula adeninivorans* Atan1p (Tannase I).

**Figur 5:** Pulsed Field Gelelektrophorese mit anschließender Southem-Hybridislerung, die mit den Stämmen S288C von *S. cerevisiae* (1), 1136 (2), 1138 (3), 1147 (4), 1148 (5), 1149 (6), 8244 (7) und LS3 (8) von *A. adeninivorans* durchgeführt wurde. Die Hybridisierung erfolgte mit einer radioaktiv markierten Sonde aus einem DNA-Fragment, auf welchem das *ATAN1-Gen* lokalisiert war.

**Figur 6:** Southem Hybridisierung von chromosomaler DNA von *A. adeninivorans* LS3, die mit den Restriktionsenzymen *Eco*RI (1), *Bam*HI (2), *Hind*III (3), *Sal*I (4), *Eco*RV (5) und *Xho*I (6) gespalten und anschließend auf eine Nitrozellulosemembran geblottet wurde. Die Hybridisierung erfolgte mit radioaktiv markierten *ATAN1*-Gensequenzen.

**Figur 7:** Nachweis der Induzierbarkeit des *ATAN1*-Gens nach Zugabe von Glukose, Tween 20, Gallussäure und Tannin zum Kultivierungsmedium. Dazu wurde *A. adeninivorans* LS3 für 48h in HMM + 2% Glukose kultiviert, geerntet und für weiter 0,5, 1, 2, 4, 8, 12, 16, 20 und 24 h auf HMM + 0,5% Glukose, Tween 20, Gallussäure bzw. Tannin inkubiert Aus den erhaltenen Hefezellen wurde die RNA Isoliert und für die Northem Hybridisierung eingesetzt. Als radioaktiv markierte Sonden wurden DNA-Fragmente mit dem *TEF1-Gen* (Kontrolle) bzw. *ATAN1-Gen* verwendet.

**Figur 8:** Atan1p Akkumulation im Kultivierungsmedium von *A. adeninivorans* LS3. Dazu wurden die Hefezellen bis zu 48 h in HMM mit verschiedenen C-Quellen kultiviert. Zur Atan1p Detektion diente ein eigener anti-ATAN1p Antikörper.

**Figur 9:** Gen- und Restriktionskarte des Plasmids pAL-ALEU2m-ATAN1.

**Figur 10:** Gen- und Restriktionskarte des *ATAN2*-Gens einschließlich Promotor und Terminator-Region.

**Figur 11:** DNA-Sequenz des *ATAN2*-Gens von *A. adeninivorans* LS3 und davon abgeleitete Aminosäuresequenz der Tannase II (Atan2p). Die potentielle CAAT-Box und TATA-Box in der Promotor-Region wurden unterstrichen. Zusätzlich wurden vom Atan2p die Signalsequenz (Aminosäure-Pos. 1-26) und die N-Glycosylierungsstelle, sowie die konservierten Cysteine hervorgehoben.

**Figur 12:** Phylogenetischer Stammbaum des *Arxula adeninivorans* Atan2p (Tannase II).

**Figur 13:** Pulsed Field Gelelektrophorese mit anschließender Southem-Hybridisierung, die mit den Stämmen S288C von *S. cerevisiae* (1), 1136 (2), 1138 (3), 1147 (4), 1148 (5), 1149 (6), 8244 (7) und LS3 (8) von *A. adeninivorans* durchgeführt wurde. Die Hybridisierung erfolgte mit einer radioaktiv markierten ATAN2-Gensequenz lokalisiert war.

**Figur 14:** Southern Hybridisierung von chromosomaler DNA von *A. adeninivorans* LS3. Die DNA wurde mit den Restriktionsenzymen EcoR1 (1), *Bam*HI (2), *Hind*III (3), *Sal*I (4), *Eco*RV (5) und *Xho*I (6) gespalten und anschließend auf eine Nitrozellulose-Membran geblottet. Die Hybridisierung erfolgte mit einer radioaktiv markierten *ATAN2*-Gensequenz.

**Figur 15:** Nachweis der Induzierbarkeit des *ATAN2*-Gens nach Zugabe von Glukose, Tween 20, Gallussäure und Tannin zum Kultivierungsmedium. Dazu wurde *A. adeninivorans* LS3 für 48h in HMM + 2 % (w/v) Glukose kultiviert, geerntet und weiter für 0,5, 1, 2, 4, 8, 12, 16, 20 und 24 h auf HMM + 0,5 % (w/v) Glukose, Tween 20, Gallussäure bzw. Tannin inkubiert. Aus den erhaltenen Hefezellen wurde die RNA isoliert und für die Northern Hybridisierung eingesetzt. Als radioaktiv markierte Sonden wurden DNA-Fragmente mit dem *TEF1*-Gen (Kontrolle) bzw. *ATAN2*-Gen verwendet.

**Figur 16:** Atan2p Akkumulation im Kultivierungsmedium von *A. adeninivorans* LS3. Dazu wurden die Hefezellen bis zu 48 h in HMM mit verschiedenen C-Quellen kultiviert. Zur Atan2p Detektion diente ein eigener anti-ATAN2p Antikörper.

**Figur 17:** Gen- und Restriktionskarte des Plasmids pAL-ALEU2m-ATAN2.

**Figur 18:** Wachstumsverlauf (□) und Lipase-Akkumulation (♦) in Kultivierungsmedium von *A. adeninivorans* G1211/pAL-ALEU2m-ATAN2 30/30. Die Transformante wurde in HMM + (A) 2%Glukose bzw. (B) 2%Xylitol kultiviert.

**Figur 19:** Substratspezifität von Atan2p. Dazu wurde das von *A. adeninivorans* LS3 (A-tan2p nativ) und *A. adeninivorans* G1211/pAL-ALEU2m-ATAN2 (Atan2p rekombinant) sezernierte Atan2p auf Lipaseaktivität getestet. Folgende Substrate wurden dazu verwendet: C2: pNP-Acetat, C4: pNP-Butyrate, C6: pNP-Caproate, C8: pNP-Caprylate, C10: pNP-Caprate, C12: pNP-Laurate, C14: pNP-Myristate, C16: pNP-Palmitate, C18: pNP-Sterate.

## Beispiele

**Methoden:**

Tannaseassay mit Rhodanin

Der Nachweis der Tannase mit Hilfe des Rhodanins, wurde nach einer Methode von Sharma et *al*. (Sharma S, Bhat TK, Dawra RK (2000) A spectrophotometric method for assay of tannase using Rhodanine. Anal. Biochem. 279: 85-89, 2000) durchgeführt.

Dafür wurden zu 250 μl Substratlösung (0,01 M Methylgallat [Aldrich/ USA]; 0,05 M Na-citrat-Puffer pH 6,0), 200 μl Puffer (0,05M Na-citrat-Puffer pH 6,0) und 50 μl eingeengter Kulturüberstand (siehe Pkt. 2.13.2) gegeben. Als Test-Kontrolle wurden 450 μl Puffer und 50 μl eingeengter Kulturüberstand zusammenpipettiert. Als Blank-Kontrolle wurden 250 μl der Substratlösung und 250 μl Puffer zusammengegeben. Diese drei verschiedenen Ansätze wurden anschließend für 5 Min bei 30°C inkubiert. Danach wurde zu jedem Ansatz 300 μl methanolisches Rhodanin (0,667% Rhodanin [Aldrich/ USA] in Methanol) zugegeben. Nach anschließender Inkubation für 5 Min bei 30°C wurden weitere 200 μl 0,5 M KOH zugegeben. Nach wiederholter Inkubation bei 30°C für 5 Min wird die Absorbtion der verschiedenen Ansätze bei 520 nm im Spektrophotometer (UV160-A/Shimadzu) gegen Wasser gemessen. Die Absorption kann nach folgender Formel berechnet werden:

$$\Delta A_{520nm} = ( A_{Test} - A_{Blank} ) - ( A_{Kontolle} - A_{Blank})$$

Mit Hilfe einer Eichkurve wurde dann die entstandene Gallatmenge ermittelt.

$$\text{Einheiten/ml Enzym} = \frac{(\mu\text{mol gebildetes Gallat/min}) * (\text{Vf})}{(0{,}05)}$$

Vf = Verdünnungsfaktor
0,05 = eingesetztes Probevolumen (in Milliliter)
Eine Einheit ist definiert als µmol gebildetes Gallat pro Minute.

Lipaseassay mit p-Nitrophenylester

Für diese Bestimmung wurde eine frische Stammlösung (3mM p-Nitrophenol in 10 ml Propanol durch 10 Min vortexen lösen) angesetzt. Für den Messansatz wurden 850 µl 0,05 M Na-Phosphat-Puffer pH 7,5 und 100 µl der Stammlösung gemischt und anschließend 50 µl Probelösung zugegeben. Als Kontrolle wurden 900 µl Puffer und 100 µl Stammlösung zusammengegeben. Beide Ansätze wurden für 5-10 Min bei 30°C inkubiert und anschließend 1 ml Aceton zum Abstoppen der Reaktion zugegeben. Danach wurde die Absorption bei 410 nm im Spektrometer (UV160-A/Shimadzu) gegen Wasser gemessen: Die Enzymaktivität kann dann nach folgender Formel berechnet werden:

$$\text{Einheiten/ml Enzym} = \frac{(\Delta A_{410nm}/\text{min Test} - \Delta A_{410nm}/\text{min Blank}) * (\text{Vf})}{(14{,}800) * (0{,}05)}$$

Vf = Verdünnungsfaktor
14800 l $mol^{-1}$ $cm^{-1}$= Extinktionskoeffizient von p-Nitrophenol bei 410nm (Quinn *et al.* 1982) 0,05 = eingesetztes Probevolumen (in Milliliter)
Eine Einheit ist definiert als µmol gebildetes p-Nitrophenol pro Minute.

Substratanalyse der Tannase mittels HPLC

Der Nachweis der Tannaseaktivität mit Hilfe der HPLC wurde nach einer Methode von Ramirez-Coronel et al. (2003) durchgeführt. Dafür wurden 0,5 mg/ml verschiedener Tannine in 1 ml 0,1 M Na-Acetat-Puffer pH 5,5 gelöst und 10 µl gereinigtes Enzym zugegeben. Zur Kontrolle wurde der Ansatz ohne Zugabe von Enzym eingesetzt. Anschließend wurden beide Ansätze für 30 Min bei 30°C inkubiert und danach bei -80°C eingefroren. Die chromatographische Analyse erfolgte in einer HPLC-Anlage mit UV Detektor bei 280 nm. Die Proben wurden über eine Phenomenex-Säule (Phenomenex prodigy 5 µ, ODS 3, 100Å, 250x4,6 mm) bei einer Durchflussrate von 1 ml $Min^{-1}$ durch ein Gradient-Elutions-System aufgetrennt. Für Pumpe A wurde Ameisensäure und Wasser (1:99, v/v) und für Pumpe B Methanol als Eluent verwendet, wobei ein linearer Gradient von 10 % bis 100 % Puffer B in 10 Min geschalten wurde.

Gallussäure wurde identifiziert durch den Vergleich der Retentionszeit mit dem reinen Produkt (Sigma).

Die spezifische Aktivität des Enzyms wurde in µmol gebildeter Gallussäure $Min^{-1}$ (mg $Protein)^{-1}$ berechnet.

**Beispiel 1**

Biochemische Charakteristika der *A. adeninivorans* LS3 Tannase

Weltweit existieren ca. 10 *A. adeninivorans* Wildtypstämme, die sich sowohl in ihrer Physiologie als auch in der Morphologie unterscheiden. Basierend auf den Ergebnissen des Vorscreenings wurde der Stamm *A. adeninivorans* LS3 sowohl als Tannase-Gendonor als auch als Wirt für die Tannaseproduktion ausgewählt.

Die Analyse der Tannaseakkumulation vom Wildtypstamm *A. adeninivorans* LS3 zeigte, dass das Enzym bei Verwendung von Tannin als C-Quelle synthetisiert und in Kultivierungsmedium sezerniert wird. Die maximale Tannaseaktivität wird 24 h nach dem Umsetzen der Hefe von HMM + Glukose auf HMM + Tannin erreicht. Zu diesem Zeitpunkt treten Tannaseaktivitäten von 85 E pro ml Medium auf. Dies sind 3-fach höhere Aktivitäten als sie jemals bei *Aspergillus niger* bzw. *Aspergillus oryzae* gemessen wurden. Im weiteren Kultivierungsverlauf sinkt die Tannaseaktivität und erreicht nach 50 h mit ca. 5 E/ml ihren Basiswert (Fig. 1).

Um genügend Tannase für die biochemische Charakterisierung zur Verfügung zu haben, wurden *A. adeninivorans* LS3 Kulturen nach dem Umsetzen von HMM + Glukose in HMM + Tannin 48 h kultiviert, die im Medium enthaltene Tannase mittels Polyethylenglykol (PEG) 6000 gefällt und über eine Anionenaustauscher-Säule gereinigt. Die dabei erhaltene Trennung der sezemierten Proteine zeigte zwei Tannasepeaks, die als Tannase I und Tannase II definiert

wurden.

1. Tannase I

Zuerst wurde das Temperatur-Optimum der Tannase I ermittelt. Dazu wurden 50μl des gereinigten Enzyms mit 250 μl 0,1 M Methylgallat für 15 min bei Temperaturen von 20 - 70°C inkubiert. Nach Abstoppen der Enzymreaktion mit methanolischem Rhodanin und Zugabe von 0,5M KOH bildet sich in Abhängigkeit vom Reaktionsprodukt Gallussäure ein roter Farbkomplex, der als Maß für die Tannaseaktivität dient (Sharma *et al.* 2000). Hierbei konnte ein Temperatur-Optimum von 40°C ermittelt werden. Die thermische Stabilität des Enzyms, d.h. der Temperatur-Bereich mit mindestens 95%iger Enzymaktivität, liegt zwischen 35°C und 45°C.

Auch das pH-Optimum der von *A. adeninivorans* LS3 synthetisierten Tannase I konnte bestimmt werden. Dazu wurde die Enzymreaktion bei pH-Werten zwischen 4,0 und 8,5 durchgeführt. Das pH-Optimum lag bei 6,0. Zwischen pH 5,0 und 6,5 weist das Enzym mindestens 95% seiner maximalen Aktivität auf.

Zur Bestimmung des $K_M$-Wertes wurde zu je 50 μl Tannase 250 μl Tanninlösung in Konzentrationen von 0,05 mg/ml bis 0,75 mg/ml zugegeben, alles 15 Min bei 30°C inkubiert und die Tannaseaktivität wie beschrieben ermittelt. Der dabei bestimmte $K_M$-Wert lag bei 0,15 mM (Tannin). Aus diesem Wert kann abgeleitet werden, dass die aus *A. adeninivorans* LS3 stammende Tannase I eine sehr hohe Affinität zum Substrat Tannin besitzt.

Parallel wurde mittels nativer und denaturierter Polyacrylamid-Gele das Molekulargewicht der Tannase I bestimmt. Es liegt bei 300.000 Da für das native Enzym und bei 76.000 Da für das denaturierte Enzym.

Zusammenfassend lässt sich zu den biochemischen Parametern der Tannase I von A. *adeninivorans* LS3 feststellen, dass dieses Enzym mit einem Temperaturoptimum von 40°C und einer thermischen Stabilität von 35°C bis 45°C relativ leicht, über einen Hitzeschritt, inaktivierbar ist und eine sehr hohe Spezifität zu Tannin aufweist.

Tabelle A:

| Substrat [0,5 mg $ml^{-1}$] | *A. adeninivorans* Tannase Aktivität [mmol $min^{-1}$ (mg protein)$^{-1}$] | *Asp. niger* Tannase Aktivität [mmol $min^{-1}$ (mg protein)$^{-1}$] |
|---|---|---|
| Catechingallat | 5,57 ± 0,04 | 0,00018 ± 0,009 |
| Gallussäuremethylester | 3,06 ± 0,27 | 0,00087 ± 0,07 |
| Gallussäureethylester | 2,88 ± 0,21 | nicht bestimmt |
| Gallussäurepropylester | 2,64 ± 0,16 | nicht bestimmt |
| Tannin | 2,17 ± 0,05 | 0,00084 ± 0,02 |
| Epicatechingallat | 1,58 ± 0,07 | 0,00043 ± 0,02 |
| Gallocatechingallat | 1,38 ± 0,06 | 0,00113 ± 0,01 |
| Epigallocatechingallat | 1,3 ± 0,04 | 0,00064 ± 0,02 |

Tab. A: Substratspektrum der Tannase I von *Arxula adeninivorans* LS3 und der *Asp. niger* Tannase *(Asp. niger* Werte siehe Ramirez-Coronel et *al*. 2003)

Das über HPLC ermittelte Substratspektrum zeigte, dass die Tannase I eine sehr hohe spezifische Aktivität (2200 U/mg Protein gegenüber Gallotannin) und ein sehr breites Substratspektrum aufweist. Hierbei werden neben hydrolysierten Tanninen auch kondensierte Tannine gespalten, während die bisher bekannten Tannasen kondensierte Tannine nicht spalten können. Die erfindungsgemäße Tannase I kann jedoch sowohl hydrolysierbare als auch kondensierte Tannine spalten. Dabei werden spezifische Aktivitäten bis zu 5570 U/mg erreicht.

Verglichen zur derzeit industriell genutzten *Asp. niger* Tannase handelt es sich bei der aus *A. adeninivorans* isolierte Tannase I um ein Enzym mit breiterem Substratspektrum und einer 3000-5000 fach höheren spezifischen Aktivität (Tab. A + B). Bei der *Asp. niger* Tannase handelt es sich jedoch um eine im *Aspergillus* Myzel lokalisierte intrazelluläre Tannase, deren effektive biotechnologische Gewinnung sehr schwierig ist. Da die aus *A. adeninivorans* stammende Tannase I extrazellulär vorliegt, ist sie einfacher und kostengünstiger zu isolieren.

Weiterhin konnte das *ATAN1*-Gen von *Arxula,* welches für die Tannase I kodiert, isoliert und in *A. adeninivorans* über-exprimiert werden.

Bisher ist dies lediglich beim *Tannase* Gen von *Aspergillus oryzae* gelungen. Die Ausbeuten der überexprimierenden Stämme liegen jedoch bei nur 7000 U/L. Verglichen zum Wildtypstamm *A. adeninivorans* LS3, kultiviert in HMM + 0,5% Gallussäure (Enzymausbeute = 70.000 U/L) sind dies 10fach geringere Enzymkonzentrationen.

Im Gegensatz zu den transgenen *Pichia pastoris* Stämmen (Fed-batch Kultivierung) werden die *A. adeninivorans* Stämme lediglich in Schüttelkolben kultiviert.

2. Tannase II

Die Tannase II wurde ebenfalls aus dem Kulturmedium einer Anzucht von *A. adeninivorans* LS3 in HMM mit 0,5 % (w/v) Tannin isoliert und über Anionenaustauschchromatographie gereinigt. Zur Bestimmung von Temperatur-Optimum, -Stabilität und pH-Optimum, -Stabilität wurde, wie bereits bei der Tannase I beschrieben, vorgegangen. Die Tannase II weist mit 30°C ein gegenüber der Tannase I niedrigeres Temperaturoptimum auf. Die thermische Stabilität des Enzyms, d.h. der Temperatur-Bereich mit mindestens 95%iger Enzymaktivität, liegt zwischen 25°C und 35°C. Das pH-Optimum der von A. *a-deninivorans* LS3 synthetisierten Tannase II liegt bei 5,0. Das Enzym ist stabil, d. h. weist mindestens 95% der maximalen Aktivität zwischen pH 5,0 und 5,5 auf.

Zur Bestimmung des $K_M$-Wertes wurde zu je 50µl Tannase-Probe 250µl Tanninlösung in Konzentrationen von 0,05 mg/ml bis 0,75 mg/ml zugegeben, alles 15 Min bei 30°C inkubiert und die Tannaseaktivität gemessen. Der dabei bestimmte $K_M$-Wert lag bei 1,1 mM (Tannin). Aus diesem Wert kann abgeleitet werden, dass die aus *A. adeninivorans* LS3 stammende Tannase II im Vergleich zur Tannase I eine niedrigere Affinität zum Tannin aufweist (Tab. B).

Tabelle B:

| Eigenschaft | Tannase I | Tannase II |
|---|---|---|
| pH-Optimum | 6,0 | 5,0 |
| pH Stabilität | 5,0 - 6,5 | 5,0 - 5,5 |
| Temperatur-Optimum | 40 °C | 30 °C |
| thermische Stabilität | 35 - 45 °C | 25 - 35 °C |
| Michaelis Konstante ($K_m$) | 0,15 mM Tannin | 1,1 mM Tannin |
| $M_r$ nativ | 300.000 Da | 100.000 Da |
| $M_r$ denaturiert | 76.000 Da | 54.000 Da |
| $M_r$ theoretisch | 64.191 Da | 48.993 Da |

Tab. B: Biochemische Charakteristika der aus *A. adeninivorans* isolierten Tannase I und Tannase II. Als pH bzw. thermische Stabilität wurden die pH- bzw. Temperatur-Bereiche bezeichnet, wo das Enzym mehr als 95% seiner Restaktivität aufweist. Die Michaelis-Menten-Konstante wurde mittels Lineweaver-Burk (1/S - 1/V) Auftragung berechnet, wobei die Reaktionsgeschwindigkeit [V] in Gegenwart unterschiedlicher Tanninkonzentrationen [S] gemessen wurde.

**Beispiel 2**

Isolierung und Charakterisierung der Tannasegene

1. *ATAN1*-Gen

1.1. Genisolierung

Durch Sequenzierung des N-terminalen Endes der Tannase I bzw. eines nach tryptischer Spaltung erhaltenen Polypeptides, Ableitung von degenerierten Nukleinsäureprimern und PCR, konnte zunächst ein ca. 0,9 kbp großes DNA-Fragment der chromosomalen *Arxula* DNA amplifiziert werden. Nach Sequenzierung ließen sich aus den Nukleinsäuredaten weitere PCR-Primer ableiten, die zur Isolierung des gesamten *ATAN1*-Gens einschließlich Promotor- und Terminator-Region dienten.

Für die Genisolierung der noch nicht bekannten DNA-Bereiche (5'- und 3'-Bereich des *ATAN1*-Gens, Promotor und Terminator des *ATAN1*-Gens) wurde der TOPO-Walker-Kit der Firma Invitrogen (USA) eingesetzt. Essentiell bei dieser Methode ist, dass ein bereits bekanntes, sequenziertes DNA Stück zur Verfügung steht (0,9 kbp DNA-Fragment mit einem Teil des *ATAN1*-Gens), von dem sich entsprechende PCR-Primer ableiten lassen.

Es wurde zuerst die chromosomale DNA von *A. adeninivorans* LS3 mit Restriktionsendonukleasen gespalten, die Fragmente mit 3'-überstehenden Enden erzeugen. An diese wurde, nach Entfernung des Phosphatrestes, der entsprechende, von der bekannten Nukleinsäuresequenz abgeleitete PCR-Primer angelagert und eine erste Auffüllreaktion mittels DNA-Polymerase durchgeführt. Es schließt sich das Ansetzen des so genannten TOPO-Linkers an das 3'-Ende der aufgefüllten DNA-Stücke an. Dieser Linker enthält zwei Primersequenzen, die für die nachfolgende PCR genutzt werden können. Dazu wird ein zweiter Primer aus dem bereits bekannten, sequenzierten DNA-Stück vom *ATAN*-Gen und ein vom TOPO-Linker abgeleiteter Primer eingesetzt. Die dazwischenliegende unbekannte DNA-Sequenz kann nun amplifiziert und die DNA-Sequenz in der nachfolgenden Sequenzierung ermittelt werden.

Mit Hilfe dieser Methode konnte ein 1968 bp großes DNA-Fragment isoliert werden, auf dem sich das *ATAN1*-Gen befindet (schraffierender Bereich von Fig. 2).

Anhand der Nukleinsäuresequenz konnten Promotor-Region, *ATAN1*-Gen und Terminator-Region identifiziert werden. So ließ sich auf den 94 bp umfassenden Promotor die potentielle CAAT-Box (Pos. Nr. -90 - -87) identifizieren. Das Gen selbst enthält 1764 bp, die ein Protein von 587 Aminosäuren codieren. In der Aminosäuresequenz ließ sich die für die Sezemierung ins Kultivierungsmedium essentielle Signalsequenz (Pos. 1-28) und 8 potentielle N-Glycosylierungsort nachweisen. Diese Daten stimmen mit denen der Protein-Charakterisierung überein. So ist die über die N-terminale Sequenzierung erhaltene Aminosäuresequenz der reifen Tannase I GSSLAEVCTSS der N-Terminus dieses Enzyms (Fig. 3).

1.2. Homologie-Vergleich der erhaltenen Tannase I Sequenz

Der Vergleich der Aminosäuresequenz von Atan1p (Tannase 1) mit den in Datenbanken (NCBI-Datenbank) verfügbaren Sequenzen zeigte Homologien mit nur 3 pilzlichen Proteinen; hypothetisches Protein AN9203.2 von *Asp. nidulans* (47%), Tannase von *Asp. oryzae* (47%), hypothetisches Protein AN2697.2 von *Asp. nidulans* (44%) (Fig. 3).

1.3. Lokalisation des *ATAN1-Gens* in der chromosomalen DNA von *A. adeninivorans*

1.3.1. Lokalisation des *ATAN1-Gens* auf den *Arxula*-Chromosomen

Es war zu prüfen, auf welchem der vier *Arxula*-Chromosomen das *ATAN1*-Gen lokalisiert ist. Dazu wurden die Chromosomen von 6 *Arxula*-Wildtypstämmen mittels Pulsed-Field-Gelelektrophorese getrennt, auf Nitrozellulose geblottet und mit dem radioaktiv markierten *ATAN1*-Gen-Fragment hybridisiert.

Es zeigte sich, dass das *ATAN1*-Gen auf dem Chromosom IV aller getesteten *Arxula* Wildtyp-Stämme lokalisiert ist. Im Gegensatz zu *A. adeninivorans* konnten mit den Chromosomen von *S. cerevisiae* S288C keine Hybridisierungssignale erhalten werden (Fig. 5).

1.3.2. Bestimmung der Genkopiezahl

Zur Bestimmung der Genkopienzahl in *A. adeninivorans* ließ sich die Southern-Hybridisierung nutzen. Dazu wurde die chromosomale DNA von *A. adeninivorans* LS3 restringiert, gelelektrophoretisch getrennt, auf Nitrozellulose geblottet und mit dem *ATAN1*-Gen enthaltenen DNA-Fragment hybridisiert. Die Experimente zur Southern Hybridisierung zeigten, dass das *ATAN1*-Gen als ein unikales Gen im Genom von *A. adeninivorans* LS3 vorliegt (Fig. 6).

1.4. Regulation des *ATAN1*-Gens

1.4.1. Transkription

Für die Untersuchung zur Regulation des *ATAN1*-Gens wurde die Northern-Hybridisierung eingesetzt. Dazu wurde *A. adeninivorans* LS3 für 48h in HMM + 2% Glukose kultiviert, anschließend auf neues HMM + 0,5% Glukose, Tween 20, Gallussäure bzw. Tannin umgesetzt und weiter kultiviert. Aus den erhaltenen Hefezellen wurde die RNA isoliert und für die Northern Hybridisierung eingesetzt. Als radioaktiv markierte Sonden wurden DNA-Fragmente mit dem *TEF1*-Gen (Kontrolle) bzw. *ATAN1*-Gen verwendet.

Bei der Hybridisierung mit dem *ATAN1*-Gen konnte 30 min nach dem Umsetzen von HMM + Glukose in in HMM + Gallussäure ein 1.8 kb großes Transkript detektiert werden. Während der weiteren Kultivierung steigt die Transkriptkonzentration weiter an und erreicht 4 h nach dem Umsetzen ihren Maximalwert, der bis zu 16 h konstant bleibt. Erst nach dieser Zeit kommt es wieder zur Verringerung der *ATAN1*-Transkriptakkumulation in *A. adeninivorans.* 24 h nach dem Umsetzen in HMM + 0,5% Gallussäure lässt sich kein *ATAN1*-Transkript mehr nachweisen. Einen ähnlichen Verlauf zeigt sich nach Umsetzen der Hefezellen von HMM + Glukose auf HMM + Tannin. Hier ist allerdings die Transkriptbildung etwas verzögert. So wird der Maximalwert erst nach 4 h erreicht. Erfolgt hingegen das Umsetzen der Hefezellen in HMM + Glukose bzw. HMM + Tween 20, so kommt es nicht zur *ATAN1*-Transkriptakkumulation, d. h. das *ATAN1-Gen* wird unter diesen Bedingungen nicht exprimiert.

Die als Kontrolle durchgeführten Hybridisierungen mit dem *TEF1*-Gen, das unabhängig von der Medienzusammensetzung konstitutiv exprimiert wird, belegen die Intaktheit der aus *A. adeninivorans* isolierten RNA. Hier lässt sich zu jedem Zeitpunkt *TEF1*-Transkript nachweisen (Fig. 7).

Somit handelt es sich beim *ATAN1*-Gen um ein durch Tannin und Gallussäure induzierbares Gen.

1.4.2. Atan1p Akkumulation

Um nachzuweisen unter welchen Bedingungen Atan1p (Tannase I) von der Hefezelle in hohen Konzentrationen ins Medium sezerniert wird, wurde ein anti-Atan1 p-Antikörper hergestellt.

Zur Analyse der Atan1p-Akkumulation wurden die Hefezellen für 48h in HMM + 2% Glukose kultiviert, anschließend in HMM unter Zusatz verschiedener C-Quellen umgesetzt und nach bestimmten Zeiten Proben aus dem Überstand dieser Zellkulturen entnommen. Anschließend wurden die im Kultivierungsmedium enthaltenen Proteine 10-fach konzentriert, im PAGE-Gel getrennt, auf eine Nitrozellulose-Membran geblottet und mit anti-Atan1 p-Antikörpem behandelt.

Die dabei erhaltenen Ergebnisse belegen, dass Atan1p bei Anzucht der Hefezellen in HMM + Tannin bzw. HMM + Gallussäure synthetisiert und ins Kulturmedium sezerniert wird. Bereits nach 4stündiger Kultivierung auf HMM + 0,5% Gallussäure bzw. nach 12stündiger Kultivierung auf HMM + 0,5% Tannin kann Atan1p im Kultivierungsmedium nachgewiesen werden. Bei der Anzucht der Hefezellen in HMM + Glukose bzw. HMM + Protocatechusäure (PCA) erfolgt keine bzw. nur eine sehr schwache Atan1 p Akkumulation (Fig. 8).

1.5. Überexpression des *ATAN1*-Gens in *A.adeninivorans*

1.5.1. Plasmidkonstruktion

Um Tannase unabhängig von der Zusammensetzung des Kultivierungsmedium in möglichst hohen Konzentrationen ins Medium zu sezernieren, muss das isolierte *ATAN1*-Gen in *A. adeninivorans* über-exprimiert werden. Dazu wird es in eine Expressionskassette mit *TEF1*-Promotor (*Arxula*) - *PHO5*-Terminator (*S. cerevisiae*) eingebaut (Rösel & Kunze 1998, Wartmann et al. 2002). Bei dem *TEF1*-Promotor handelt es sich um einen sehr starken konstitutiven Promotor, der alle Voraussetzungen für eine sehr gute *ATAN1*-Genexpression erfüllt. Die erhaltene Kassette mit *TEF1*-Promotor - *ATAN1*-Gen - *PHO5*-Terminator wird anschließend in das *Arxula* Plasmid pAL-ALEU2m eingebaut, welches nach Linearisierung mit *Esp*3I direkt in *A. adeninivorans* G1211 [*aleu2*], transformiert werden kann. Die erhaltenen Transformanten haben dieses Plasmid stabil in der 25S rDNA-Region integriert (Rösel & Kunze 1998, Wartmann et *al*. 2003 - Fig. 9).

1.5.2. Charakterisierung *der A. adeninivorans* Transformanten

Die Transformanten *A. adeninivorans* G1211/pAL-ALEU2m-ATAN1 werden über ihre Komplementation der *aleu2*-Mutation selektiert. Das bedeutet, dass sie ohne Zugabe in HMM wachsen. Da die *Arxula* Transformanten das Plasmid mit der *ATAN1*-Expressionskassette in ein oder mehreren Kopien im Genom integriert haben können und die *ATAN1*-Expression mit der Plasmidkopiezahl korreliert, müssen im nun folgenen Screening die Transformanten mit den höchsten Kopiezahlen ermittelt werden.

2. *ATAN2*-Gen

2.1. Genisolierung

Durch Sequenzierung des N-terminalen Endes der Tannase II bzw. zweier nach tryptischer Spaltung erhaltener Polypeptide, Ableitung von degenerierten Nukleinsäureprimern und PCR, konnte ein ca. 1,1 kbp großes DNA-Fragment von der chromosomalen *Arxula* DNA amplifiziert werden. Nach Sequenzierung liessen sich aus den erhaltenen Nukleinsäuredaten weitere PCR-Primer ableiten und so das gesamte *ATAN2*-Gen einschließlich Promotor- und Terminator-Region isolieren.

Auch für die Isolierung des gesamten *ATAN2-Gens* ließ sich der TOPO-Walker-Kit der Firma Invitrogen (USA) einsetzen. So konnte das gesamte Gen einschließlich Promotor-und Terminator-Region auf einem 3,0 kbp DNA-Fragment von *A. adeninivorans* LS3 identifiziert werden (Fig. 10).

Anhand der Nukleinsäuresequenz konnten Promotor-Region, *ATAN2*-Gen und Terminator-Region detailliert analysiert werden. So ließ sich auf den 624 bp umfassenden Promotor die potentielle TATA- (Pos.-Nr. -66 - -59) und CAAT-Box (Pos. Nr. -41 - -38) identifizieren. Das Gen selbst enthält 1347 bp, die ein Protein von 448 Aminosäuren codieren. In der Aminosäuresequenz ließ sich die für die Sezemierung ins Kultivierungsmedium essentielle Signalsequenz (Pos. 1-18) und nur ein N-Glycosylierungsort nachweisen. Diese Daten stimmen mit denen der Protein-Charakterisierung überein. So ist die über die N-terminale Sequenzierung erhaltene Aminosäuresequenz der reifen Tannase II DAVTPPSEDPFY der N-Terminus dieses Enzyms. Als Besonderheit muss der relativ geringe Glycosylierungsgrad angesehen werden. So unterscheidet sich das aus der Aminosäuresequenz berechnete Molekulargewicht von dem Molekulargewicht der denaturierten Tannase II lediglich um ca. 7 kDa, was durch die Existenz von nur einer N-Glycosylierungsstelle zum Ausdruck kommt (Fig. 11).

2.2. Homologie-Vergleich der erhaltenen Tannase II Sequenz

Der phylogenetische Stammbaum von Atan2p zeigt, das das Protein zu Lipasen von *Candida albicans* und *Gibberella zeae* homolog ist (Fig. 12).

2.3. Lokalisation des *ATAN2-Gens* in der chromosomalen DNA von A. *adeninivorans*

2.3.1. Lokalisation des *ATAN2*-Gens auf den *Arxula*-Chromosomen

Auch beim *ATAN2-Gen* war zu prüfen, auf welchem der vier *Arxula*-Chromosomen dieses Gen lokalisiert ist. Dazu wurden die Chromosomen von 8 *Arxula*-Wildtypstämmen mittels Pulsed-Field-Gelelektrophorese getrennt, auf Nitrozellulose geblottet und mit dem radioaktiv markierten *ATAN2*-Gen-Fragment hybridisiert.

Es zeigte sich, dass das *ATAN2*-Gen auf dem Chromosom I aller getesteten *Arxula* Wildtyp-Stämme lokalisiert ist. Im Gegensatz zu *A. adeninivorans* konnten mit den Chromosomen von *S. cerevisiae* S288C keine Hybridisierungssignale erhalten werden (Fig. 13).

2.3.2. Bestimmung der Genkopiezahl

Zur Bestimmung der *ATAN2* Genkopienzahl in *A. adeninivorans* ließ sich die Southern-Hybridisierung nutzen. Dazu wurde die chromosomale DNA von *A. adeninivorans* LS3 restringiert, gelelektrophoretisch aufgetrennt, auf Nitrozellulose geblottet und mit dem *ATAN2*-Gen enthaltenen DNA-Fragment hybridisiert.

Die Experimente zur Southern Hybridisierung zeigten, dass das *ATAN2*-Gen als ein unikales Gen im Genom von *A. adeninivorans* LS3 vorliegt (Fig. 14).

2.4. Regulation des *ATAN2*-Gens

2.4.1. Transkription

Für die Untersuchung zur Regulation des *ATAN2*-Gens wurde die Northern-Hybridisierung eingesetzt. Dazu wurde *A. adeninivorans* LS3 für 48h in HMM + 2% Glukose kultiviert, anschließend in neues HMM + 0,5% Glukose, Tween 20, Gallussäure bzw. Tannin umgesetzt und weiter kultiviert. Aus den erhaltenen Hefezellen wurde die RNA isoliert und für die Northem Hybridisierung eingesetzt. Als radioaktiv markierte Sonden wurden DNA-Fragmente mit dem *TEF1*-Gen (Kontrolle) bzw. *ATAN2*-Gen verwendet.

Bei der Hybridisierung mit dem *ATAN2*-Gen konnte 30 min nach dem Umsetzen von HMM + Glukose in HMM + Gallussäure ein 1.3 kb großes Transkript detektiert werden. Während der weiteren Kultivierung steigt die Transkriptkonzentration weiter an und erreicht 2 h nach dem Umsetzen ihren Maximalwert, der bis zu 24 h konstant bleibt. Ein ähnlichen Verlauf zeigt sich nach Umsetzen der Hefezellen von HMM + Glukose auf HMM + Tannin bzw. HMM + Tween 20. Hier ist allerdings die Transkriptbildung etwas verzögert. So wird der Maximalwert erst nach 4-8 h erreicht, bleibt dann bis 20 h konstant und fällt anschließend wieder ab. Erfolgt hingegen das Umsetzen der Hefezellen in HMM + Glukose, so kommt es zu einer geringen *ATAN2*-Transkript-akkumulation, d.h. das *ATAN2*-Gen wird unter diesen Bedingungen nur gering exprimiert.

Das *ATAN2* Gen, welches die Tannase II codiert, ist neben Gallotannin und Gallussäure auch durch weitere C-Quellen wie Tween 20, 40, 60 und 80 induzierbar.

Die als Kontrolle durchgeführten Hybridisierungen mit dem *TEF1*-Gen, das unabhängig von der Medienzusammensetzung konstitutiv exprimiert wird, belegen die Intaktheit der aus *A. adeninivorans* isolierten RNA. Hier lässt sich zu jedem Zeitpunkt *TEF1*-Transkript nachweisen (Fig. 15).

Somit handelt es sich beim *ATAN2-Gen* um ein durch Tannin, Gallussäure und Tween 20 induzierbares Gen.

2.4.2. Atan2p-Akkumulation

Um nachzuweisen unter welchen Bedingungen Atan2p (Tannase II) von der Hefezelle in hohen Konzentrationen ins Medium sezerniert wird, wurde ein anti-Atan2p-Antikörper hergestellt.

Zur Analyse der Atan2p-Akkumulation wurden die Hefezellen für 48h in HMM + 2% Glukose kultiviert, anschließend in HMM unter Zusatz verschiedener C-Quellen kultiviert und nach bestimmten Zeiten Proben aus dem Überstand dieser Zellkulturen entnommen. Anschließend wurden die im Kultivierungsmedium enthaltenen Proteine 10-fach konzentriert, im PAGE-Gel getrennt, auf eine Nitrozellulose-Membran geblottet und mit anti-Atan2p-Antikörpem behandelt.

Es ist zu erkennen, dass Atan2p besonders bei Anzucht der Hefezellen in HMM + Tannin bzw. HMM + Gallat synthetisiert und ins Kulturmedium sezerniert wird. Bereits nach 4stündiger Kultivierung auf HMM + 0,5% Gallat bzw.

nach 16stündiger Kultivierung auf HMM + 0,5%Tannin kann Atan2p mit den anti-Atan2p Antikörpern im Kultivierungsmedium nachgewiesen werden. Bei der Anzucht der Hefezellen in HMM + Glukose bzw. HMM + Protocatechusäure (PCA) erfolgt keine bzw. nur eine schwache Atan2p Akkumulation nach 32stündiger Kultivierung (Fig. 16).

2.5. Überexpression des *ATAN2-Gens* in *A.adeninivorans*

2.5.1. Plasmidkonstruktion

*Das ATAN2-Gen* ist in *A. adeninivorans* überexprimiert und ein Stamm konstruiert worden, der Tannase unabhängig von der Zusammensetzung des Kultivierungsmediums in möglichst hohen Konzentrationen ins Medium sezerniert. Dazu wurde das *ATAN2-Gen* in eine Expressionskassette mit *TEF1*-Promotor (*Arxula*) - *PHO5*-Terminato (S. *cerevisiae*) eingebaut (Rösel & Kunze 1998, Wartmann et *al*. 2002). Bei dem *TEF1*-Promotor handelt es sich um einen sehr starken konstitutiven Promotor, der alle Voraussetzungen für eine sehr gute *ATAN2*-Genexpression erfüllt. Die erhaltene Kassette mit *TEF1*-Promotor - *ATAN2*-Gen - *PHO5*-Terminator wurde anschließend in das *Arxula* Plasmid pAL-ALEU2m eingebaut, welches nach Linearisierung mit *Bgl*II direkt in *A. adeninivorans* G1211 [*aleu2*], transformiert werden kann. Die erhaltenen Transformanten sollten dieses Plasmid stabil in der 25S rDNA-Region integriert haben (Rösel & Kunze 1998, Wartmann et *al*. 2003 - Abb. 17). Das Plasmid wurde konstruiert und in *A. adeninivorans* G1211 transformiert.

2.5.2. Charakterisierung der *A. adeninivorans* Transformanten auf rekombinante Atan2p

Die Transformanten *A. adeninivorans* G1211/pAL-ALEU2m-ATAN2 wurden über ihre Komplementation der *aleu2*-Mutation selektiert. Das bedeutet, dass sie in HMM ohne Zugabe von Leucin wachsen. Um diejenigen auswählen zu können, die besonders viel Tannase ins Medium sezernieren, wurde eine hohe Anzahl an Transformanten auf Tannase-Sekretion gescreent.

Zur Analyse der rekombinanten Atan2p-Akkumulation wurden die Transformanten in HMM + 2% Glukose bzw. Xylitol kultiviert und nach bestimmten Zeiten Proben aus dem Überstand dieser Zellkulturen entnommen. Die in diesen enthaltenen Proteine wurden im PAGE-Gel getrennt, auf eine Nitrozellulose-Membran geblottet und mit anti-Atan2p-Antikörpern behandelt.

Hier zeigte sich, dass im Gegensatz zum nicht-transformierten Stamm Atan2p ins Kulturmedium sezerniert wird. Bereits nach 36stündiger Kultivierung auf HMM + Glukose bzw. HMM + Xylitol kann rekombinantes Atan2p mit den anti-Atan2p Antikörpern im Kultivierungsmedium nachgewiesen werden.

2.5.3. Charakterisierung der *A. adeninivorans* Transformanten auf rekombinante Lipaseaktivität

Da Atan2p eine sehr hohe Homologie zu Lipasen aufweist, wurde *A. adeninivorans* G1211/pAL-ALEU2m-ATAN2 parallel auf sekretorische Lipase-Aktivität mit *p*-Nitrophenyl-Caprate geprüft. Dazu erfolgte eine Anzucht in HMM + 2% Glukose bzw. HMM + 2% Xylitol, Bedingungen unter denen das native *ATAN2*-Gen nicht exprimiert wird, d. h. auch kein natives sekretorisches Atan2p auftritt. Die Wachstumsdichte wurde über Extinktionsmessungen bei 600nm verfolgt und die sekretorische Lipase-Aktivität mit Hilfe von *p*-Nitrophenyl-Caprate gemessen (Fig. 18).

*A. adeninivorans* G1211/pAL-ALEU2m-ATAN2 30/30 wurde dazu im Schüttelkolben mit 100ml HMM + entsprechender Zucker kultiviert. Die dabei erhaltenen maximalen Lipaseaktivitäten im Kultivierungsmedium sind in Tab. C dargestellt. Im Gegensatz dazu wies der als Kontrollstamm mitgeführte *A. adeninivorans* G1211/pAL-ALEU2m keine Lipaseaktivität auf.

**Tabelle C:**

| Medium (HMM +) | Proteinkonzentration (mg) | Spezifische Aktivität (E/mg) | Gesamtaktivität (E/100ml HMM) |
|---|---|---|---|
| 2% Glukose | 5,99 | 36,19 | 206 |
| 2% Xylitol | 6,27 | 43,70 | 265 |

**Tab. C:** Lipaseaktivitäten im Kultivierungsmedium von *A. adeninivorans* G1211/pAL-ALEU2m-ATAN2. Dazu wurde die Hefe-Transformante in HMM + Glukose bzw. HMM + Xylitol kultiviert, das Kultivierungsmedium von den Zellen per Zentrifugation getrennt und die darin enthaltene Proteinkonzentration bzw. Enzymaktivität ermittelt. 1 Einheit ist die Menge an Enzym, die zur vollständigen Spaltung von 1,0 μmol p-Nitrophenol-Caprate bei 30°C und pH 7,5 notwendig ist.

Damit war bewiesen, dass die Tannase II auch eine Lipaseaktivität aufweist. Das Substratspektrum dieser Lipase wurde ermittelt. So spaltet die Lipase besonders Nitrophenolate bzw. Triacylglyceride, z.B. pNP-Caprylate (C8)

und pNP-Caprate (C10). Die höchsten Lipaseaktivitäten werden gegenüber Lipiden mit mittlerer Kettenlänge der Fettsäure (C10-C12) erreicht. Substrate mit geringeren und höheren Kettenlängen werden zwar von der Lipase gespalten, allerdings mit geringerer Aktivität (Fig. 19). Ein Unterschied zwischen nativer und rekombinanter Lipase ließ sich nicht feststellen (z.B. native Lipase: $K_M$ (pNP-Caprate = 0,40 mM, $v_{max}$ = 0,043 µmol x $min^{-1}$; rekombinante Lipase: $K_M$ (pNP-Caprate = 0,71 mM, $v_{max}$ = 0,036 µmol x $min^{-1}$).

Die Tannase II liegt ebenfalls extrazellulär vor, was deren biotechnologische Gewinnung vereinfacht.

## Patentansprüche

1. Nukleinsäure, umfassend eine aus der folgenden Gruppe ausgewählte Nukleinsäuresequenz:

    a) Nukleinsäuresequenz nach SEQ ID NO: 1;
    b) Nukleinsäuresequenz, die für das Polypeptid nach SEQ ID NO: 3 kodiert.

2. Expressionskonstrukt, umfassend eine Nukleinsäure gemäß Anspruch 1, wobei die Nukleinsäuresequenz operativ mit einer regulatorischen Nukleinsäuresequenz verbunden ist, die Signale für die Transkriptions- und/oder Translationssteuerung enthält und die Expression der Nukleotidsequenz in der Wirtszelle kontrolliert.

3. Wirtszelle, die das Expressionskonstrukt gemäß Anspruch 2 umfasst.

4. Wirtszelle nach Anspruch 3, die eine prokaryotische Zelle ist.

5. Wirtszelle nach Anspruch 3, die eine eukaryotische Zelle ist.

6. Wirtszelle nach Anspruch 3 oder 5, die eine nicht-menschliche Zelle ist.

7. Wirtszelle nach Anspruch 5 oder 6, die eine Hefe ist.

8. Wirtszelle nach Anspruch 7, die *Arxula adeninivorans* ist.

9. Verfahren zum Herstellen eines Polypeptids mit Tannase-Aktivität, umfassend die folgenden Schritte: a) Kultivieren einer Wirtszelle gemäß einem der Ansprüche 3-8 unter für eine Expression des Polypeptids geeigneten Bedingungen, b) Anreichern des Polypeptids.

10. Verfahren gemäß Anspruch 9, wobei die Wirtszelle *Arxula adeninivorans* ist und das Polypeptid isoliert und aufgereinigt wird.

11. Verfahren gemäß einem der Ansprüche 9 oder 10, wobei das Polypeptid in hohen Mengen in das Kulturmedium sezerniert wird.

**12.** Polypeptid mit Tannase-Aktivität, herstellbar mittels eines Verfahrens gemäß einem der Ansprüche 9, 10 oder 11.

**13.** Polypeptid mit Tannase-Aktivität, umfassend die in SEQ ID NO: 3 angegebene Sequenz, umfassend optional weitere aminoterminale Methionine.

**14.** Polypeptid, das durch die Nukleinsäuren gemäß Anspruch 1 kodiert wird.

**15.** Antikörper oder Fragment davon, der spezifisch an ein Polypeptid gemäß einem der Ansprüche 12-14 bindet.

**16.** Antikörper gemäß Anspruch 15, der ein monoklonaler Antikörper ist.

**17.** Hybridoma-Zelllinie, die einen monoklonalen Antikörper gemäß Anspruch 16 bildet.

**18.** Biosensor, der ein Polypeptid gemäß einen der Ansprüche 12-14 oder ein Polypeptid, gewonnen mittels des Verfahrens gemäß Anspruch 9-11, umfasst.

**19.** Unlösliches Trägermaterial, an das ein Polypeptid gemäß einem der Ansprüche 12-14 oder ein Polypeptid, gewonnen mittels des Verfahrens gemäß Anspruch 9-11, gebunden ist.

**20.** Enzymatische Zusammensetzung, die ein Polypeptid gemäß einem der Ansprüche 12-14 oder ein Polypeptid, gewonnen mittels des Verfahrens gemäß Anspruch 9-11, umfasst.

**21.** Transgene Pflanze, enthaltend eine Nukleinsäure gemäß Anspruch 1.

**22.** Transgene Pflanze, die das Polypeptid gemäß einem der Ansprüche 12-14 überexprimiert.

**23.** Verwendung einer transgenen Pflanze gemäß Anspruch 21 oder 22 bei der Herstellung von Tannin-reduzierten Nahrungsmitteln oder Futtermitteln.

**24.** Verfahren zur Detektion oder Quantifizierung von Tannase in einer Probe, das das Kontaktieren einer Probe, von der angenommen wird, dass sie Tannase enthält, mit einem spezifischen Tannase-Antikörper oder Fragment desselben, gemäß einem der Ansprüche 15-16 umfasst, sowie die Detektion der Bindung des Antikörpers oder Fragments.

**25.** Verfahren zur Detektion oder Quantifizierung von Tanninen in einer Probe, umfassend:

(i) das Kontaktieren einer Probe, von der angenommen wird, dass sie Tannine enthält, mit dem Biosensor gemäß Anspruch 18, sowie (ii) die Detektion von Tanninen.

**26.** Verfahren zur Reduktion der Menge an Tanninen in einer Zusammensetzung, umfassend das Kontaktieren der Zusammensetzung mit einem Polypeptid gemäß einem der Ansprüche 12-14 oder einem Polypeptid, gewonnen mittels des Verfahrens gemäß Anspruch 9-11, mit der Wirtszelle gemäß einem der Ansprüche 3-8, mit dem unlöslichen Trägermaterial gemäß Anspruch 19 oder mit der enzymatischen Zusammensetzung gemäß Anspruch 20.

**27.** Verfahren gemäß Anspruch 26 zur Reduktion der Menge an Tanninen in Nahrungsmitteln, Futtermitteln und/oder in einer mit diesen Stoffen kontaminierten Zusammensetzung.

**28.** Verfahren gemäß Anspruch 27 zur Reduktion der Menge an Tanninen in landwirtschaftlichen und industriellen Abwässern.

**29.** Verfahren gemäß Anspruch 27 zur Verwendung innerhalb der Chemie-, Pharma-, Nahrungsmittel-, Waschmittel-, Leder-, Papier- und/oder Kosmetikindustrie.

**30.** Verwendung des Polypeptids gemäß einem der Ansprüche 12-14, der Wirtszelle gemäß einem der Ansprüche 3-9, des unlöslichen Trägermaterials gemäß Anspruch 19 oder der enzymatischen Zusammensetzung gemäß Anspruch 20 zur Reduktion der Menge an Tanninen in einer Zusammensetzung.

**31.** Verwendung gemäß Anspruch 30, wobei die Tannin-haltige Zusammensetzung ein Futtermittel, Nahrungsmittel

oder eine mit Tannin kontaminierte Zusammensetzung ist.

**32.** Verwendung gemäß Anspruch 31 zur besseren Futterverwertung durch Nutztiere.

**33.** Verwendung gemäß Anspruch 30, wobei die Tannin-haltige Zusammensetzung landwirtschaftliches oder industrielles Abwasser ist.

**34.** Verwendung des Polypeptids gemäß einem der Ansprüche 12-14 oder des Polypeptids, gewonnen mittels des Verfahrens gemäß Anspruch 9-11 in der Chemie-, Pharma-, Nahrungsmittel-, Waschmittel-, Papier- und/oder Kosmetikindustrie.

**35.** Verwendung des Polypeptids gemäß einem der Ansprüche 12-14 oder des Polypeptids, gewonnen mittels des Verfahrens gemäß Anspruch 9-11, zur Herstellung von Gallussäure.

**36.** Verwendung des Biosensors gemäß Anspruch 18 zur Detektion von Tanninen in Abwässern, Nahrungsmitteln oder Futtermitteln.

## Claims

**1.** Nucleic acid, comprising a nucleic acid sequence selected from the following group:

a) nucleic acid sequence according to SEQ ID NO: 1,
b) nucleic acid sequence that codes for the polypeptide according to SEQ ID NO: 3.

**2.** Expression construct, comprising a nucleic acid according to claim 1, wherein the nucleic acid sequence is operatively linked to a regulatory nucleic acid sequence that contains signals for the transcription control and/or translation control and controls the expression of the nucleotide sequence in the host cell.

**3.** Host cell which comprises the expression construct according to claim 2.

**4.** Host cell according to claim 3, which is a prokaryotic cell.

**5.** Host cell according to claim 3, which is a eukaryotic cell.

**6.** Host cell according to claim 3 or 5, which is a non-human cell.

**7.** Host cell according to claim 5 or 6, which is a yeast.

**8.** Host cell according to claim 7, which is *Arxula adeninivorans.*

**9.** Method for producing a polypeptide with tannase activity, comprising the following steps: a) cultivation of a host cell according to one of the claims 3-8 under conditions suitable for expression of the polypeptide, b) enrichment of the polypeptide.

**10.** Method according to claim 9, wherein the host cell is *Arxula adeninivorans* and the polypeptide is isolated and purified.

**11.** Method according to one of the claims 9 or 10, wherein the polypeptide is secreted in great quantities into the culture medium.

**12.** Polypeptide with tannase activity, which can be produced by means of a method according to one of the claims 9, 10 or 11.

**13.** Polypeptide with tannase activity, comprising the sequence defined in SEQ ID NO: 3, optionally comprising additional aminoterminal methionine.

**14.** Polypeptide that is encoded by the nucleic acids according to claim 1.

**15.** Antibody or fragment thereof, which binds specifically to a polypeptide according to one of the claims 12-14.

**16.** Antibody according to claim 15, which is a monoclonal antibody.

**17.** Hybridoma cell line which forms a monoclonal antibody according to claim 16.

**18.** Biosensor which comprises a polypeptide according to one of the claims 12-14 or a polypeptide obtained by means of the method according to claim 9-11.

**19.** Insoluble carrier material, bound to which is a polypeptide according to one of the claims 12-14 or a polypeptide obtained by means of the method according to claim 9-11.

**20.** Enzymatic composition that comprises a polypeptide according to one of the claims 12-14 or a polypeptide obtained by means of the method according to claim 9-11.

**21.** Transgenic plant containing a nucleic acid according to claim 1.

**22.** Transgenic plant which overexpresses the polypeptide according to one of the claims 12-14.

**23.** Use of a transgenic plant according to claim 21 or 22 in the production of tannin-reduced food products or animal feed.

**24.** Method for the detection or quantification of tannase in a sample, which comprises the contacting of a sample that is assumed to contain tannase with a specific tannase antibody or fragment thereof according to one of the claims 15-16, as well as the detection of the binding of the antibody or fragment.

**25.** Method for the detection or quantification of tannins in a sample, comprising: (i) the contacting of a sample that is assumed to contain tannins with the biosensor according to claim 18, and (ii) the detection of tannins.

**26.** Method for reducing the quantity of tannins in a composition, comprising the contacting of the composition with a polypeptide according to one of the claims 12-14 or with a polypeptide obtained by means of the method according to claim 9-11, with the host cell according to one of the claims 3-8, with the insoluble carrier material according to claim 19 or with the enzymatic composition according to claim 20.

**27.** Method according to claim 26 for reducing the quantity of tannins in food products, animal feed and/or in a composition contaminated with these substances.

**28.** Method according to claim 27 for reducing the quantity of tannins in agricultural and industrial waste water.

**29.** Method according to claim 27 for use within the chemical, pharmaceutical, food, detergent, leather, paper and/or cosmetics industries.

**30.** Use of the polypeptide according to one of the claims 12-14, of the host cell according to one of the claims 3-9, of the insoluble carrier material according to claim 19, or of the enzymatic composition according to claim 20 in order to reduce the quantity of tannins in a composition.

**31.** Use according to claim 30, wherein the composition that contains tannin is an animal feed, food product, or a composition contaminated with tannin.

**32.** Use according to claim 31 for better feed conversion by working animals.

**33.** Use according to claim 30, wherein the composition that contains tannin is agricultural or industrial waste water.

**34.** Use of the polypeptide according to one of the claims 12-14 or of the polypeptide obtained by means of the method according to claim 9-11 in the chemical, pharmaceutical, food, detergent, leather, paper and/or cosmetics industries.

**35.** Use of the polypeptide according to one of the claims 12-14 or of the polypeptide obtained by means of the method according to claim 9-11 for the production of gallic acid.

**36.** Use of the biosensor according to claim 18 for the detection of tannins in waste water, food products or animal feed.

## Revendications

**1.** Acide nucléique comprenant une séquence d'acides nucléiques choisie dans le groupe suivant :

a) une séquence d'acides nucléiques selon la séquence SEQ ID N°: 1 ;
b) une séquence d'acides nucléiques qui code le polypeptide selon la séquence SEQ ID N°: 3.

**2.** Construction d'expression comprenant un acide nucléique selon la revendication 1, dans laquelle la séquence d'acides nucléiques est reliée de manière opérationnelle à une séquence d'acides nucléiques régulatrice qui comporte des signaux de commande de transcription et/ou de traduction génétique et contrôle l'expression de la séquence d'acides nucléiques dans la cellule hôte.

**3.** Cellule hôte qui comporte la construction d'expression selon revendication 2.

**4.** Cellule hôte selon la revendication 3 qui est une cellule procaryote.

**5.** Cellule hôte selon la revendication 3 qui est une cellule eucaryote.

**6.** Cellule hôte selon la revendication 3 ou 5 qui est une cellule non humaine.

**7.** Cellule hôte selon la revendication 5 ou 6 qui est une levure.

**8.** Cellule hôte selon la revendication 7 qui est une cellule d'*Arxula adeninivorans*.

**9.** Procédé de fabrication d'un polypeptide ayant une activité tannase, comprenant les étapes suivantes :

a) culture d'une cellule hôte selon l'une des revendications 3 à 8 sous des conditions appropriées pour l'expression du polypeptide ; b) concentration du polypeptide.

**10.** Procédé selon revendication 9, dans lequel la cellule hôte est une cellule *d'Arxula adeninivorans* et le polypeptide est isolé et nettoyé.

**11.** Procédé selon l'une des revendications 9 ou 10, dans lequel le polypeptide est sécrété en grande quantité dans le milieu de culture.

**12.** Polypeptide à activité tannase, qui peut être fabriqué à l'aide d'un procédé selon l'une des revendications 9, 10 ou 11.

**13.** Polypeptide à activité tannase, comprenant la séquence correspondant à la séquence SEQ ID N°: 3 qui comporte optionnellement des méthionines supplémentaires à l'extrémité amino-terminale.

**14.** Polypeptide codé par les acides nucléiques selon la revendication 1.

**15.** Anticorps ou fragment correspondant qui se lie spécifiquement à un polypeptide selon l'une des revendications 12 à 14.

**16.** Anticorps selon la revendication 15 qui est un anticorps monoclonal.

**17.** Lignée cellulaire d'hybridomes produisant un anticorps monoclonal selon la revendication 16.

**18.** Biocapteur comportant un polypeptide selon l'une des revendications 12 à 14 ou un polypeptide obtenu à l'aide d'un procédé selon l'une des revendications 9 à 11.

**19.** Matériau de support insoluble auquel est lié un polypeptide selon l'une des revendications 12 à 14 ou un polypeptide obtenu à l'aide du procédé selon l'une des revendications 9 à 11.

**20.** Composition enzymatique qui comporte un polypeptide selon l'une des revendications 12 à 14 ou un polypeptide obtenu à l'aide du procédé selon l'une des revendications 9 à 11.

**21.** Plante transgénique comportant un acide nucléique selon la revendication 1.

**22.** Plante transgénique qui sur-exprime le polypeptide selon l'une des revendications 12 à 14.

**23.** Utilisation d'une plante transgénique selon la revendication 21 ou 22 dans la fabrication d'aliments pour humains ou pour animaux à teneur en tannins réduite.

**24.** Procédé de détection ou de quantification de tannase dans un échantillon, qui comporte la mise en contact d'un échantillon, dont on suppose qu'il contient de la tannase, avec un anticorps spécifique de la tannase ou un fragment de celui-ci, selon l'une des revendications 15 à 16, ainsi que la détection de la liaison dudit anticorps ou fragment.

**25.** Procédé de détection ou de quantification de tannins dans un échantillon, comprenant : (i) la mise en contact d'un échantillon, dont on suppose qu'il contient des tannins, avec le biocapteur selon la revendication 18, ainsi que (ii) la détection de tannins.

**26.** Procédé de réduction de la quantité de tannins dans une composition, comprenant la mise en contact de la composition avec un polypeptide selon l'une des revendications 12 à 14 ou un polypeptide obtenu à l'aide d'un procédé selon l'une des revendications 9 à 11 avec la cellule hôte selon l'une des revendications 3 à 8, avec le matériau de support insoluble selon la revendication 19, ou avec la composition enzymatique selon la revendication 20.

**27.** Procédé selon revendication 26 pour la réduction de la quantité de tannins dans des aliments pour humains ou pour animaux, ou dans une composition contaminée par des tannins.

**28.** Procédé selon revendication 27 pour la réduction de la quantité de tannins dans des eaux usées agricoles et industrielles.

**29.** Procédé selon revendication 27 pour une utilisation dans l'industrie chimique, pharmaceutique, alimentaire, des détergents, du cuir, du papier ou des produits cosmétiques.

**30.** Utilisation du polypeptide selon l'une des revendications 12 à 14, de la cellule hôte selon l'une des revendications 3 à 9, du matériau de support insoluble selon la revendication 19 ou de la composition enzymatique selon la revendication 20 pour la réduction de la quantité de tannins présents dans une composition.

**31.** Utilisation selon revendication 30, dans laquelle la composition comportant des tannins est un produit alimentaire pour humains ou pour animaux, ou une composition contaminée par des tannins.

**32.** Utilisation selon revendication 31 pour l'amélioration de l'indice de consommation d'aliments pour animaux d'élevage.

**33.** Utilisation selon revendication 30, dans laquelle la composition contenant des tannins est une eau usée agricole ou industrielle.

**34.** Utilisation du polypeptide selon l'une des revendications 12 à 14 ou du polypeptide obtenu à l'aide d'un procédé selon l'une des revendications 9 à 11, dans l'industrie chimique, pharmaceutique, alimentaire, des détergents, du papier ou des produits cosmétiques.

**35.** Utilisation du polypeptide selon l'une des revendications 12 à 14 ou du polypeptide obtenu à l'aide d'un procédé selon l'une des revendications 9 à 11, pour la production d'acide gallique.

**36.** Utilisation du biocapteur selon la revendication 18 pour la détection de tannins dans les eaux usées ou les aliments pour humains ou pour animaux.

# FIGUR 1

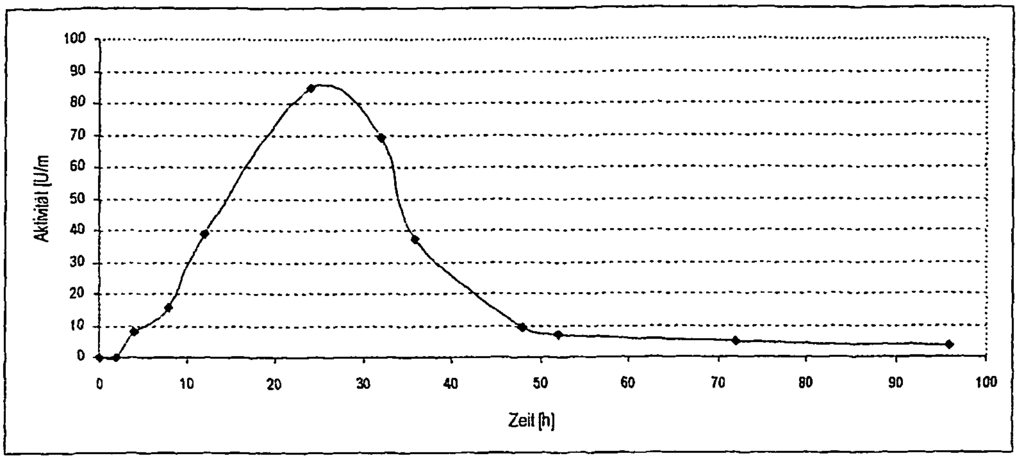
Aktivität [U/m
100
90
80
70
60
50
40
30
20
10
0
0
10
20
30
40
50
60
70
80
90
100
Zeit [h]

# Figur 2

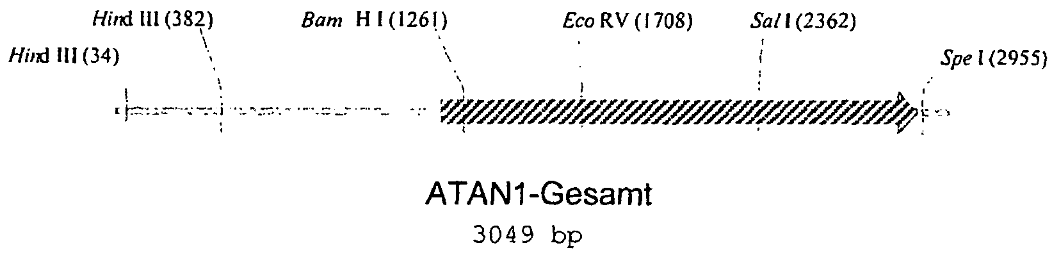
Hind III (382)
Hind III (34)
Bam H I (1261)
Eco RV (1708)
Sal I (2362)
Spe I (2955)
ATAN1-Gesamt
3049 bp

# Figur 3

```
-1173                               cca  tggatcagca  tgccaaaact  gaaggactaa
-1140 agcttttgtt  gcaagattcg caactgtagc  tacgtattat  attatgtttt  taaatgtatt
-1080 aacagtctaa  ataatctacc ctatcagtac  ctggacaagt  tgggtgagct  aaatggacac
-1020 cataagaaaa  agtctccccg aaatgaattt  taaaatacat  ttggtgagta  ttctaaaaac
-960  tttgaaatca  aggcacgatc ttgtcctctg  ggatgtagct  tgccgcagga  aggcacgtat
-900  catctatcgt  gaagcattat ctggaccctg  acctaacgtt  tttccatatt  tagactgata
-840  cgcgtacctg  catggggccg atcagcgccg  atcattcttt  gactctaaag  cttaaaaaaa
-780  tccgttcgcc  tcaagaccac cctattgcct  tggttctcat  ttacgggctt  gatgcatcaa
-720  ccatagttct  ctaggcgtaa gaagaaggga  caaactgacc  tggcctgtga  cccgacactt
-660  tcccaagata  taaagcccgg tccactccaa  agatggtagg  tgataacagt  tccgcatctc
-600  gcgatacatt  cgccaatacc gtaatatgag  aagccttctt  tcgaaccact  taaatatatc
-540  tttacgagta  accctggagt ttgtttcttt  atctgtgatg  gaggtgccag  cagcagctga
-480  tgtagtatca  gccaatggcc gttaatgaga  gggtgcccca  ctcaaataat  tgacgaaatt
-420  tttgaatatc  aagggccaac tcaccagtgt  cctcaataag  agcaccacat  ttactgacct
-360  gcgagaacta  ttccccacaa gtatcggtcc  gagcttccga  aatgatccgc  gcgaccaagt
-300  taaagtttgt  ggggtaatca ggatgaaaat  cttcgggaac  tacgactcta  aattagatag
-240  atcctcaacg  gacagtggaa aggtcgcccg  aaatctaatg  gtataggata  cagttatttg
-180  tcgaggctac  agatcatctg gtgtacaagt  ttcggaacaa  aagcaagggt  gaaggacaaa
-120  aagctacttt  acgcccaata gctgcagact  caattgcagg  cacggtgccg  attacgtacc
-60   cccccatgtg  cttagtgcct ttcatgctac  ggatgttgga  cactataaaa  gatacgtaaa
1     atggcaagca  taccattctt tgttgagatg  aagcattttc  tcggacaatc  tttattgaca
1      M  A  S    I  P  F  F  V  E  M    K  H  F     L  G  G  S   L  L  T
                                    Signalsequenz
61    agtctgcttg  cggcaggagc ctttggatcc  tcgcttgccg  aagtctgtac  ttcctcccgc
21     S  L  L    A  A  G  A  F  G  S    S  L  A     E  V  C  T   S  S  R
                              ▲
121   atccggaccg  ccttaccaaa ggatggagcc  atcgcaggga  tctctatgga  cccagacagt
41     I  R  T    A  L  P  K  D  G  A    I  A  G     I  S  M  D   P  D  S
```

| | | | | | | |
|---|---|---|---|---|---|---|
| 181 | atcactgcca | atccagtgta | taatgcatct | gctggctata | gcgtgtttta | ccccgaggga |
| 61 | I T A | N P V Y | N A S | A G Y | S V F Y | P E G |
| 241 | aactttgatt | actgcaatgt | gactgtttcc | tactgtcata | ttggcaaggg | tgacaaagtc |
| 81 | N F D | Y C N V | T V S | Y C H | I G K G | D K V |
| 301 | aatctgcagt | attggcttcc | tagtccagac | aagttccaaa | accgttacct | ggctacaggc |
| 101 | N L Q | Y W L P | S P D | K F Q | N R Y L | A T G |
| 361 | ggcgggggat | atgccatcaa | ctctggaact | cagtcactgc | ctggaggggt | catgtatgga |
| 121 | G G G | Y A I N | S G T | Q S L | P G G V | M Y G |
| 421 | gcagttgctg | gtagaaccga | tggaggattt | ggagggtttg | atgtccaagt | ttctgaagcc |
| 141 | A V A | G R T D | G G F | G G F | D V Q V | S E A |
| 481 | atcttgtacg | ccaatggatc | tctcaattac | gatagtctat | acatgtttgg | atatcgagca |
| 161 | I L Y | A N G S | L N Y | D S L | Y M F G | Y R A |
| 541 | attggtgagc | agaccatgat | tggccaggag | ttagcgcgag | gattctgtga | attgggggac |
| 181 | I G E | Q T M I | G Q E | L A R | G F C E | L G D |
| 601 | gagaagaaga | tttacacata | ctaccagggg | tgttcggaag | gagtacgtga | aggctggagt |
| 201 | E K K | I Y T Y | Y Q G | C S E | G V R E | G W S |
| | | | | Nukleophiles Serin | | |
| 661 | caaatcctaa | aatttccaga | tctctacgat | ggagtaatcc | ctgctgcccc | tgccttcaga |
| 221 | Q I L | K F P D | L Y D | G V I | P A A P | A F R |
| 721 | tatgggcatc | agcaagtgaa | ccacctgttt | ccaggggtca | tagaacaagg | catgaactat |
| 241 | Y G H | Q Q V N | H L F | P G V | I E Q G | M N Y |
| 781 | taccctccac | cttgtgaaat | ggctcgtatc | gtcaatgcca | caattgaggc | ttgcgacaag |
| 261 | Y P P | P C E M | A R I | V N A | T I E A | C D K |
| 841 | ctggatggca | agatagacgg | agtagtgtcc | aggacagatc | tgtgtctgtt | gaactttgac |
| 281 | L D G | K I D G | V V S | R T D | L C L L | N F D |
| 901 | tttaattcta | caattgggct | ccattacact | tgcgaagcag | gctccaaccc | tatgacggga |
| 301 | F N S | T I G L | H Y T | C E A | G S N P | M T G |
| 961 | gactccaccc | cagcacaaaa | cggtactgtt | tccaccaagg | ctgctgagct | tgctcgggtg |
| 321 | D S T | P A Q N | G T V | S T K | A A E L | A R V |
| 1021 | ttgacagaag | ggctccatga | ttcacaaggc | aacaaggcat | acgtctttta | tcagattacc |
| 341 | L T E | G L H D | S Q G | N K A | Y V F Y | Q I T |
| | | Konserviertes Histidin | | | | |
| 1081 | gccgggtatg | acgatgcaga | caccaagtac | aaccctgcca | ccgggcagtt | tgaattgtca |
| 361 | A G Y | D D A D | T K Y | N P A | T G Q F | E L S |
| 1141 | gtgagcagtc | ttggtggtga | gtgggttaca | aagctcttgc | agcttgtcga | ccttgacaat |
| 381 | V S S | L G G E | W V T | K L L | Q L V D | L D N |
| 1201 | ctaccaaacc | ttgacaatgt | tactgtggac | acgctggttg | attggatgca | atgcggttgg |
| 401 | L P N | L D N V | T V D | T L V | D W M Q | C G W |
| 1261 | caaacttacg | aagatgtgtt | acagacaacc | aggcctgatc | tttctctgta | tgaaagagcc |

```
421   Q  T  Y     E  D  V  L    Q  T  T     R  P  D     L  S  L  Y    E  R  A

1321  ggaggaaaga  tcttgacatt  ccacggggag  tctgacaaca  gcatccctgc  aggatcatca
441   G  G  K     I  L  T  F    H  G  E     S  D  N     S  I  P  A    G  S  S

1381  gtacattttt  acgagtcagt  gagaaacgta  atgtaccctg  gaatctcgtt  taatcaaagc
461   V  H  F     Y  E  S  V    R  N  V     M  Y  P     G  I  S  F    N  Q  S

1441  acagatgcca  tgggcgagtg  gtacaggctc  tatcttgtcc  ccggagctgc  ccattgcagt
481   T  D  A     M  G  E  W    Y  R  L     Y  L  V     P  G  A  A    H  C  S

1501  atcaacgctt  tacaacccaa  tggtccattc  ccacaaacca  cccttgaagt  aatgattgac
501   I  N  A     L  Q  P  N    G  P  F     P  Q  T     T  L  E  V    M  I  D

1561  tgggtagaaa  atggcaatac  tccaaccacc  cttcaggcta  catacttggt  tggtgacaat
521   W  V  E     N  G  N  T    P  T  T     L  Q  A     T  Y  L  V    G  D  N

1621  aagggcaaac  cagctgagat  ttgtccatgg  cccctgcgcc  caacttggac  tgatgaagga
541   K  G  K     P  A  E  I    C  P  W     P  L  R     P  T  W  T    D  E  G

1681  agcaagttac  aatgcgttta  tgatcatacc  tcgatcaata  cctggatgta  tgattttaac
561   S  K  L     Q  C  V  Y    D  H  T     S  I  N     T  W  M  Y    D  F  N

1741  gctttttctc  tacccgtcta  ctaatgtagc  aatactctac  tagtgctcta  tatctaccca
581   A  F  S     L  P  V  Y    *

1801  atgcaccttt  cactatagat  acggtggggt  tgacgaagaa  ggctgaagcc  ttgtgatgag

1861  acgtatttag  ttcc
```

Figur 3: DNA-Sequenz des *ATAN*-Gens von *A. adeninivorans* LS3 und die davon abgeleitete Aminosäuresequenz der Tannase I (Atan1p). Die potentielle CAAT-Box in der Promotor-Region wurde unterstrichen. Zusätzlich wurden vom Atan1p die Signalsequenz (Aminosäure-Pos. 1-28) und die potentiellen N-Glycosylierungsstellen hervorgehoben.

FIGUR 4

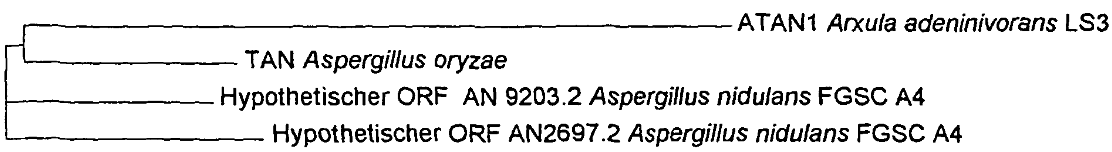
ATAN1 *Arxula adeninivorans* LS3
TAN *Aspergillus oryzae*
Hypothetischer ORF AN 9203.2 *Aspergillus nidulans* FGSC A4
Hypothetischer ORF AN2697.2 *Aspergillus nidulans* FGSC A4

FIGUR 5

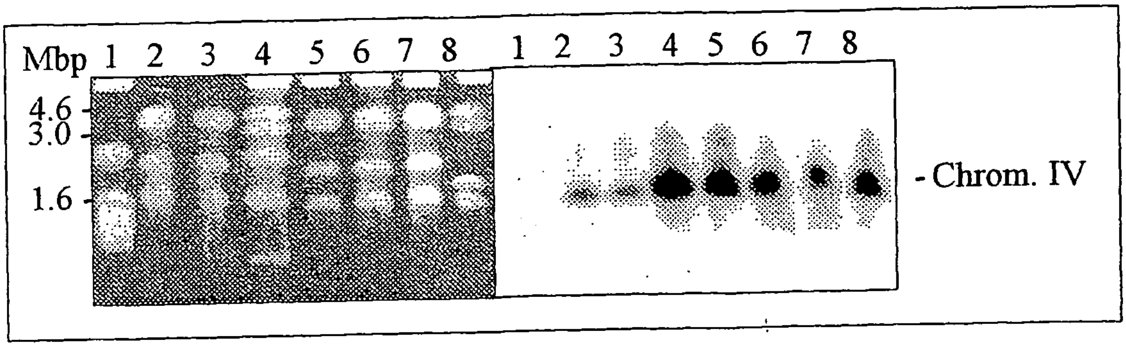

# FIGUR 6

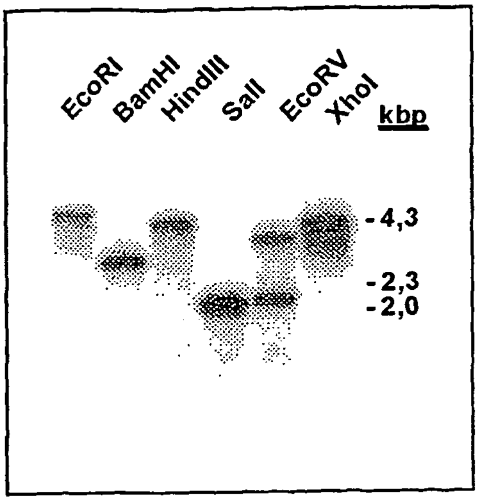

# FIGUR 7

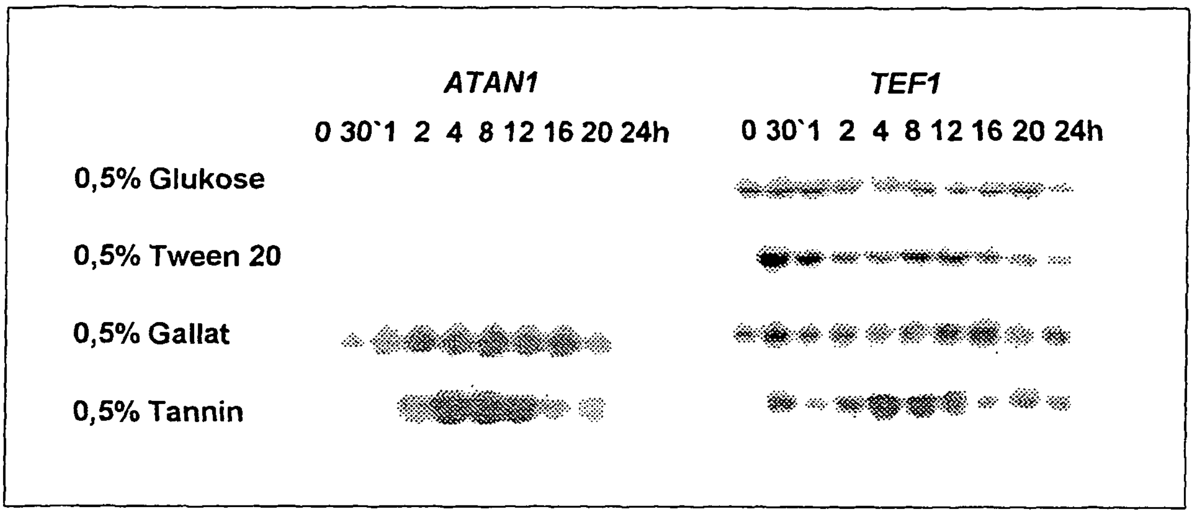
ATAN1
TEF1
0 30`1 2 4 8 12 16 20 24h
0 30`1 2 4 8 12 16 20 24h
0,5% Glukose
0,5% Tween 20
0,5% Gallat
0,5% Tannin

FIGUR 8

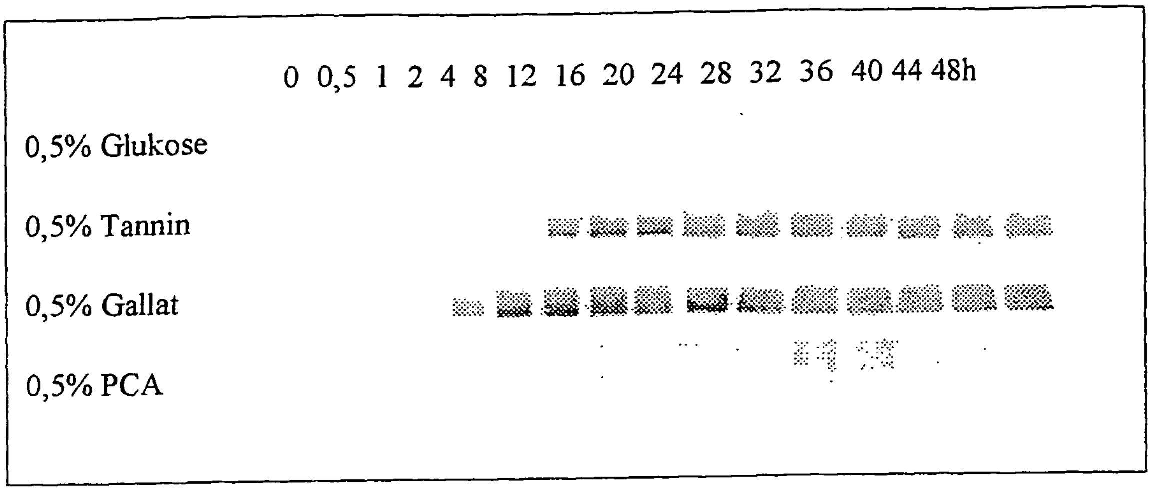
0 0,5 1 2 4 8 12 16 20 24 28 32 36 40 44 48h
0,5% Glukose
0,5% Tannin
0,5% Gallat
0,5% PCA

# FIGUR 9

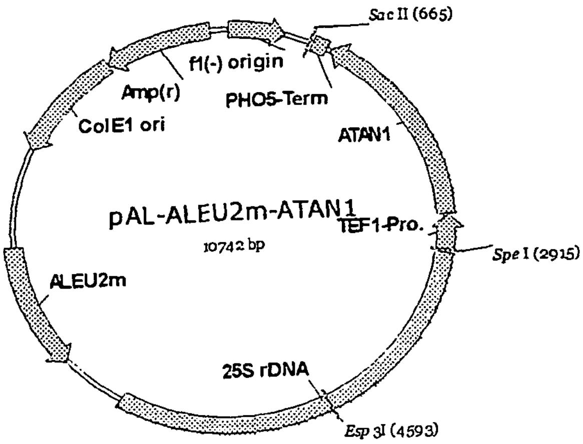
Sac II (665)
f1(-) origin
Amp(r)
PHO5-Term
ColE1 ori
ATAN1
pAL-ALEU2m-ATAN1
10742 bp
TEF1-Pro.
Spe I (2915)
ALEU2m
25S rDNA
Esp 3I (4593)

# FIGUR 10

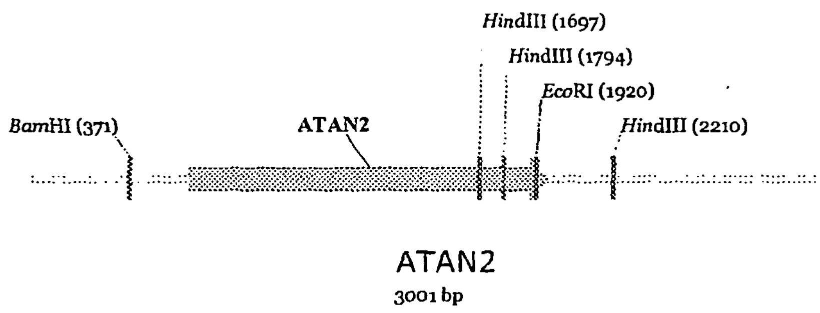
HindIII (1697)
HindIII (1794)
EcoRI (1920)
BamHI (371)
ATAN2
HindIII (2210)
ATAN2
3001 bp

# Figur 11

```
-624                                         tcgc  aattcatctc  ccaagccctt
-600  ttgcttatgg  caagcgaatt  aacaggtggg  tgtacaaatt  gtgacccgcc  caccacaggc
-540  ccaagtattg  tgggctattg  gctgtcaatc  aaccgttatc  gcctcggaag  ttcccctcac
-480  gggcccgtac  cgtttgcgct  caccagcgtg  cccggcctgc  agggttctat  cggcattacc
-420  ccgcaaaacg  gcgcgtgcat  catgtaaaca  gagagtgcag  gggggtgaga  aaaaagagaa
-360  aacccttgaa  tgactgtttg  gtcggcaaaa  cgactcaatc  aacgtcgaac  taattccaaa
-300  cggtcgtggg  atcgtatctc  tgcgatctca  ttgcagttca  tgtccggatc  cacttgaaca
-240  actccggtct  ggaatgaaaa  atttaagggg  tagctctgca  tttctggcgt  ttgggccagc
-180  agacatgcag  atgtgcagaa  tcggtcccgt  tccggggtaa  gattagctat  ggcttttgga
-120  gagatcagca  tagtttacac  gttcatgtgc  gcagataggc  gtgaacaaga  ggtgtatata
-60   atggggtccc  attcgcatgc  aatttaagaa  gtgcgcgatc  ttattgctgg  tcggttgaac

1     atgctgaagc  tcctgctact  tgtggcccaa  ctggtggctt  ttgtatttgg  tgcagcgctg
1      M  L  K    L  L  L  L   V  A  Q    L  V  A    F  V  F  G   A  A  L
                     Signalsequenz
61    cctgaagagc  gtgtatatgc  tcgcgatgct  gtgaccccac  cctctgaaga  tccgttctac
21     P  E  E    R  V  Y  A   R  D  A    V  T  P    P  S  E  D   P  F  Y
         Pro-Sequenz
121   acgccagatg  atggttatga  gaaggaagag  cctggtacaa  ttctgcgatg  gagacacgct
41     T  P  D    D  G  Y  E   K  E  E    P  G  T    I  L  R  W   R  H  A

181   cctcagatgc  ctgcttttac  tgtttttaag  cagaatctta  atgcgtccat  tcagatcctg
61     P  Q  M    P  A  F  T   V  F  K    Q  N  L    N  A  S  I   Q  I  L

241   taccgtacta  ctgatactca  gggacagccc  acggccactg  tggtgactgt  catgatccct
81     Y  R  T    T  D  T  Q   G  Q  P    T  A  T    V  V  T  V   M  I  P

301   cacaaccatg  ccgaaggaaa  gctgctgtcg  taccagtcct  gggaagactc  tgcttggatc
101    H  N  H    A  E  G  K   L  L  S    Y  Q  S    W  E  D  S   A  W  I

361   aactgtgctc  cgtcgtatgc  aattcagttt  ggtgctaacc  ctcaagggat  catttctcag
121    N  C  A    P  S  Y  A   I  Q  F    G  A  N    P  Q  G  I   I  S  Q
          C
421   gtggatatgc  ttactcttca  atccgctctc  aatgagggtt  ggattgtttc  aactcccgat
141    V  D  M    L  T  L  Q   S  A  L    N  E  G    W  I  V  S   T  P  D

481   tatgagggtc  caaagtcttc  gttcacgtca  gccatcatct  ccggacaggc  cactctggac
161    Y  E  G    P  K  S  S   F  T  S    A  I  I    S  G  Q  A   T  L  D

541   tcgattagag  cagtgctcaa  gtcggagtcg  tttaccgggg  tcaagcctga  ttcaaaggta
181    S  I  R    A  V  L  K   S  E  S    F  T  G    V  K  P  D   S  K  V

601   gccatgtggg  gatactctgg  gggatcaatc  gcatcaggtt  gggcagcagc  tctccaaccc
201    A  M  W    G  Y  S  G   G  S  I    A  S  G    W  A  A  A   L  Q  P
                Nukleophiles Serin
661   acgtatgcgc  cagagctcaa  aattgcagga  gcagctctcg  gaggagtaat  ccagaacatt
221    T  Y  A    P  E  L  K   I  A  G    A  A  L    G  G  V  I   Q  N  I
```

```
721   actagtgtgg ccgttcaggt gaataagggc ccgtttgttg gtctggtacc ggctggaatc
241    T  S  V   A  V  Q  V   N  K  G   P  F  V   G  L  V  P   A  G  I

781   aagggattat catcccaata tccagagcta gaagattaca ttaacgatca acttcttccc
261    K  G  L   S  S  Q  Y   P  E  L   E  D  Y   I  N  D  Q   L  L  P

841   gacaagagag atgactttga aaaggccgga aagcagtgtc tttcagtaga cgttctgact
281    D  K  R   D  D  F  E   K  A  G   K  Q  C   L  S  V  D   V  L  T
                                               C
901   tatgcgttcc aggattggtt ttcatacaca aaggctgggg accgagtttt gtacaatgag
301    Y  A  F   Q  D  W  F   S  Y  T   K  A  G   D  R  V  L   Y  N  E

961   accattcaaa aagtcctgga tgagaatgcc atgggaaagc agaaacctca gattccattg
321    T  I  Q   K  V  L  D   E  N  A   M  G  K   Q  K  P  Q   I  P  L

1021  ctgttttacc acggtgtcca tgatgaggtt atgccaattg ctgatgtgga caagctttat
341    L  F  Y   H  G  V  H   D  E  V   M  P  I   A  D  V  D   K  L  Y
        Konserviertes Histidin
1081  tacgagtact gttccaatgg cgttaccgtg gaatactaca gagaggaggg atcagagcat
361    Y  E  Y   C  S  N  G   V  T  V   E  Y  Y   R  E  E  G   S  E  H
                 C
1141  gttcttgaaa tgatcactgg attccctaaa gcttacaatt atgtcaagaa cctgctcgat
381    V  L  E   M  I  T  G   F  P  K   A  Y  N   Y  V  K  N   L  L  D

1201  ggtggctccg tgagctcggg atgccagaga cacgatgtgt tttctaacgc cttcgatgaa
401    G  G  S   V  S  S  G   C  Q  R   H  D  V   F  S  N  A   F  D  E
                              C
1261  gatgcattgc ctacctactc tgccgaaatt tggggaattc tcaagggttt gctcggagcc
421    D  A  L   P  T  Y  S   A  E  I   W  G  I   L  K  G  L   L  G  A

1321  cctgttggac cggctgccat cagttaatct aaactaatgt tggtcctctg ttggtgttat
441    P  V  G   P  A  A  I   S  *

1381  taacgcagtg gttttgactt tgacttctga tcattttatc aaacagtcgt tctgtgaagt

1441  caacagggca tattctcact taatgaagga gctgcgcggt ttcggctcga ataattttgt

1501  ttttggtttt gtttttgttt tttgttttta tttaaattca ttattaaaca gtctcttcga

1561  aagttaacat gggctataga cactaagctt caattgttcg aaggtaaaaa attggactta

1621  aaagtacatt agatagtctt acttagactt aaaatctgtt tattagacag tccttttgat

1681  aagtcacgtg gaccatgcta gactgtgttc gtatatactt gtagttacta ttcaattgtt

1741  tgaaggccga cctctcaatt cagtgagccc tttgctttag caacttcaaa cataaaaaaa

1801  aaaaaaaagt gcggctgaaa gcgatttgtg aagtacctct agtaggtctt aagattctca

1861  ttttcaggat ctggtgttgt ggcgcaatgg tgagcgcatc ggatttcggt tgtgaatctt

1921  ccgaaggttg caggttcgag tcctgtcaac atcggatctt tttttgcaat ccgttttgtc

1981  tgagttaagg agattgacaa tagctgggct ctgtcttgtc ttcgtaattg caacagaaaa

2041  taaagttgtc attattgaag cctctttttc gccctacggt ctgtttaaag tatcaaggtt

2101  aaagttttac agagatcatc gctttagcat atcaacaact ctccttcttt cgacatcagt

2161  cctgaattgc taaccttact tttgctcaat taatttctag agcgacgcat tcatctaata
```

```
2221  atctgtcttt  tcaaacttta  agcctgacat  gcaccggata  agatttaaaa  ctatccatcc
2281  taaatgcaat  catagatgaa  gtccttatat  acgcagtata  taatctatat  ttttcgatcg
2341  cactgactag  aagaaattgg  aatcacatat  cgcccat
```

Fig. 11: DNA-Sequenz des *ATAN2*-Gens von *A. adeninivorans* LS3 und davon abgeleitete Aminosäuresequenz der Tannase II (Atan2p). Die potentielle CAAT-Box und TATA-Box in der Promotor-Region wurden unterstrichen. Zusätzlich wurden vom Atan2p die Signalsequenz (Aminosäure-Pos. 1-18), die Prosequenz (Aminosäure-Pos. 19-26) und die N-Glycosylierungsstellen, sowie die konservierten Cysteine hervorgehoben.

# FIGUR 12

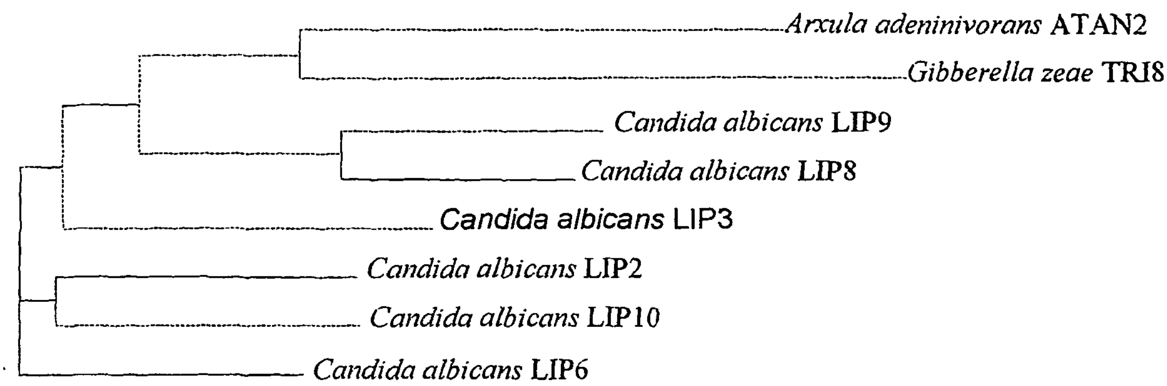
Arxula adeninivorans ATAN2
Gibberella zeae TRI8
Candida albicans LIP9
Candida albicans LIP8
Candida albicans LIP3
Candida albicans LIP2
Candida albicans LIP10
Candida albicans LIP6

FIGUR 13

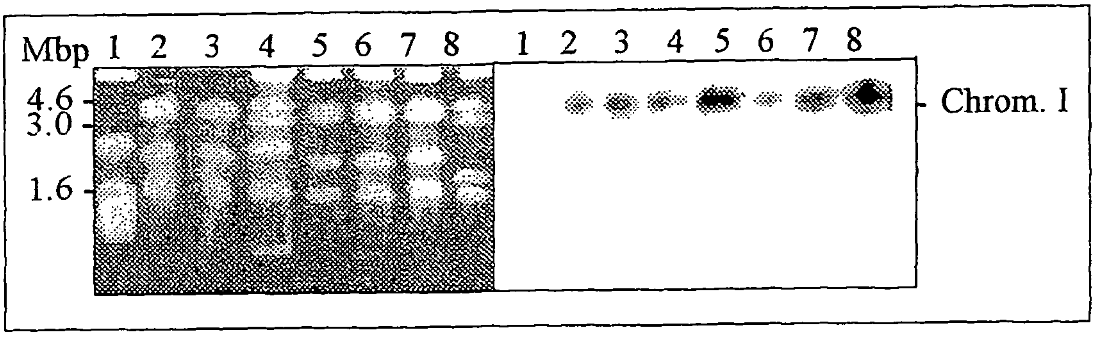

FIGUR 14

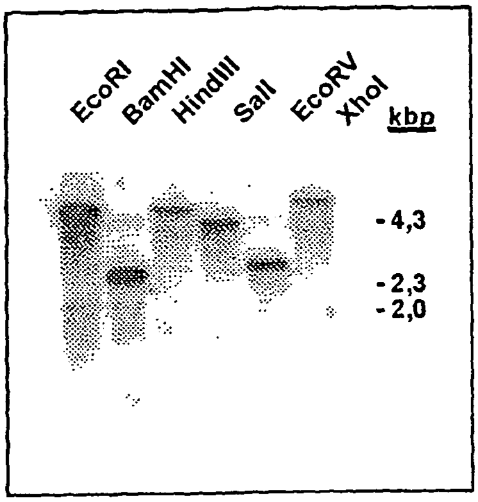

# FIGUR 15

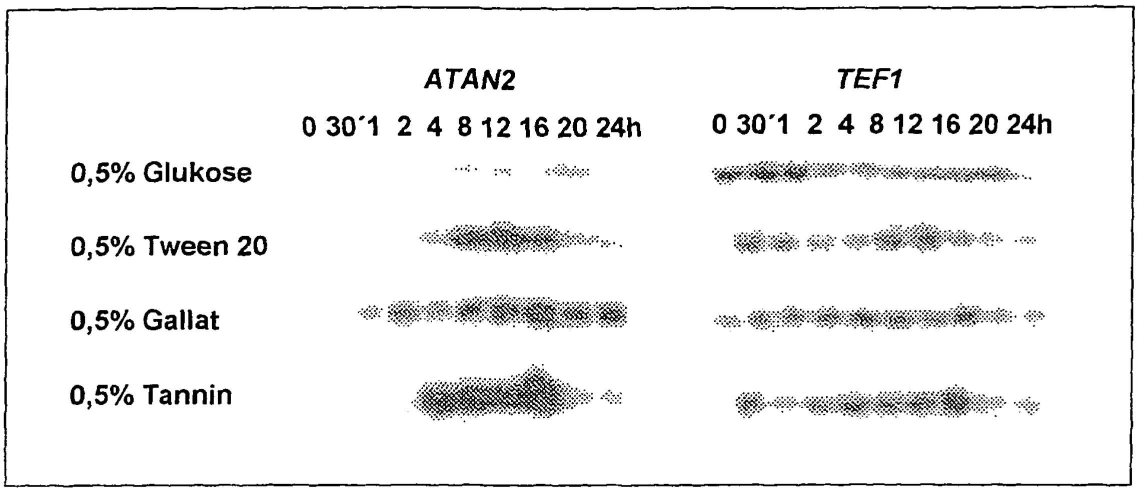
ATAN2
TEF1
0 30´1 2 4 8 12 16 20 24h
0 30´1 2 4 8 12 16 20 24h
0,5% Glukose
0,5% Tween 20
0,5% Gallat
0,5% Tannin

FIGUR 16

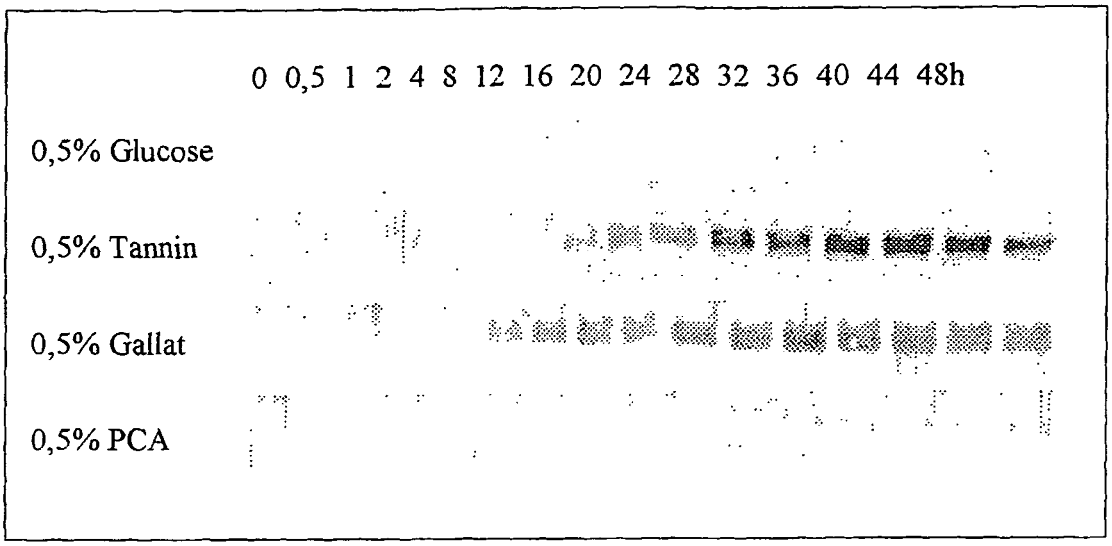
0 0,5 1 2 4 8 12 16 20 24 28 32 36 40 44 48h
0,5% Glucose
0,5% Tannin
0,5% Gallat
0,5% PCA

# FIGUR 17

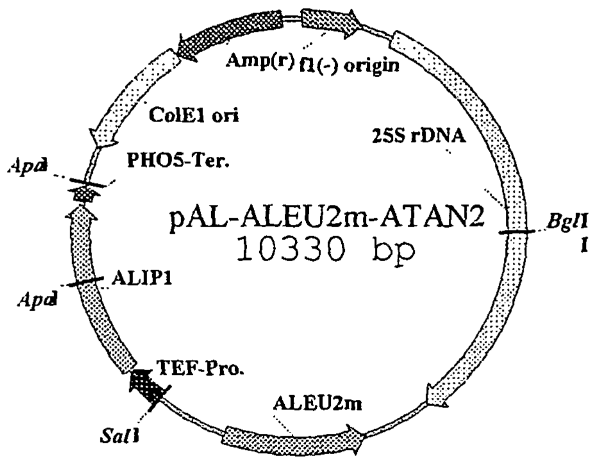
Amp(r)
f1(-) origin
ColE1 ori
25S rDNA
ApaI
PHO5-Ter.
pAL-ALEU2m-ATAN2
10330 bp
BglII
ALIP1
ApaI
TEF-Pro.
SalI
ALEU2m

## Figur 18

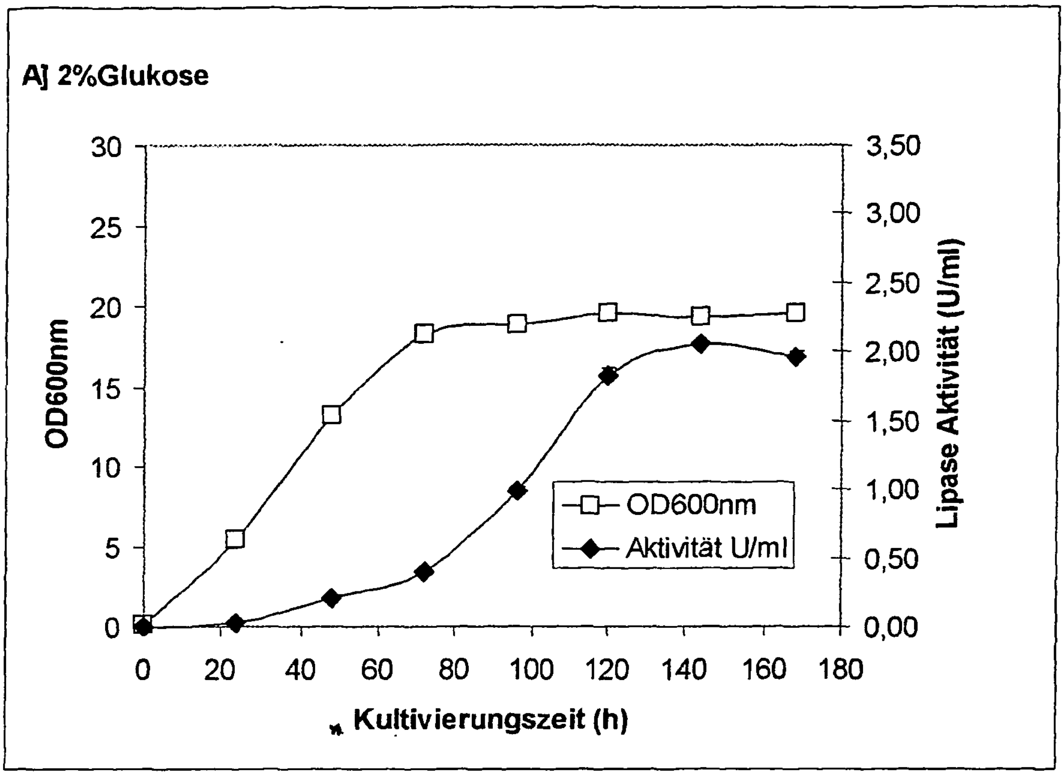
A] 2%Glukose
30
25
20
15
10
5
0
3,50
3,00
2,50
2,00
1,50
1,00
0,50
0,00
OD600nm
Lipase Aktivität (U/ml)
OD600nm
Aktivität U/ml
0 20 40 60 80 100 120 140 160 180
Kultivierungszeit (h)

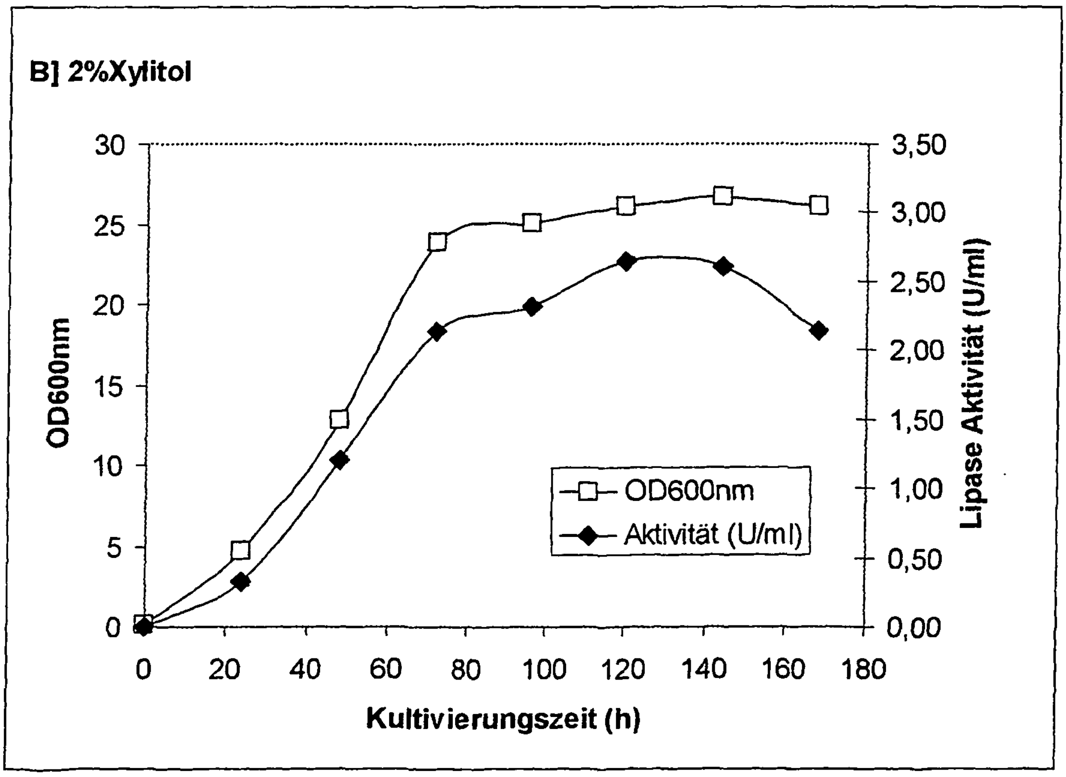
B] 2%Xylitol
30
25
20
15
10
5
0
3,50
3,00
2,50
2,00
1,50
1,00
0,50
0,00
OD600nm
Lipase Aktivität (U/ml)
OD600nm
Aktivität (U/ml)
0 20 40 60 80 100 120 140 160 180
Kultivierungszeit (h)

# Figur 19

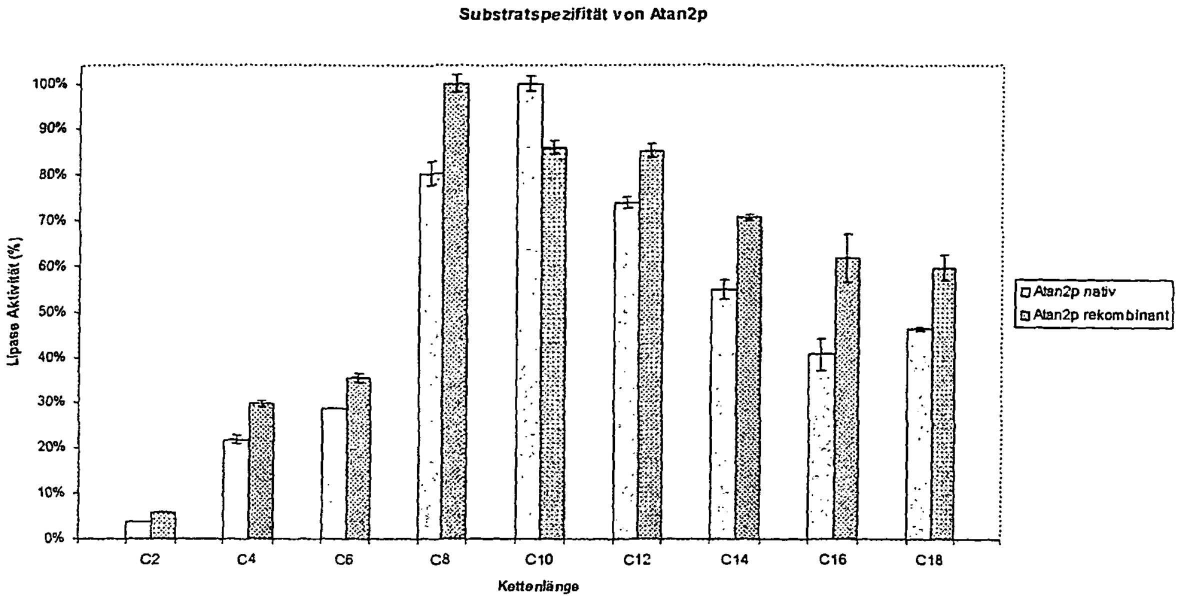
Substratspezifität von Atan2p
100%
90%
80%
70%
60%
50%
40%
30%
20%
10%
0%
Lipase Aktivität (%)
C2
C4
C6
C8
C10
C12
C14
C16
C18
Kettenlänge
Atan2p nativ
Atan2p rekombinant

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5820901 A
- US 6482450 B
- GB 11249932 A
- DE 10022334 A1
- EP 01938205 A
- DE 10034578 A1
- DE 10345388


### In der Beschreibung aufgeführte Nicht-Patentliteratur


- **VON CHUNG K. ; WONG, T. ; WEI, C ; HUANG Y.** *Critical Reviews in Food Science and Nutrition,* 1998, vol. 38 (6), 421-464
- **LEHKA, P. K ; LONSANE B. K.** *Advances in Applied Microbiology,* 1997, vol. 44
- **BHAT, T. K. ; SINGH, B. ; SHARMA O. P.** *Biodegradation,* 1998, vol. 9, 343-357
- **DESCHAMPS A. M. ; OUTUK, G. ; LEBCAULT, J. M.** *J. of Fermentation Technology,* 1983, vol. 61, 55-59
- **FREUDENBERG et al.** *Z. Physiol. Chem.,* 1927, vol. 164, 262
- **YAMADA, K. et al.** *J. Ferment. Tech.,* 1967, vol. 45, 233
- **PINTO et al.** *Brazilian J. Microbiol.,* 2001, vol. 32, 24-26
- **RAMIREZ-CORONEL MA ; VINIEGRA-GONZALEZ G ; DARVILL A ; AUGUR C.** A novel tannase from Aspergillus niger with ß-glucosidase activity. *Microbiology,* 2003, vol. 149, 2941-2946
- **BATZER et al.** *Nucleic Acid Res.,* 1991, vol. 19, 5081
- **SHARMA S ; BHAT TK ; DAWRA RK.** A spectrophotometric method for assay of tannase using Rhodanine. Anal. *Biochem.,* 2000, vol. 279, 85-89
- **S. F. ALTSCHUL et al.** Basic local alignment search tool. *J. Mol. Biol.,* 1990, vol. 215, 403-410
- **DEVEREUX, J. et al.** *Nucleic Acid Research,* 1984, vol. 12 (12), 287
- **ALTSCHUL, S. et al.** *J. Mol. Biol.,* 1999, vol. 215, 403-410
- **ALTSCHUL S. et al.** BLAST Handbuch. NCB NLM NIH Bethesda MD 20894
- **ALTSCHUL, S. et al.** *Mol. Biol.,* 1990, vol. 215, 403-410
- **NEEDLEMAN ; WUNSCH.** *J. Mol. Biol.,* 1970, vol. 48, 443-453
- **HENIKOFF ; HENIKOFF.** *PNAS USA,* vol. 89 (19), 10915-10919
- **CUNNIGHAM et al.** *Science,* 1998, vol. 244, 1081-1085
- **KYTE et al.** *J. Mol. Biol.,* 1982, vol. 157, 105-131
- **SAMBROOK ; FRITSCH ; MANIATIS.** Molecular Cloning: A Laboratory Manual. Cold Spring Harbor Laboratory, 1989
- **SAMBROOK ; FRITSCH ; MANIATIS.** Molecular Cloning: A Laboratory Manual. Cold Spring harbor Laboratory, 1989
- Zur Biochemie und Genetik von Hefen sei verwiesen auf. 1987
- **RÖSEL H ; KUNZE G.** *Curr. Genet.,* 1998, vol. 33, 157-163
- **WARTMANN T ; BÖER E ; PICO AH ; SIEBER H ; BARTELSEN O ; GELLISSEN G ; KUNZE G.** *FEMS Yeast Res,* 2002, vol. 2, 363-369
- **RÖSEL ; KUNZE ; WARTMANN T ; STOLTENBURG R ; BÖER R ; SIEBER H ; BARTELSEN O ; GELLISSEN G ; KUNZE G.** *FEMS Yeast Res.,* 2003, vol. 3, 223-232
- **WARTMANN T et al.** *Yeast,* 1998, vol. 14, 1017-1025
- **RÖSEL ; KUNZE.** *Curr. Genet.,* 1998, vol. 33, 157-163
- **SHARMA S ; BHAT TK ; DAWRA RK.** A spectrophotometric method for assay of tannase using Rhodanine. *Anal. Biochem.,* 2000, vol. 279, 85-89